# EUROPEAN PATENT APPLICATION

(11) **EP 2 818 544 A1**
(43) Date of publication of application: **31.12.2014**
(21) Application number: 14173429.3
(22) Date of filing: 23.06.2014
(51) Int. Cl.: C12N 5/078, G01N 33/50, A61K 35/00

(54) **Method for counting number of stem cells in human or animal samples**

(30) Priority: 24.06.2013 US 201361838359 P; 06.12.2013 US 201361912563 P
(71) Applicant: Stembios Technologies, Inc., Monterey Park CA 91754 (US)
(72) Inventor: Chen, Steve K, Monterey Park, CA California 91754 (US); LIn, Mou-Shiung, Monterey Park, CA California 91754 (US); Wang, James, Monterey Park, CA California 91754 (US); Yun, Yen, Monterey Park, CA California 91754 (US)
(74) Representative: Wittmann, Günther

(57) **Abstract**

A method of obtaining stem cells includes (1) a subject (such as a human or an animal) taking or being subjected to an action, (2) after the subject taking or being subject to the action, the subject waiting for a predetermined time interval (such as between 30 minutes and 2 hours), (3) after the subject waiting for the predetermined time interval, taking a tissue sample (such as a peripheral blood of the subject) from the subject, and (4) collecting the stem cells from the tissue sample. The step of the subject taking or being subjected to the action may include the subject taking a herb medicine or an object containing fucoidan. The stem cells may be configured for a dental implant surgery. The stem cells may include a CD9(+), CD349(+) cell between 0.1 and 6.0 micrometers in size and/or a Lgr5(+) cell between 0.1 and 6.0 micrometers in size.

## Description

### RELATED APPLICATION

This application claims priority to U.S. provisional application No. 61/838,359, filed on Jun. 24, 2013, and to U.S. provisional application No. 61/912,563, filed on Dec. 6, 2013, all of which are incorporated herein by reference in their entirety.

### BACKGROUND OF THE DISCLOSURE

### Field of the Disclosure

The disclosure is related to a method of increasing stem cells in a living being, and in particular, to a method of increasing the number of stem cells in a human body or an animal body.

### Brief Description of the Related Art

Stem cells have the ability to self-renew to generate more stem cells and also to turn into other types of cells. Stem cell research is useful for learning about human development and is one of the most fascinating areas of contemporary biology. Therefore, stem cells offer exciting promise for future medical science.

### SUMMARY OF THE DISCLOSURE

The present invention provides exemplary methods for cultivating, activating, mobilizing, stimulating, generating or increasing stem cells such as pluripotent stem cells in a human body or an animal body, and then obtaining, collecting or harvesting the stem cells from the peripheral blood of the human body or the animal body.

The present invention also provides exemplary methods for obtaining a stem-cell data (such as the count per unit volume of a type of stem cells in a tissue sample obtained from a human body or an animal body).

An exemplary embodiment of the present disclosure provides a method of obtaining stem cells, including: (1) a subject (such as a human or an animal) taking or being subjected to an action; (2) after the subject taking or being subject to the action, the subject waiting for a predetermined time interval; (3) after the subject waiting for the predetermined time interval, taking a tissue sample from the subject; and (4) collecting the stem cells from the tissue sample. The step of the subject taking or being subjected to the action may include the subject taking a herb medicine or an object containing fucoidan. The predetermined time interval may be between 30 minutes and 2 hours, between 1 hour and 12 hours, or between 12 hours and 36 hours. The tissue sample may include a peripheral blood of the subject. The stem cells may include multiple pluripotent stem cells (such as CD9(+), CD349(+) cells between 0.1 and 6.0 micrometers in size, CD349(+) cells between 0.1 and 6.0 micrometers in size, Lgr5(+) cells between 0.1 and 6.0 micrometers in size, or CD9(-), SSEA4(+) cells between 0.1 and 6.0 micrometers in size). The method of obtaining the stem cells may include, after collecting the stem cells from the tissue sample, storing the stem cells in a temperature lower than 0 degrees Celsius. The stem cells may be configured for a dental implant surgery, a plastic surgery, a knee meniscus injury treatment, or an arthritis treatment.

Another exemplary embodiment of the present disclosure provides a method of obtaining a stem-cell data, including: (1) obtaining a tissue sample from a subject (such as a human or an animal); (2) processing the tissue sample into a test sample; (3) using a hemocytometer to count a first group of particles in a first portion of the test sample so as to obtain a first data; (4) using a flow cytometer to count a type of stem cells in a second group of particles in a second portion of the test sample so as to obtain a second data; and (5) obtaining a third data based on the first and second data. The tissue sample may include a peripheral blood of the subject. The first and second groups of particles may have sizes greater than or substantially equal to a threshold size (such as 1 micrometer). The second group of particles may include multiple white blood cells, multiple red blood cells, multiple blood platelets, and/or multiple granulocytes. The type of stem cells may include a type of multipotent stem cells or a type of pluripotent stem cells (such as CD9(+), CD349(+) cells between 0.1 and 6.0 micrometers in size, CD349(+) cells between 0.1 and 6.0 micrometers in size, Lgr5(+) cells between 0.1 and 6.0 micrometers in size, or CD9(-), SSEA4(+) cells between 0.1 and 6.0 micrometers in size). The first data may include the count per unit volume of the first group of particles in the test sample. The second data may include a percentage of the count of the type of stem cells to that of the second group of particles. The third data may include the count per unit volume of the type of stem cells in the tissue sample.

Another exemplary embodiment of the present disclosure provides a method of obtaining a stem-cell data, including: (1) obtaining a tissue sample from a subject (such as a human or an animal); (2) processing the tissue sample into a test sample; (3) counting a first group of particles in a first portion of the test sample so as to obtain a first data; (4) counting a type of stem cells in a second group of particles in a second portion of the test sample so as to obtain a second data; and (5) obtaining a third data based on the first and second data. The tissue sample may include a peripheral blood of the subject. The first and second groups of particles have sizes greater than or substantially equal to a threshold size (such as 1 micrometer). The second group of particles may include multiple white blood cells, multiple red blood cells, multiple blood platelets, and/or multiple granulocytes. The type of stem cells may include a type of multipotent stem cells or a type of pluripotent stem cells (such as CD9(+), CD349(+) cells between 0.1 and 6.0 micrometers in size, CD349(+) cells between 0.1 and 6.0 micrometers in size, Lgr5(+) cells between 0.1 and 6.0 micrometers in size, or CD9(-), SSEA4(+) cells between 0.1 and 6.0 micrometers in size). The first data may include the count per unit volume of the first group of particles in the test sample. The second data may include a percentage of the count of the type of stem cells to that of the second group of particles. The third data may include the count per unit volume of the type of stem cells in the tissue sample. The method of obtaining the stem-cell data may further include using a hemocytometer to perform counting the first group of particles in the first portion of the test sample so as to obtain the first data. The method of obtaining the stem-cell data may further include using a flow cytometer to perform counting the type of stem cells in the second group of particles in the second portion of the test sample so as to obtain the second data.

These, as well as other components, steps, features, benefits, and advantages of the present disclosure, will now become clear from a review of the following detailed description of illustrative embodiments, the accompanying drawings, and the claims.

### BRIEF DESCRIPTION OF THE DRAWINGS

The drawings disclose illustrative embodiments of the present disclosure. They do not set forth all embodiments. Other embodiments may be used in addition or instead. Details that may be apparent or unnecessary may be omitted to save space or for more effective illustration. Conversely, some embodiments may be practiced without all of the details that are disclosed. When the same reference number or reference indicator appears in different drawings, it may refer to the same or like components or steps.

Aspects of the disclosure may be more fully understood from the following description when read together with the accompanying drawings, which are to be regarded as illustrative in nature, and not as limiting. The drawings are not necessarily to scale, emphasis instead being placed on the principles of the disclosure. In the drawings:
Figs. 1-6 show various process flow diagrams of purification processes;
Fig. 7A shows a flow chart of obtaining, collecting or harvesting a large number of stem cells from a tissue sample extracted, taken, obtained or derived from a subject according to an embodiment of the present disclosure;
Fig. 7B shows a flow chart of discovering or detecting a new type or types of stem cells according to an embodiment of the present disclosure;
Fig. 7C shows a flow chart of identifying, determining or evaluating the effectiveness of one or more actions or stimuli for curing or treating a subject having a specific disease according to an embodiment of the present disclosure;
Figs. 8A-8F show stem-cell data or information of three human subjects before and after orally taking 30 pills of a brown algae fucoidan supplement;
Fig. 9 shows a flow chart of obtaining stem-cell data or information related to a human subject according to an embodiment of the present disclosure;
Fig. 10 shows a forward scattering coefficient (FSC)/side scattering coefficient (SSC) graph for a flow cytometer;
Fig. 11 shows the content/ingredient information of a brown algae fucoidan supplement;
Fig. 12 shows a portion of the molecule structure of fucoidan;
Fig. 13 shows a flow chart of obtaining stem-cell data or information related to a subject according to an embodiment of the present disclosure;
Figs. 14A-14C are flow charts for illustrating a measurement or analysis used to obtain stem-cell data or information according to an embodiment of the present disclosure;
Fig. 15 shows a microscopic grid of a hemocytometer;
Fig. 16 shows a forward scattering coefficient (FSC)/side scattering coefficient (SSC) graph for a flow cytometer;
Fig. 17A shows stem-cell data or information related to a human subject at 1.5 hours after ingestion of brown algae fucoidan supplement;
Fig. 17B is a fluorescence graph of a flow cytometer, which shows a fluorescence intensity distribution of cells in a region of high nucleus/cytoplasm ratio;
Fig. 18 shows a flow chart of obtaining stem-cell data or information related to an experimental subject at four specific time points according to an embodiment of the present disclosure;
Fig. 19 shows a flow chart of obtaining stem-cell data or information related to a control subject at four specific time points according to an embodiment of the present disclosure;
Figs. 20A-20J show stem-cell data or information of five human subjects before and after orally taking 30 pills of a brown algae fucoidan supplement; and
Figs. 20K and 20L show stem-cell data or information of a human subject without orally taking 30 pills of a brown algae fucoidan supplement at four specific time points.

While certain embodiments are depicted in the drawings, one skilled in the art will appreciate that the embodiments depicted are illustrative and that variations of those shown, as well as other embodiments described herein, may be envisioned and practiced within the scope of the present disclosure.

### DETAILED DESCRIPTION OF THE INVENTION

Illustrative embodiments are now described. Other embodiments may be used in addition or instead. Details that may be apparent or unnecessary may be omitted to save space or for a more effective presentation. Conversely, some embodiments may be practiced without all of the details that are disclosed.

Before describing embodiments of the present invention, a definition or description has been included for these various terms. These definitions or descriptions are provided to assist in teaching a general understanding of the present invention.

### Definition of size (Z) of a cell:

The size (Z) of a cell such as a stem cell or a biological cell, mentioned in all following paragraphs, of the present disclosure may be, but not limited to, described or defined as (1) the conventional definition of the size or representative length of a cell in the field of cell biology or the field of stem cells, (2) the diameter of a cell especially when the cell is substantially spherical, (3) the length of the major axis of a cell especially when the cell is substantially ellipsoidal, (4) the width of a cell when the shape of the cell has an approximate shape of a square, (5) the length of a cell when the shape of the cell has an approximate shape of a rectangle, or (6) the greatest cross-sectional or transverse dimension of a cell. The size (Z), either the diameter, length, width, or greatest cross-sectional or transverse dimension, can be, but not limited to, determined or measured, for example, using an image of the cell obtained from an optical microscope or from an electron microscope (e.g., scanning electron microscope (SEM)), or using data (e.g., two-dimensional dot, contour or density plot) of the cell obtained from a flow cytometer. The image of the cell obtained from the optical microscope or electron microscope may be a two-dimensional (2D) cross section or three-dimensional (3D) structure of the cell. As an example, the size (Z) of the cell may be obtained by, e.g., measuring the greatest cross-sectional or transverse dimension of the cell in a 2D cross-sectional image obtained from an optical microscope or an electron microscope (e.g., SEM).

### Description of stem cells:

There are various types of stem cells, including pluripotent stem cells, multipotent stem cells, and progenitor stem cells (also called unipotent stem cells). SB-1 cells, SB-2 cells, blastomere-like stem cells (BLSCs), and very small embryonic-like stem cells (VSELs) are pluripotent stem cells. Mesenchymal stem cells (MSCs), multipotent adult progenitor cells (MAPCs), bone marrow derived multipotent stem cells (BMSCs), and multipotent adult stem cells (MASCs) are multipotent stem cells. Neural stem cells, retina stem cells, olfactory bulbs stem cells, epidermal stem cells, muscle stem cells, intestine stem cells, pancreatic stem cells, heart stem cells, liver stem cells, kidney stem cells, endothelial stem cells, adipocyte or adipose-derived stem cells, marrow-isolated adult multilineage inducible (MIAMI) cells, pre-mesenchymal stem cells (pre-MSCs), mesenchymal progenitor cells, hematopoietic progenitor cells (HPCs), multipotent progenitor cells (MPPs), lineage-restricted progenitor cells (LRPs), common myeloid progenitor cells (CMPs), and common lymphocyte progenitor cells (CLPs) are progenitor stem cells.

In the following paragraphs the sign "+" following a cell (surface) marker means the stem cells can express the cell (surface) marker; in the other term, the cell (surface) marker existing in the cell surfaces of the stem cells may be detected by performing a flow cytometry using a (marker-specific) antibody. And, the sign "-" following a cell (surface) marker means the stem cells do not express the cell (surface) marker; in the other term, the cell (surface) marker, not existing in the cell surfaces of the stem cells, may not be detected by performing a flow cytometry using a (marker-specific) antibody. The positive sign "+" is a positive expression of a cell (surface) marker for a stem cell, and the negative sign "-" is a negative expression of a cell (surface) marker for a stem cell.

A SB-1 cell, which is a pluripotent stem cell having a nucleus, may be a CD9(+), CD349(+) cell or a CD349(+) cell. The CD9(+), CD349(+) pluripotent stem cell or the CD349(+) pluripotent stem cell, i.e., the SB-1 cell, may be smaller than or equal to 4, 5 or 6 micrometers, such as between 0.1 and 6.0 micrometers, between 0.5 and 6.0 micrometers, between 1.0 and 6.0 micrometers, between 0.1 and 5.0 micrometers, between 0.5 and 5.0 micrometers, between 1.0 and 5.0 micrometers, between 0.1 and 4.0 micrometers, between 0.5 and 4.0 micrometers, or between 1.0 and 4.0 micrometers, in size (as defined by the above-mentioned size (Z) of a cell). In the case of the CD9(+), CD349(+) pluripotent stem cell, the SB-1 cell may express the two cell (surface) markers CD9 and CD349 and may be characterized by CD9(+) and CD349(+). In the case of the CD349(+) pluripotent stem cell, the SB-1 cell may express the cell (surface) marker CD349 and may be characterized by CD349(+).

A SB-2 cell, which is a pluripotent stem cell having a nucleus, may be a CD9(-), SSEA4(+) cell or a Lgr5(+) cell. The CD9(-), SSEA4(+) pluripotent stem cell or the Lgr5(+) pluripotent stem cell, i.e., the SB-2 cell, may be smaller than or equal to 4, 5 or 6 micrometers, such as between 0.1 and 6.0 micrometers, between 0.5 and 6.0 micrometers, between 1.0 and 6.0 micrometers, between 0.1 and 5.0 micrometers, between 0.5 and 5.0 micrometers, between 1.0 and 5.0 micrometers, between 0.1 and 4.0 micrometers, between 0.5 and 4.0 micrometers or between 1.0 and 4.0 micrometers, in size (as defined by the above-mentioned size (Z) of a cell). In the case of the CD9(-), SSEA4(+) pluripotent stem cell, the SB-2 cell may express the cell (surface) marker SSEA4 and lacks expression of the cell (surface) marker CD9 and may be characterized by CD9(-) and SSEA4(+). In the case of the Lgr5(+) pluripotent stem cell, the SB-2 cell may express the cell (surface) marker Lgr5 and may be characterized by Lgr5(+).

A blastomere-like stem cell (BLSC), which is a pluripotent stem cell having a nucleus, may be a CD66e(+) cell. The CD66e(+) pluripotent cell, i.e., the BLSC, may be smaller than or equal to 4, 5 or 6 micrometers, such as between 0.1 and 6.0 micrometers, between 0.5 and 6.0 micrometers, between 1.0 and 6.0 micrometers, between 0.1 and 5.0 micrometers, between 0.5 and 5.0 micrometers, between 1.0 and 5.0 micrometers, between 0.1 and 4.0 micrometers, between 0.5 and 4.0 micrometers or between 1.0 and 4.0 micrometers, in size (as defined by the above-mentioned size (Z) of a cell). The BLSC may express the cell (surface) marker CD66e and may be characterized by CD66e(+).

A very small embryonic-like stem cell (VSEL), which is a pluripotent stem cell having a nucleus, may be a CD133(+), CD45(-), Lin(-) cell or a CXCR4(+), CD45(-), Lin(-) cell. The CD133(+), CD45(-), Lin(-) pluripotent stem cell or the CXCR4(+), CD45(-), Lin(-) pluripotent stem cell, i.e., the VSEL, may be smaller than or equal to 4, 5 or 6 micrometers, such as between 0.1 and 6.0 micrometers, between 0.5 and 6.0 micrometers, between 1.0 and 6.0 micrometers, between 0.1 and 5.0 micrometers, between 0.5 and 5.0 micrometers, between 1.0 and 5.0 micrometers, between 0.1 and 4.0 micrometers, between 0.5 and 4.0 micrometers or between 1.0 and 4.0 micrometers, in size (as defined by the above-mentioned size (Z) of a cell). In the case of the CD133(+), CD45(-), Lin(-) pluripotent stem cell, the VSEL may express the cell (surface) marker CD133 and lacks expression of the two cell (surface) markers CD45 and Lin and may be characterized by CD133(+), CD45(-), and Lin(-). In the case of the CXCR4(+), CD45(-), Lin(-) pluripotent stem cell, the VSEL may express the cell (surface) marker CXCR4 and lacks expression of the two cell (surface) markers CD45 and Lin and may be characterized by CXCR4(+), CD45(-), Lin(-). In addition, the VSEL may be described with the following characteristics: (1) are slightly smaller than red blood cells; and (2) are enriched in the CD133(+), CD45(-), Lin(-) cell fraction or the CXCR4(+), CD45(-), Lin(-) cell fraction in humans.

A mesenchymal stem cell (MSC), which is a multipotent stem cell having a nucleus, may be a CD13(+), CD29(+), CD44(+), CD73(+), CD90(+) or CD105(+) multipotent stem cell. The MSC may express one or more of the cell (surface) markers CD13, CD29, CD44, CD73, CD90 and CD105 and may be characterized by one or more of CD13(+), CD29(+), CD44(+), CD73(+), CD90(+) and CD105(+).

Mesenchymal stem cells (MSCs) are very heterogeneous populations which mean to have various sizes and shapes. Some types of MSCs may be smaller than or equal to 4, 5 or 6 micrometers, such as between 0.1 and 6.0 micrometers, between 0.5 and 6.0 micrometers, between 1.0 and 6.0 micrometers, between 0.1 and 5.0 micrometers, between 0.5 and 5.0 micrometers, between 1.0 and 5.0 micrometers, between 0.1 and 4.0 micrometers, between 0.5 and 4.0 micrometers or between 1.0 and 4.0 micrometers, in size (as defined by the above-mentioned size (Z) of a cell). The other types of MSCs may be greater than 6, 7 or 10 micrometers in size (as defined by the above-mentioned size (Z) of a cell).

A hematopoietic stem cell (HSC), which is a multipotent stem cell having a nucleus, may be a CD34(+), cKit(-), CD38(-), Lin(-) cell or a CD150(+), CD244(-), CD48(-) cell. The CD34(+), cKit(-), CD38(-), Lin(-) multipotent stem cell or the CD150(+), CD244(-), CD48(-) multipotent stem cell, i.e., the HSC, may be smaller than or equal to 4, 5 or 6 micrometers, such as between 0.1 and 6.0 micrometers, between 0.5 and 6.0 micrometers, between 1.0 and 6.0 micrometers, between 0.1 and 5.0 micrometers, between 0.5 and 5.0 micrometers, between 1.0 and 5.0 micrometers, between 0.1 and 4.0 micrometers, between 0.5 and 4.0 micrometers or between 1.0 and 4.0 micrometers, in size (as defined by the above-mentioned size (Z) of a cell). In the case of the CD34(+), cKit(-), CD38(-), Lin(-) multipotent stem cell, the HSC may express the cell (surface) marker CD34 and lacks expression of the cell (surface) markers cKit, CD38 and Lin and may be characterized by CD34(+), cKit(-), Lin(-) and CD38(-). In the case of the CD150(+), CD244(-), CD48(-) multipotent stem cell, the HSC may express the cell (surface) marker CD150 and lacks expression of the two cell (surface) markers CD244 and CD48 and may be characterized by CD150(+), CD244(-) and CD48(-).

A multipotent adult progenitor cell (MAPC), which is a multipotent stem cell having a nucleus, may be a CD13(+), SSEA1(+) cell. The CD13(+), SSEA1(+) multipotent stem cell, i.e., the MAPC, may express the cell (surface) markers CD 13 and SSEA1 and may be characterized by CD13(+) and SSEA1(+). A bone marrow derived multipotent stem cell (BMSC), which is a multipotent stem cell having a nucleus, may be a CD105(+) multipotent stem cell, a CD117(+) multipotent stem cell, or a CD13(+), SSEA3(+) multipotent stem cell. In the case of the CD105(+) multipotent stem cell, the BMSC may express the cell (surface) marker CD105 and may be characterized by CD105(+). In the case of the CD117(+) multipotent stem cell, the BMSC may express the cell (surface) marker CD 117 and may be characterized by CD117(+). In the case of the CD13(+), SSEA3(+) multipotent stem cell, the BMSC may express the cell (surface) markers CD13 and SSEA3 and may be characterized by CD13(+) and SSEA3(+). A multipotent adult stem cell (MASC), which is a multipotent stem cell having a nucleus, may be a Oct4(+) multipotent stem cell, a Nanog(+) multipotent stem cell, or a RexI(+) multipotent stem cell. In the case of the Oct4(+) multipotent stem cell, the MASC may express the cell (surface) marker Oct4 and may be characterized by Oct4(+). In the case of the Nanog(+) multipotent stem cell, the MASC may express the cell (surface) marker Nanog and may be characterized by Nanog(+). In the case of the RexI(+) multipotent stem cell, the MASC may express the cell (surface) marker RexI and may be characterized by RexI(+). All the MAPC, the BMSC and the MASC may be greater than 6, 7 or 10 micrometers, such as between 8 and 15 micrometers, in size (as defined by the above-mentioned size (Z) of a cell).

A marrow-isolated adult multilineage inducible (MIAMI) cell, which is a progenitor stem cell having a nucleus, may be a CD29(+), CD63(+), CD81(+), CD122(+), CD164(+) progenitor stem cell. The MIAMI cell may express the cell (surface) markers CD29, CD63, CD81, CD122 and CD164 and may be characterized by CD29(+), CD63(+), CD81(+), CD122(+) and CD164(+). A pre-mesenchymal stem cell (pre-MSC), which is a progenitor stem cell having a nucleus, may be a SSEA1(+) progenitor stem cell. The pre-MSC may express the cell (surface) marker SSEA1 and may be characterized by SSEA1(+). Each of the MIAMI cell and the pre-MSC may be greater than 6, 7 or 10 micrometers, such as between 8 and 15 micrometers, in size (as defined by the above-mentioned size (Z) of a cell).

A multipotent progenitor cell (MPP), which is a progenitor stem cell having a nucleus, may be a CD34(+), cKit(+), CD38(-), Lin(-) cell or a CD150(-), CD244(+), CD48(-) cell. The CD34(+), cKit(+), CD38(-), Lin(-) progenitor stem cell or the CD150(-), CD244(+), CD48(-) progenitor stem cell, i.e., the MPP, may be greater than 6, 7 or 10 micrometers, such as between 8 and 15 micrometers, in size (as defined by the above-mentioned size (Z) of a cell). In the case of the CD34(+), cKit(+), CD38(-), Lin(-) progenitor stem cell, the MPP may express the two cell (surface) markers CD34 and cKit and lacks expression of the two cell (surface) markers CD38 and Lin and may be characterized by CD34(+), cKit(+), CD38(-) and Lin(-). In the case of the CD150(-), CD244(+), CD48(-) progenitor stem cell, the MPP may express the cell (surface) marker CD244 and lacks expression of the two cell (surface) markers CD150 and CD48 and may be characterized by CD150(-), CD244(+) and CD48(-).

A lineage-restricted progenitor cell (LRP), which is a progenitor stem cell having a nucleus, may be a CD34(-), cKit(+), CD38(-), Lin(-) cell or a CD150(-), CD244(+), CD48(+) cell. The CD34(-), cKit(+), CD38(-), Lin(-) progenitor stem cell or the CD150(-), CD244(+), CD48(+) progenitor stem cell, i.e., the LRP, may be greater than 6, 7 or 10 micrometers, such as between 8 and 15 micrometers, in size (as defined by the above-mentioned size (Z) of a cell). In the case of the CD34(-), cKit(+), CD38(-), Lin(-) progenitor stem cell, the LRP may express the cell (surface) marker cKit and lacks expression of the cell (surface) markers CD34, CD38 and Lin and may characterized by CD34(-), cKit(+), CD38(-) and Lin(-). In the case of the CD150(-), CD244(+), CD48(+) progenitor stem cell, the LRP may express the two cell (surface) markers CD244 and CD48 and lacks expression of the cell (surface) marker CD150 and may be characterized by CD150(-), CD244(+) and CD48(+).

A common myeloid progenitor (CMP) cell or common lymphocyte progenitor (CLP) cell is a progenitor stem cell having a nucleus and may be a CD90(+) cell. The CMP or CLP cell, e.g., CD90(+) progenitor stem cell, may be greater than 6, 7 or 10 micrometers, such as between 8 and 15 micrometers, in size (as defined by the above-mentioned size (Z) of a cell). The CMP or CLP may express the cell (surface) marker CD90 and may be characterized by CD90(+). A hematopoietic progenitor cell (HPC), which is a progenitor stem cell having a nucleus, may be a CD24(+), CD38(+), CD48(+), CD244(+) or CXCR4(+) progenitor stem cell. The HPC may express one or more of the cell (surface) markers CD24, CD38, CD48, CD244 and CXCR4 and may be characterized by one or more of CD24(+), CD38(+), CD48(+), CD244(+) and CXCR4(+). A mesenchymal progenitor cell, which is a progenitor stem cell having a nucleus, may be a CXCR4(+), SSEA1(+), CD271(+) or Stro-1(+) progenitor stem cell. The mesenchymal progenitor cell may express one or more of the cell (surface) markers CXCR4, SSEA1, CD271 and Stro-1 and may be characterized by one or more of CXCR4(+), SSEA1(+), CD271(+) and Stro-1(+).

### Description of actions or stimuli (X):

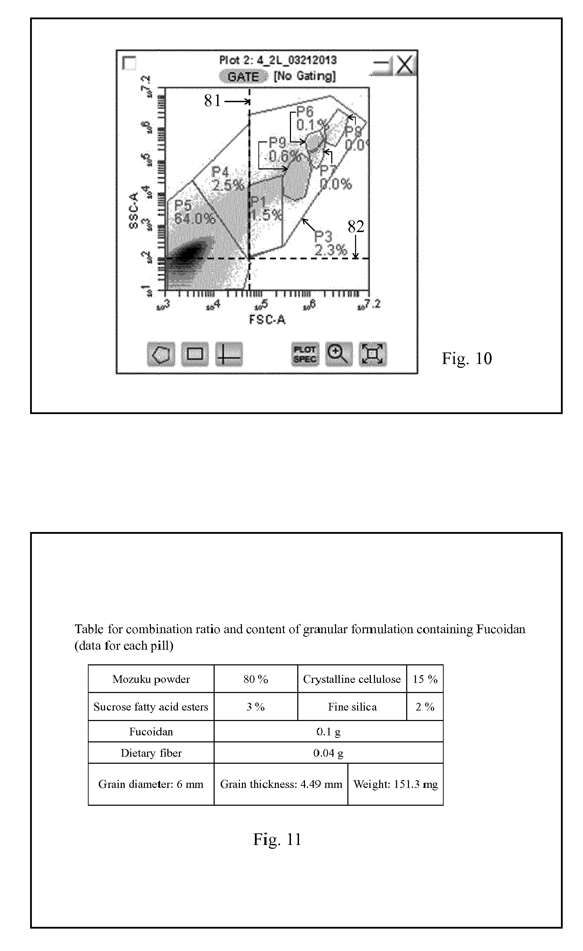

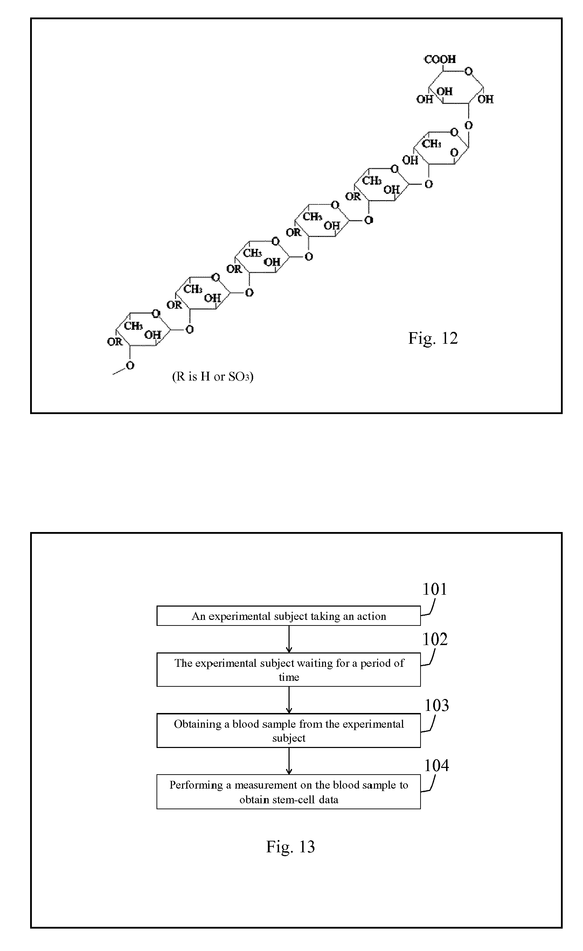

The following items are examples of the actions or stimuli (X), which may be previously evaluated effective in increasing the number of cells for a type or selected types of stem cells in vivo in a subject such as a human body or entity or a non-human body or entity. The non-human body or entity may be an animal body or entity. The actions or stimuli (X) include:
1. Taking drugs such as synthetic drugs or drugs including extractions from nature;
2. Taking herbal or Chinese medicines such as Cordyceps sinensis, ginseng, Lycium Chinense Mill, Ganoderma lucidum (lingzhi), Taiwanofungus camphoratus, and/or Brazil mushroom;
3. Taking nutrients or dietary supplements, such as nutrition pills or powder, including or comprising the following materials or elements: vitamins (Vitamin A, B, B complex, B₁₂, D, D₃, E, etc.), macro and/or trace minerals (e.g., calcium, sodium, potassium, fluorine, bromine, chromium, iodine, silicon, selenium, beryllium, lithium, cobalt, vanadium and/or nickel), polysaccharides, high molecular weight fucose-containing glycoproteins, seaweed (including green algae, blue-green algae, brown algae, and etc.), fucose, fucoidan that is a major component of brown algae, oligo fucoidan, algae, brown algae containing fucoidan (for example, brown algae grown and produced in Okinawa, Japan), Japanese Mozuku, green algae, blue-green algae (or blue algae), brown algae (including mozuku, kelp, undaria, sargassum fusiforme, pinnatifida, and etc.), phytochemical (e.g., isoflavones or phytoestrogen), lycopene, epigallocatechin gallate (EGCG), green tea essence, gluconutrients (e.g., Xylose, Galactose, Glucose, Mannose N-acetylglucosamine, N-acetylgalaetosanmine, or N-acetylneuraminic acid), fish oil, China toona (toona sinensis), and/or nutrients extracted from plant, leaf, fruit, vegetable, fish, seaweed, or algae;
4. Practicing a vegetarian dietary;
5. Taking or eating healthy food or organic food;
6. Taking alternative (non-traditional) medicine;
7. Being subjected to alternative therapy or treatment such as the Gerson therapy or the Breuss cancer cure;
8. Being subjected to acupuncture;
9. Being subjected to massage such as foot massage;
10. Exercising such as walking, jogging, dancing, gymnastics, Yoga, aerobic exercise, and/or Taijiquan (Chinese shadow exercise);
11. Sleeping (for purpose of measuring the quality of sleep);
12. Meditating;
13. Exercising a health improvement program or a disease curing program designed by an individual, a health professional, or a medical doctor;
14. Taking a certain nutrient for improving health of a certain organ in a body, for example, taking lycopene to improve the health of prostate;
15. Taking a rehabilitation program to heal the injury, or to heal the wounds caused by surgery, or to cure a disease;
16. Taking a medicinal liquor (or called medicinal wine, medicated liquor or medicated wine) made from, e.g., immersing one Chinese medicine or multiple Chinese medicines in liquor or wine for a period of time, such as ginseng wine made from immersing ginseng in a high alcohol concentration rice wine for a month;
17. Taking one or more drugs approved by a government department or authority, such as U.S. food and drug administration (U.S. FDA), for curing a specific disease (e.g., a type of cancer, skin disease, kidney disease and/or so on);
18. Taking or being subjected to treatments or therapies approved by a government department for curing a specific disease (e.g., a type of cancer, skin disease, or kidney disease);
19. Exercising or participating a religious activity, such as praying for peace or worshiping God;
20. Being exposed directly or indirectly to sunshine or sunlight (in the morning between, for example, 10 minutes before sunrise and 50 minutes after sunrise (containing significant amount of infrared (IR) light); or around noon, for example, between 11:30 AM to 12:30 PM (containing significant amount of ultra-violet (UV) light); or in the afternoon, for example, between 50 minutes before sunset and 10 minutes after sunset (containing significant amount of infrared (IR) light));
21. Being exposed to the lamp light or the light emitting diode (LED) light, which may include a whole spectrum of visible lights, IR light, red light, green light, blue light, or UV light, or a combination of more than one of the above lights;
22. Exercising or being subjected to programs, therapies, methods, apparatus and/or systems for improving body's self-healing, for example, a method or therapy (e.g., Hyperbaric oxygen therapy) performed after injury or surgery for improving self-healing;
23. Drinking coffee such as black coffee;
24. Drinking tea such green tea, black tea, or jasmine tea;
25. Drinking red wine;
26. Taking melatonin;
27. Listening to music such as Mozart's or Beethoven's symphony;
28. Orally taking one or more pills or tablets of a brown algae supplement, each of which may contain more than 60 percent in weight of fucoidan or oligo fucoidan, wherein the brown algae supplement may be referred to the following first experiment and Fig. 11;
29. Injecting a substance (e.g., a nutrient or supplement) containing fucoidan or oligo fucoidan;
30. Taking or ingesting 4.5 grams (or greater than 3.5 grams) of an Okinawa brown algae supplement, as mentioned in the following first experiment and Fig. 11, containing 80% (or more than 70%) of a mozuku powder in weight, wherein the 4.5 grams of the Okinawa brown algae supplement may include 3 grams of fucoidan (or greater than 2 grams, or more than 60% of fucoidan in weight), and a portion of the molecule structure of fucoidan may be referred to Fig. 12;
31. Taking hormone supplements or being subjected to a hormone injection; and
32. Taking a nutrient, a nutrient product, a nutrient fluid, a nutrient drink, a nutrient liquid, or a nutrient food containing (1) varieties of amino acids (such as Arginine, Histidine, Lysine, Aspartic acid, Glutamic acid, Serine, Threonine, Asparagine, Glutamine, Cysteine, Valine, Proline, Glycine, Selenocysteine, Alanine, Isoleucine, Leucine, Phenylalanine, Methionine, Tyrosine, or Tryptophan), (2) balanced amino acids, or (3) 9 essential amino acids (i.e., Histidine, Isoleucine, Leucine, Lysine, Methionine, Phenylalanine, Threonine, Tryptophan and Valine) for human bodies. For examples: (a) Product produced or extracted from the fermentation of red, green, black beans; (b) Liquid, fluid, or drink produced from fermentation of a fruit or a combination of fruits, such as sugar beet, apple, guava, kiwi, grape, pineapple, red pitaya (dragon fruit), green papaya, tomato, and/or avocado, etc.; (c) A medicinal liquor (or called medicinal wine, medicated liquor, or medicated wine) made from, e.g., immersing one Chinese medicine or multiple Chinese medicines in liquor or wine for a period of time, such as ginseng wine made from immersing ginseng in a high alcohol concentration rice wine for a month.

The actions or stimuli (X) may be performed based on the dose, intensity, duration, frequency (each action may comprise more than one sub-actions, for example, taking 1 pill of drug for three times with one hour apart), and/or the time (for example, the time of a day (in the morning, at noon, in the afternoon, in the evening, or in the night), the time before, with or after the meal, or the time of the year (spring, summer, autumn or winter)). As an example, when a subject (such as human or non-human body) is taking a drug or a nutrient, the dose (for example, the amount in grams), the time (for example, before or after breakfast, or before sleep) are factors needed to be considered. As another example, when the subject is exposed to the sunshine or sunlight, the time of the day (morning, noon or afternoon), the time of seasons (spring, summer, autumn, or winter), or the exposing duration (30 minutes, 1 hour, 2 hours) are factors needed to be considered.

### Description of subject (S) and tissue sample (P):

A subject (S), which may be applied to all following paragraphs, may be a human body or entity, such as child, teenager, man, woman, or graybeard. Alternatively, the subject (S) may be a non-human body or entity (creature), such as pet animal, farm animal, experimental animal, or disease-model animal. Examples of the pet, farm, experimental or disease-model animal are as below: primate (e.g., monkey or gorilla), dog, rodent (e.g., mouse or guinea pig), cat, horse, cow, cattle, sheep, pig, chicken, duck, goose, bird, elephant, frog, and fish.

A tissue sample (P) containing various cells may be extracted, taken, obtained or derived from a bodily fluid or solid tissue of the subject (S). The bodily fluid of the subject (S) may be blood (e.g., peripheral blood) or bone marrow. In the case of the subject (S) being a human body or entity, the bodily fluid may be human bone marrow or human blood such as peripheral blood. Peripheral blood is the flowing, circulating blood of the body. The solid tissue of the subject (S) may be nerve, muscle, skin, gut, bone, kidney, liver, pancreas, thymus, or adipocyte. The tissue sample (P) may be a bodily fluid sample (e.g., peripheral-blood sample, whole-blood sample, or bone-marrow sample) when the tissue sample (P) is obtained from the bodily fluid of the subject (S). Whole blood may be collected from peripheral blood of the subject (S) such as human body or entity) and then typically combined with an anticoagulant, but is generally otherwise unprocessed. The tissue sample (P) may be a solid tissue sample (e.g., muscle sample, adipocyte sample, or fat tissue) when the tissue sample (P) is obtained from the solid tissue of the subject (S). In the case of the subject (S) being a human body or entity, the tissue sample (P) may be a human bone-marrow sample or a human blood sample such as peripheral-blood sample or whole-blood sample.

### Description of test or measurement (M0):

When a tissue sample extracted, taken, obtained or derived from a subject that may be referred to the above-mentioned subject (S) is a blood sample such as peripheral-blood sample or whole-blood sample, the test or measurement (M0) may be performed to obtain results or data related to one or more types of the above-mentioned stem cells from the blood sample by steps of performing a pre-assay preparation (Y0) and then performing an assay process (T0). The above results or data may include the number of stem cells of each type.

The pre-assay preparation (Y0) may be performed in one of the following two approaches to treat or process the blood sample from the subject. In a first approach, a blood sample is obtained or prepared by adding a cocktail (e.g., RosetteSep^{®} Human Progenitor Enrichment Cocktail) to a tube containing whole blood (e.g., adding 100 µL of the Human Progenitor Enrichment Cocktail to 2 mL of whole blood) and then mixing the whole blood and the cocktail well. The whole blood is obtained from the subject. Next, the blood sample is incubated at room temperature (e.g., about 27 degrees Celsius) for a suitable time period (e.g., about 20 minutes). After incubation, the blood sample is diluted with a first medium (e.g., 2% fetal bovine serum (FBS)/phosphate-buffered saline (PBS)) and then mixed gently so as to form a diluted sample. Next, the diluted sample is poured into a tube containing a reagent such as Ficoll-Paque^{®} slowly so as to be prevented from mixing with the reagent and to be overlaid or layered on top of the reagent. Next, by centrifuging the mixture of the diluted sample and the reagent at a suitable rotational speed (e.g., 3,000 rpm) for a suitable time period (e.g., 20 minutes) at room temperature (e.g., about 27 degrees Celsius), four distinct layers are formed. The four distinct layers may be, from the top to the bottom: a first layer of plasma, a second layer of multi-type cells, a third layer of the reagent, and a fourth layer of red blood cells. Next, the first and second layers and the top half of the third layer are transferred into a tube and then centrifuged at a suitable rotational speed (e.g., 3,000 rpm) for a suitable time period (e.g., 20 minutes) so as to from a centrifuged sample. Next, the supernatant of the centrifuged sample is discarded, and the multi-type cells of the centrifuged sample are re-suspended in a second medium (e.g., 2% FBS/PBS). Next, a suitable buffer (e.g., 3X red-blood-cell (RBC) lysis buffer) is added to the second medium. After being incubated in the second medium at room temperature for a suitable time period (e.g., 10 minutes), the multi-type cells are washed with a third medium (e.g., 2% FBS/PBS) twice and then re-suspended in a fourth medium (e.g., 1% or 2% bovine serum albumin (BSA) in PBS) so as to obtain a mixture including the multi-type cells and the fourth medium. 1% BSA in PBS is made by dissolving 0.1g of BSA into 10mL of PBS; 2% BSA in PBS is made by dissolving 0.2g of BSA into 10mL of PBS. Next, a stain with appropriate antibodies may be added to the mixture including the multi-type cells and the fourth medium so as to form a test sample, which may be used in the following assay process (T0).

In a second approach, a blood sample is obtained or prepared by adding one volume of 6% Hetastarch to five volumes of whole blood and then incubated at room temperature (e.g., about 27 degrees Celsius) for a suitable time period (e.g., at least 30 minutes). The whole blood is obtained from the subject. The 6% Hetastarch is a solution containing 6% of Hetastarch in a phosphate-buffered saline (PBS), wherein every 100 mL PBS contains 6g of Hetastarch. Once two separate layers are formed in the blood sample, the top layer (i.e., cell layer) of the blood sample is pipetted out into a tube without disturbance of the underlying layer (i.e., red-blood-cell (RBC) layer) of the blood sample. Next, the content in the tube is centrifuged at a suitable rotational speed (e.g., 3,000 rpm) for a suitable time period (e.g., 15 minutes) so as to obtain a centrifuged sample. After centrifugation, the supernatant of the centrifuged sample is discarded, and then the pellets from the centrifuged sample are washed with a suitable salt solution (e.g., PBS). Next, the pellets are re-suspended in a suitable medium (e.g., 1% or 2% BSA in PBS) so as to obtain a mixture including the pellets and the suitable medium. Next, a stain with appropriate antibodies may be added to the mixture including the pellets and the suitable medium so as to form a test sample, which may be used in the following assay process (T0).

After the pre-assay preparation (Y0) is performed, the assay process (T0) is performed to run and analyze the test sample produced by the first or second approach of the pre-assay preparation (Y0) so as to obtain a set of results or data related to one or more types of stem cells from the blood sample descriptively, qualitatively or quantitatively. Here the one or more types of stem cells may include one or more of the following ones: at least one type of pluripotent stem cells (e.g., SB-1 cells, SB-2 cells, BLSCs, VSELs, any other type of pluripotent stem cells, and/or a group of selected two or more than two types of pluripotent stem cells), at least one type of multipotent stem cells (e.g., MSCs, any other type of multipotent stem cells, and/or a group of selected two or more than two types of multipotent stem cells), hematopoietic stem cells (HSCs), and at least one type of progenitor stem cells. The assay process (T0) may be flow cytometry. The flow cytometry may be employed to analyze many parameters of the blood sample using a flow cytometer, which is a type of assay device, to perform steps of cell counting, sorting, and biomarker detecting. The flow cytometer may be used to analyze the positive expression of specific markers of the blood sample. Using the flow cytometry, the set of results or data related to the one or more types of the above-mentioned stem cells may include types of stem cells and parameters, such as the number, the percentage and/or the sizes, of the one or more types of the above-mentioned stem cells.

### Description of test or measurement (M1):

The test or measurement (M1) may be performed by steps of performing a purification process (Y1) and then performing an assay process (T1). The purification process (Y1) may be performed in one of the following three approaches to treat or process a tissue sample from a subject that may be referred to the above-mentioned subject (S). The tissue sample may be referred to the above-mentioned tissue sample (P). For example, the tissue sample is a bodily fluid sample (e.g., peripheral-blood sample, whole-blood sample, or bone-marrow sample) when the tissue sample is obtained from a bodily fluid (e.g., human blood, peripheral blood, or bone marrow) of the subject. Alternatively, the tissue sample is a solid tissue sample (e.g., muscle sample or adipocyte sample) when the tissue sample is obtained from a solid tissue (e.g., muscle or adipocyte) of the subject. If the tissue sample is obtained from the solid tissue of the subject, collagenase shall be added to treat the tissue sample for at least 6 or 8 hours at a suitable temperature (e.g., about 37 °C, greater than 30 °C, between 35 °C and 39 °C, or between 30 °C and 40 °C) prior to the purification process (Y1).

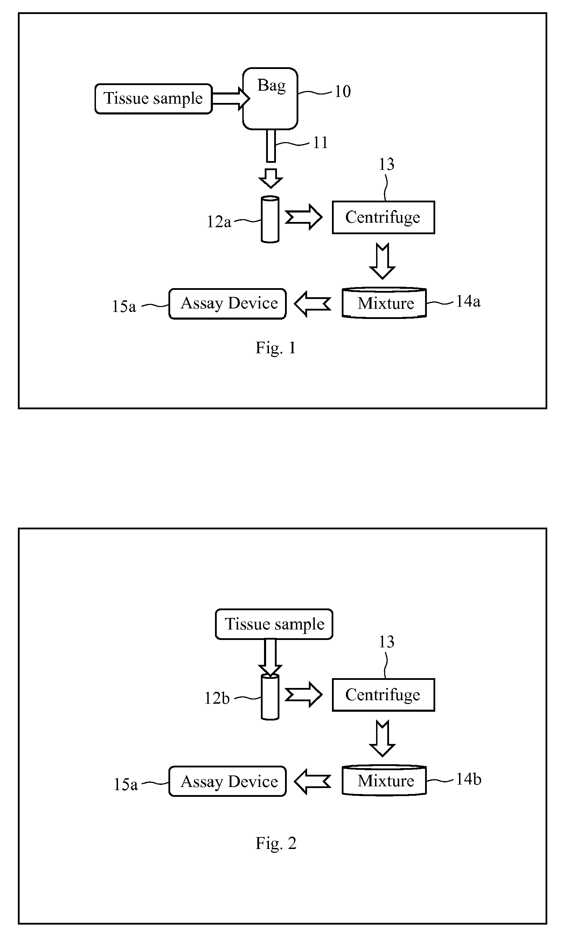

In a first approach, referring to Fig. 1, the tissue sample is transferred to a bag 10 that contains an anti-clotting reagent such as divalent cation chelating agent. The divalent cation chelating agent may be an ethylenediaminetetraacetic acid (EDTA). A filter 11 is attached to the bottom of the bag 10 and used to ensure that only small cells flow out of the bag 10 and are collected in a tube 12a. The small cells flowing through the filter 11 are smaller than or equal to 4, 5 or 6 micrometers, such as between 0.1 and 6.0 micrometers, between 0.5 and 6.0 micrometers, between 1.0 and 6.0 micrometers, between 0.1 and 5.0 micrometers, between 0.5 and 5.0 micrometers, between 1.0 and 5.0 micrometers, between 0.1 and 4.0 micrometers, between 0.5 and 4.0 micrometers or between 1.0 and 4.0 micrometers, in size (as defined by the above-mentioned size (Z) of a cell). Next, the tube 12a is centrifuged by a centrifuge 13 at a suitable centrifugal force (e.g., about 1,000 g, greater than 700 g, greater than 1,300 g, between 500 g and 2,500 g, or between 800 g and 1,600 g) for a suitable time period (e.g., about 18 minutes, longer than 12 minutes, longer than 16 minutes, between 10 minutes and 30 minutes, or between 15 minutes and 22 minutes) to isolate the small cells. Next, the small cells are placed in a medium, including phosphate-buffered saline (PBS) and bovine serum albumin (BSA) for example, and re-suspended in the medium so as to obtain a test sample 14a including the small cells and the medium. The test sample 14a may be used in the following assay process (T1).

In a second approach, referring to Fig. 2, if the tissue sample is obtained from the solid tissue, the tissue sample treated with the collagenase is placed into a tube 12b. Next, the tube 12b is centrifuged by the centrifuge 13 at a low speed of a suitable centrifugal force (e.g., about 100 g, between 50 g and 450 g, or between 80 g and 250 g) for a suitable time period (e.g., about 10 minutes, longer than 8 minutes, longer than 12 minutes, between 8 minutes and 15 minutes, or between 9 minutes and 20 minutes) and then at a high speed of a suitable centrifugal force (e.g., about 1,000 g, greater than 700 g, greater than 1,300 g, between 500 g and 2,500 g, or between 800 g and 1,600 g) for a suitable time period (e.g., about 18 minutes, longer than 12 minutes, longer than 16 minutes, between 10 minutes and 30 minutes, or between 15 minutes and 22 minutes) so as to isolate small cells from the tissue sample. The isolated small cells are smaller than or equal to 4, 5 or 6 micrometers, such as between 0.1 and 6.0 micrometers, between 0.5 and 6.0 micrometers, between 1.0 and 6.0 micrometers, between 0.1 and 5.0 micrometers, between 0.5 and 5.0 micrometers, between 1.0 and 5.0 micrometers, between 0.1 and 4.0 micrometers, between 0.5 and 4.0 micrometers or between 1.0 and 4.0 micrometers, in size (as defined by the above-mentioned size (Z) of a cell). Next, the small cells are placed in a medium, including PBS and BSA for example, and re-suspended in the medium so as to obtain a test sample 14b including the small cells and the medium. The test sample 14b may be used in the following assay process (T1).

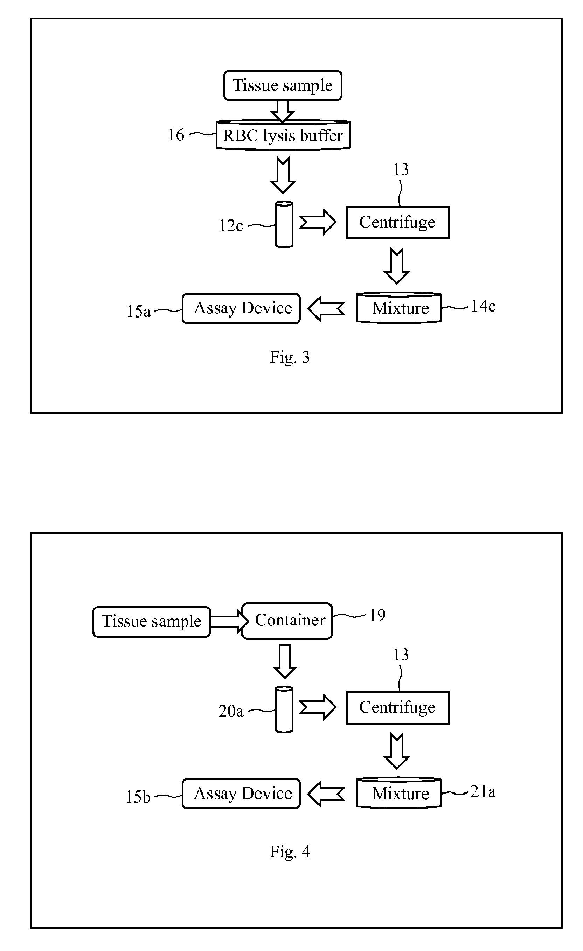

In a third approach, referring to Fig. 3, if the tissue sample is obtained from the bodily fluid, the tissue sample is incubated with a red-blood-cell (RBC) lysis buffer 16 for a suitable time period (e.g., about 10 minutes, longer than 8 minutes, longer than 12 minutes, between 8 minutes and 15 minutes, or between 9 minutes and 20 minutes) in order to eliminate substantially all of red blood cells. Next, the mixture including the tissue sample and the buffer 16 is poured into a tube 12c. Next, the tube 12c is centrifuged by the centrifuge 13 at a low speed of a suitable centrifugal force (e.g., about 100 g, between 50 g and 450 g, or between 80 g and 250 g) for a suitable time period (e.g., about 10 minutes, longer than 8 minutes, longer than 12 minutes, between 8 minutes and 15 minutes, or between 9 minutes and 20 minutes) and then at a high speed of a suitable centrifugal force (e.g., about 1,000 g, greater than 700 g, greater than 1,300 g, between 500 g and 2,500 g, or between 800 g and 1,600 g) for a suitable time period (e.g., about 18 minutes, longer than 12 minutes, longer than 16 minutes, between 10 minutes and 30 minutes, or between 15 minutes and 22 minutes) so as to isolate small cells from the mixture including the tissue sample and the buffer 16. The isolated small cells are smaller than or equal to 4, 5 or 6 micrometers, such as between 0.1 and 6.0 micrometers, between 0.5 and 6.0 micrometers, between 1.0 and 6.0 micrometers, between 0.1 and 5.0 micrometers, between 0.5 and 5.0 micrometers, between 1.0 and 5.0 micrometers, between 0.1 and 4.0 micrometers, between 0.5 and 4.0 micrometers or between 1.0 and 4.0 micrometers, in size (as defined by the above-mentioned size (Z) of a cell). Next, the small cells are placed in a medium, including PBS and BSA for example, and re-suspended in the medium so as to obtain a test sample 14c including the small cells and the medium. The test sample 14c may be used in the following assay process (T1).

After the purification process (Y1) is performed, an assay device 15a, such as flow cytometer or RT-PCR device, as shown in Fig. 1, 2 or 3 is used to perform the assay process (T1) to analyze the test sample 14a, 14b or 14c so as to obtain a set of results or data of one or more types of small stem cells from the tissue sample descriptively, qualitatively or quantitatively. The set of results or data of the one or more types of small stem cells may include types of small stem cells and parameters, such as the number, the percentage and/or the sizes, of the one or more types of small stem cells. Here the one or more types of small stem cells may include one or more of the following ones: at least one type of pluripotent stem cells (e.g., SB-1 cells, SB-2 cells, BLSCs, VSELs, any other type of pluripotent stem cells, and/or a group of selected two or more than two types of pluripotent stem cells), at least one type of multipotent stem cells (e.g., MSCs, any other type of multipotent stem cells, and/or a group of selected two or more than two types of multipotent stem cells), hematopoietic stem cells (HSCs), and/or at least one type of progenitor stem cells, which may be smaller than or equal to 4, 5 or 6 micrometers, such as between 0.1 and 6.0 micrometers, between 0.5 and 6.0 micrometers, between 1.0 and 6.0 micrometers, between 0.1 and 5.0 micrometers, between 0.5 and 5.0 micrometers, between 1.0 and 5.0 micrometers, between 0.1 and 4.0 micrometers, between 0.5 and 4.0 micrometers or between 1.0 and 4.0 micrometers, in size (as defined by the above-mentioned size (Z) of a cell).

The assay process (T1) may be achieved by flow cytometry or reverse transcription polymerase chain reaction (RT-PCR) analysis such as real-time RT-PCR analysis. The flow cytometry may be employed to analyze many parameters of a sample using a flow cytometer, which is a type of assay device 15a, to perform steps of cell counting, sorting, and biomarker detecting. The flow cytometer 15a may be used to analyze or look at the positive expression of specific cell (surface) markers of the sample. The RT-PCR analysis may be employed to detect and quantify gene expression in stem cells of a sample by using a (real-time) RT-PCR device, which is a type of assay device 15a. The RT-PCR results reveal that stem cells express various genes that are associated with activated stem cells, such as Oct4, Nanog, Nestin and alpha-feto protein.

### Description of test or measurement (M2):

The test or measurement (M2) may be performed by steps of performing a purification process (Y2) and then performing an assay process (T2). The purification process (Y2) may be performed in one of the following three approaches to treat or process a tissue sample from a subject that may be referred to the above-mentioned subject (S). The tissue sample may be referred to the above-mentioned tissue sample (P). For example, the tissue sample is a bodily fluid sample (e.g., peripheral-blood sample, whole-blood sample, or bone-marrow sample) when the tissue sample is obtained from a bodily fluid (e.g., human blood, peripheral blood, or bone marrow) of the subject. Alternatively, the tissue sample is a solid tissue sample (e.g., muscle sample or adipocyte sample) when the tissue sample is obtained from a solid tissue (e.g., muscle or adipocyte) of the subject. If the tissue sample is obtained from the solid tissue of the subject, collagenase shall be added to treat the tissue sample for at least 6 or 8 hours at a suitable temperature (e.g., about 37 °C, greater than 30 °C, between 35 °C and 39 °C, or between 30 °C and 40 °C) prior to the purification process (Y2).

In a first approach, referring to Fig. 4, the tissue sample is transferred to a container or bag 19 containing an anti-clotting reagent such as divalent cation chelating agent (e.g., EDTA) so as to obtain a mixture including the tissue sample and the anti-clotting reagent. The mixture is then poured into a tube 20a. Next, the tube 20a is centrifuged by a centrifuge 13 at a suitable centrifugal force (e.g., about 1,000 g, greater than 700 g, greater than 1,300 g, between 500 g and 2,500 g, or between 800 g and 1,600 g) for a suitable time period (e.g., about 18 minutes, longer than 12 minutes, longer than 16 minutes, between 10 minutes and 30 minutes, or between 15 minutes and 22 minutes) so as to isolate all cells including small and large cells. The small cells may be smaller than or equal to 4, 5 or 6 micrometers, such as between 0.1 and 6.0 micrometers, between 0.5 and 6.0 micrometers, between 1.0 and 6.0 micrometers, between 0.1 and 5.0 micrometers, between 0.5 and 5.0 micrometers, between 1.0 and 5.0 micrometers, between 0.1 and 4.0 micrometers, between 0.5 and 4.0 micrometers or between 1.0 and 4.0 micrometers, in size (as defined by the above-mentioned size (Z) of a cell). The large cells may be greater than 6 micrometers in size (as defined by the above-mentioned size (Z) of a cell). Next, the cells including the small and large cells are placed in a medium, including PBS and BSA for example, and re-suspended in the medium so as to obtain a test sample 21a containing the medium and the cells including the small and large cells. The test sample 21 a may be used in the following assay process (T2).

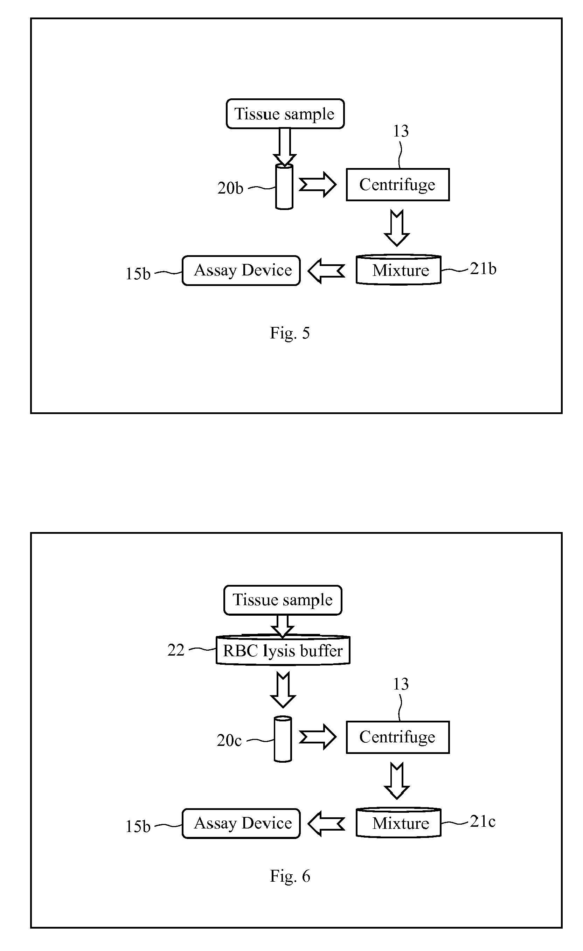

In a second approach, referring to Fig. 5, if the tissue sample is obtained from the solid tissue, the tissue sample treated with the collagenase is placed into a tube 20b. Next, the tube 20b is centrifuged by the centrifuge 13 at a high speed of a suitable centrifugal force (e.g., about 1,000 g, greater than 700 g, greater than 1,300 g, between 500 g and 2,500 g, or between 800 g and 1,600 g) for a suitable time period (e.g., about 18 minutes, longer than 12 minutes, longer than 16 minutes, between 10 minutes and 30 minutes, or between 15 minutes and 22 minutes) so as to isolate all cells including the small and large cells as defined in the first approach of the purification process (Y2). Next, the cells including the small and large cells are placed in a medium, including PBS and BSA for example, and re-suspended in the medium so as to obtain a test sample 21b containing the medium and the cells including the small and large cells. The test sample 21b may be used in the following assay process (T2).

In a third approach, referring to Fig. 6, if the tissue sample is obtained from the bodily fluid, the tissue sample is incubated with a RBC lysis buffer 22 for a suitable time period (e.g., about 10 minutes, longer than 8 minutes, longer than 12 minutes, between 8 minutes and 15 minutes, or between 9 minutes and 20 minutes) in order to eliminate substantially all of red blood cells. Next, the mixture including the tissue sample and the buffer 22 is poured into a tube 20c. Next, the tube 20c is centrifuged by the centrifuge 13 at a high speed of a suitable centrifugal force (e.g., about 1,000 g, greater than 700 g, greater than 1,300 g, between 500 g and 2,500 g, or between 800 g and 1,600 g) for a suitable time period (e.g., about 18 minutes, longer than 12 minutes, longer than 16 minutes, between 10 minutes and 30 minutes, or between 15 minutes and 22 minutes) so as to isolate all cells including the small and large cells as defined in the first approach of the purification process (Y2). Next, the cells including the small and large cells are placed in a medium, including PBS and BSA for example, and re-suspended in the medium so as to obtain a test sample 21c containing the medium and the cells including the small and large cells. The test sample 21c may be used in the following assay process (T2).

After the purification process (Y2) is performed, an assay device 15b, such as flow cytometer or RT-PCR device, as shown in Fig. 4, 5 or 6 is used to perform the assay process (T2) to analyze the test sample 21a, 21b or 21c so as to obtain a set of results or data of one or more types of stem cells from the tissue sample descriptively, qualitatively or quantitatively. The set of results or data of the one or more types of stem cells from the tissue sample include types of stem cells and parameters, such as the number, the percentage and/or the sizes, of the one or more types of stem cells. The one or more types of stem cells from the tissue sample may be or may include the one or more types of small stem cells as mentioned or analyzed in the above assay process (T1) and/or one or more types of large stem cells, such as at least one type of multipotent stem cells (e.g., some types of MSCs), which may be greater than 6 micrometers in size (as defined by the above-mentioned size (Z) of a cell).

The assay process (T2) may be achieved by flow cytometry or RT-PCR analysis (e.g., real-time RT-PCR analysis). The flow cytometry may be employed to analyze many parameters of a sample using a flow cytometer, a type of assay device 15b, to perform steps of cell counting, sorting, and biomarker detecting. The flow cytometer 15b may be used to analyze or look at the positive expression of specific cell (surface) markers of the sample. The RT-PCR analysis may be employed to detect and quantify gene expression in stem cells of a sample by using a (real-time) RT-PCR device, a type of assay device 15b. The RT-PCR results reveal that stem cells express various genes that are associated with activated stem cells, such as Oct4, Nanog, Nestin and alpha-feto protein.

All terms, specifications, definitions, methods, materials, processes, equipments, procedures described and mentioned in the previous paragraphs are applied to all the following embodiments in the following paragraphs. For example, in the following paragraphs, the term "size" may be referred to as the above-mentioned size (Z), the term "subject" may be referred to as the above-mentioned subject (S), and the term "tissue sample" may be referred to as the above-mentioned tissue sample (P).

### Embodiments:

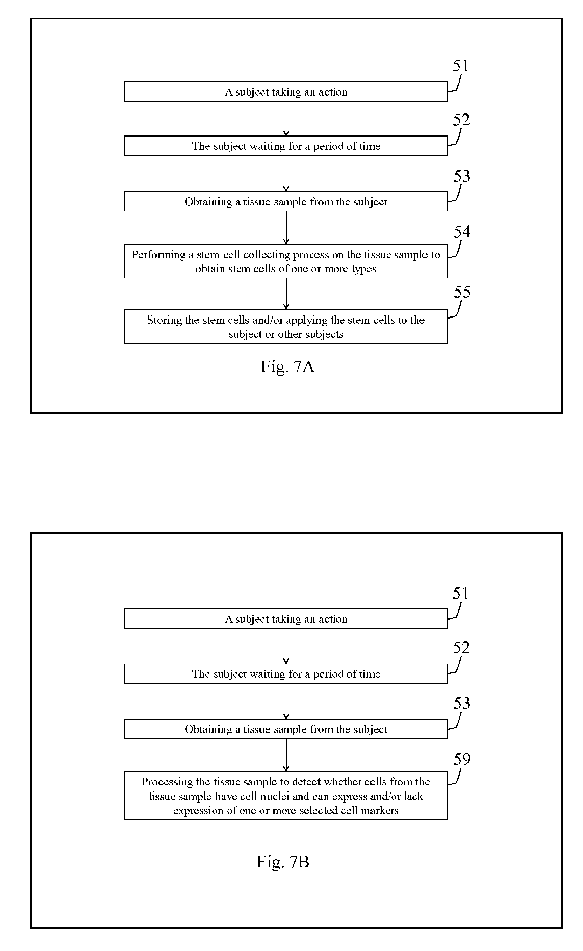

A method of obtaining, collecting or harvesting a large amount of stem cells from a living being (such as a human body or an animal body) is described below. Fig. 7A is a flow chart of obtaining, collecting or harvesting a large number of stem cells from a tissue sample extracted, taken, obtained or derived from a subject according to an embodiment of the present disclosure. The stem cells are described and mentioned in the paragraphs in "Description of stem cells". Referring to Fig. 7A, in step 51, the subject takes or is subjected to one or more of the above-mentioned actions or stimuli (X), such as taking or ingesting a nutrient or dietary supplement (e.g., a brown algae supplement containing 60 percent or more of fucoidan or oligo fucoidan in weight, or one or more pills of a brown algae supplement as mentioned in the following first experiment and Fig. 11), and/or taking one or more drugs approved by a government department (e.g., U.S. FDA), and/or taking a herb medicine or a Chinese medicine. The subject (i.e., the living being) may be referred to the above-mentioned subject (S), such as human being or animal (e.g., dog, cat, pig, chicken, duck, or cattle). The one or more actions or stimuli in the step 51 may be performed with controlling the dose, intensity, duration, frequency (for example, taking one pill of nutrient or dietary supplement or drug for three times with one hour apart), and/or the time (for example, the time of a day (in the morning, at noon, in the afternoon, in the evening, or in the night), the time before, with or after the meal, or the time of the year (spring, summer, autumn or winter)). As an example, when the subject is taking a drug or a supplement (e.g., the brown algae fucoidan supplement), the dose (for example, the amount in grams), the time (for example, before or after breakfast, or before sleep) are factors to be considered or controlled. The one or more actions or stimuli in the step 51 can increase one or more specific types of stem cells of interest in peripheral blood of the subject after having taken or been subjected to the one or more actions or stimuli in the step 51 for a period, wherein the one or more specific types of stem cells of interest may be referred to the paragraphs in "Description of stem cells".

In step 52, after taking or being subjected to the one or more of the above-mentioned actions or stimuli (X), the subject waits for a (predetermined) time interval, such as between 15 minutes and 60 minutes, between 20 minutes and 100 minutes, between 30 minutes and 2 hours, between 0.5 hours and 3 hours, between 1 hour and 12 hours, between 12 hours and 36 hours, or between 36 hours and 50 hours. With the steps 51-52, stem cell increase or expansion may be achieved in vivo. Next, in step 53, a tissue sample is extracted, taken, obtained or derived from the subject. The tissue sample may be referred to the above-mentioned tissue sample (P), such as peripheral-blood sample. Next, in step 54, a stem-cell obtaining, collecting or harvesting process or method is performed on the tissue sample to obtain, collect or harvest a large number of stem cells of one or more types mentioned in the paragraphs in "Description of stem cells" from the tissue sample. For example, a large number of the SB-1 cells and/or SB-2 cells may be obtained, collected or harvested by performing the stem-cell obtaining, collecting or harvesting process or method on the tissue sample.

Next, in step 55, the stem cells of the one or more types obtained, collected or harvested from the tissue sample through the stem-cell obtaining, collecting or harvesting process or method may be stored in a temperature of lower than 0 degrees Celsius, such as lower than -30 degrees Celsius or lower than -80 degrees Celsius, in a suitable facility (e.g., refrigerator) provided by an organization (e.g., cell bank) for a period of time (for example, longer than 1 month or longer than 1 year) for later purposes (PS) mentioned in the following paragraph. The stored stem cells may be applied to the subject or another subject for the purposes (PS) in the future, for example one, three or six months later or one, two, five or even ten years later after the stem cells are obtained, collected or harvested. The other subject herein may be a species in the same biological category as the subject and may have the same blood type, e.g., blood type A, B, O or AB, as the subject.

The stored stem cells may be used for the purposes (PS) such as stem cell therapies, anti-ageing treatments or products, reverse ageing treatments or products, trauma treatments or products, face-lift treatments or products, plastic surgery, invasive medical-aesthetic treatments, non-invasive medical-aesthetic treatments or products, reconstructive plastic surgery, cancer treatments, degenerative disease treatments, autoimmune disease treatments, teeth treatments, dental (tooth) implant surgery, burn recovery treatments, knee meniscus injury treatments, arthritis treatments, knee arthritis treatments, bone injury treatments, bone fracture treatments, tissue repair treatments, tissue or organ cloning, reproductive cloning, therapeutic cloning, cosmetics, skin care products, facial masks or masques, feed additives, nutrients, or supplements. In one example, the stored stem cells may be applied to, such as injected into, or spread over or in, a tissue or organ of the subject or the other subject, such as skin, bone, joint, tooth, liver or kidney. In another example, the stored stem cells may be mixed with some buffers, such as salts, and the stored stem cells mixed with the buffers may be applied to, such as injected into, or spread over or in, a tissue or organ of the subject or the other subject, such as skin, bone, joint, tooth, liver or kidney.

In an alternative embodiment, the stem cells of the one or more types obtained, collected or harvested from the tissue sample through the stem-cell obtaining, collecting or harvesting process or method may be used for the above-mentioned purposes (PS) with omission of the above-mentioned step of storing the obtained, collected or harvested stem cells of the one or more types in the temperature of lower than 0 degrees Celsius in the suitable facility. Alternatively, the stem cells of the one or more types obtained, collected or harvested from the tissue sample through the stem-cell obtaining, collecting or harvesting process or method may be applied to, such as injected into, or spread over or in, a tissue or organ of the subject or another subject, such as skin, bone, joint, tooth, liver or kidney, with omission of the above-mentioned step of storing the obtained, collected or harvested stem cells of the one or more types in the temperature of lower than 0 degrees Celsius in the suitable facility. The other subject herein may be a species in the same biological category as the subject and may have the same blood type as the subject.

In an alternative embodiment, the tissue sample obtained in the step 53 may be stored in a temperature of lower than 0 degrees Celsius, such as lower than -30 degrees Celsius or lower than -80 degrees Celsius, in a cell bank or a suitable cold storage device such as refrigerator or freezer. After stored for a time interval, such as one month, three months, six months, one year, two years, five years, or ten years, the tissue sample is taken out from the cold storage device and processed by a stem-cell obtaining, collecting or harvesting process or method to obtain, collect or harvest one or more types of stem cells as mentioned in the paragraphs in "Description of stem cells". For example, the SB-1 cells and/or SB-2 cells may be obtained, collected or harvested by performing the stem-cell obtaining, collecting or harvesting process or method on the tissue sample. Next, the stem cells of the one or more types obtained, collected or harvested from the tissue sample through the stem-cell obtaining, collecting or harvesting process or method may be (1) used for the purposes (PS) mentioned above, or (2) applied to, such as injected into, or spread over or in, a tissue or organ of the subject or another subject, such as skin, bone, joint, tooth, liver or kidney. The other subject herein may be a species in the same biological category as the subject and may have the same blood type as the subject.

In an alternative embodiment, the tissue sample obtained in the step 53 may be stored in a temperature of lower than 0 degrees Celsius, such as lower than -30 degrees Celsius or lower than -80 degrees Celsius, in a cell bank or a suitable cold storage device such as refrigerator or freezer. Here the tissue sample contains a type or selected types of stem cells as mentioned in the paragraphs in "Description of stem cells", such as SB-1 cells and/or SB-2 cells. After stored for a time interval, such as one month, three months, six months, one year, two years, five years, or ten years, the tissue sample is taken out from the cold storage device and then may be (1) used for the purposes (PS) mentioned above, or (2) applied to, such as injected into, or spread over or in, a tissue or organ of the subject or another subject, such as skin, bone, joint, tooth, liver or kidney. The other subject herein may be a species in the same biological category as the subject and may have the same blood type as the subject.

In an alternative embodiment, the tissue sample obtained in the step 53, without stored at (very) low temperature in a cell bank or a cold storage device such as refrigerator or freezer for a period of time (for example, longer than 1 month or longer than 1 year), may be (1) used for the purposes (PS) mentioned above, or (2) applied to, such as injected into, or spread over or in, a tissue or organ of the subject or another subject, such as skin, bone, joint, tooth, liver or kidney. The other subject herein may be a species in the same biological category as the subject and may have the same blood type as the subject. Here the tissue sample contains a type or selected types of stem cells as mentioned in the paragraphs in "Description of stem cells", such as SB-1 cells and/or SB-2 cells.

In an alternative embodiment, the tissue sample obtained in the step 53 may be used to discover or detect a new type or types of stem cells that are not yet identified or found. The following description in this embodiment uses the tissue sample obtained from the peripheral blood of the subject as an example. Before the subject takes or is subjected to the one or more of the actions or stimuli (X) in the step 51, the new type or types of stem cells may not exist, or may rarely exist (that means with a very low or un-detectable amount of quantity), in the peripheral blood of the subject so that the new type or types of stem cells cannot be found or are difficult to be found using a flow cytometer or other measurement tools. After the subject performs the steps 51 and 52, the new type or types of stem cells are increased, activated, mobilized, generated, or stimulated in the peripheral blood of the subject. Therefore, the new type or types of stem cells can be easily found using a flow cytometer or other measurement tools, as illustrated in the following step 59.

The new type or types of stem cells can be discovered or detected using methods of discovering or detecting a new type of stem cells. Fig. 7B shows a flow chart of discovering or detecting a new type or types of stem cells. Referring to Fig. 7B, after the above-mentioned steps 51-53 are performed in sequence, step 59 is performed to process the tissue sample (such as peripheral-blood sample) obtained in the step 53 using a analysis method (for example, the above-mentioned test or measurement (M0), (M1) or (M2)) for detecting the new type or types of stem cells. The analysis method includes using procedures to detect whether cells from the tissue sample have cell nuclei and can express and/or lack expression of one or more selected cell (surface) markers.

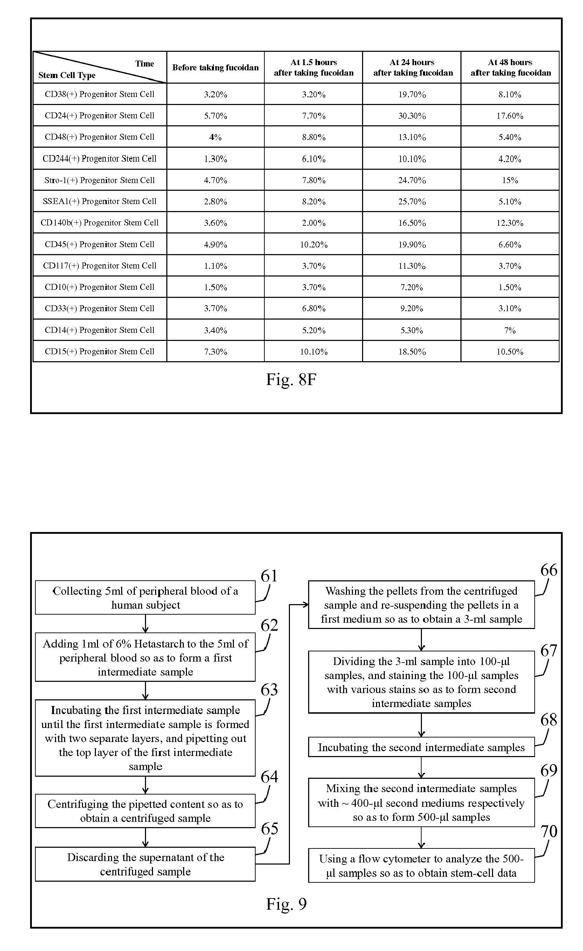

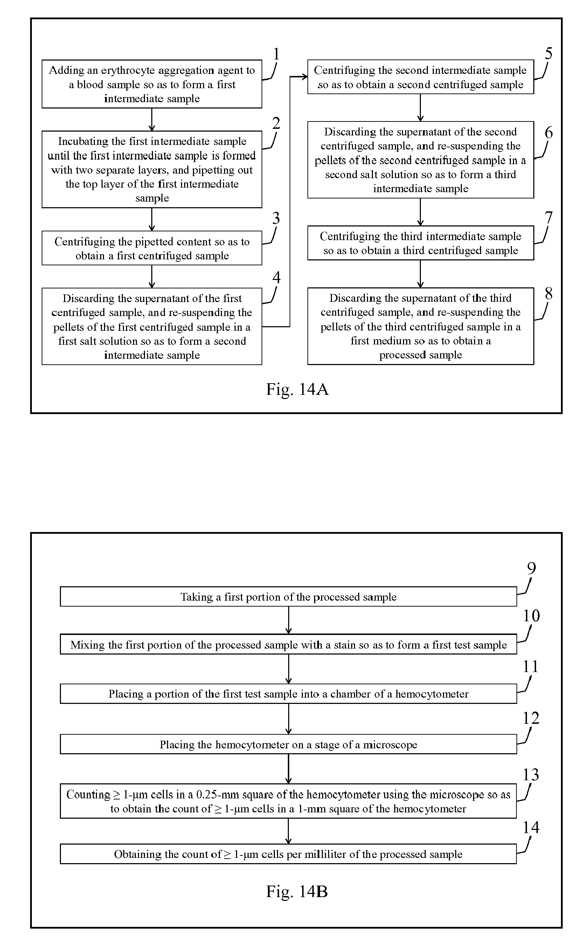

In an alternative embodiment, the tissue sample obtained in the step 53 of Fig. 7A may be used to obtain stem-cell data related to the subject after taking or being subjected to the one or more of the actions or stimuli (X) in the step 51 (hereinafter the "Data A") by, e.g., the aforementioned test or measurement (M0), (M1) or (M2), the following steps 61-70 in Fig. 9, the following steps 1-20 in Figs. 14A-14C, the following integrated system, apparatus, device or tool (AD), or the following single piece of system, device, tool or apparatus (TD). The Data A may include the numbers of stem cells of various specific types, the percentages of various specific types of stem cells, and/or the count of one or more types of stem cells per milliliter of the tissue sample obtained in the step 53 (i.e., the count per unit volume of the one or more types of stem cells in the tissue sample obtained in the step 53). These types of stem cells may be referred to the paragraphs in "Description of stem cells". For example, the Data A may include the number or percentage of the SB-1 cells, the number or percentage of the SB-2 cells, the number or percentage of the BLSCs, the number or percentage of the VSELs, the number or percentage of the MSCs, the number or percentage of the HSCs, the number or percentage of one or more types of pluripotent, multipotent and/or progenitor stem cells, and/or the count per unit volume of one or more types of pluripotent, multipotent and/or progenitor stem cells in the tissue sample obtained in the step 53. The Data A may be used for clinical diagnosis or used to monitor, determine, identify or evaluate disease stage, treatment progress, health conditions and/or physiological conditions of the subject.

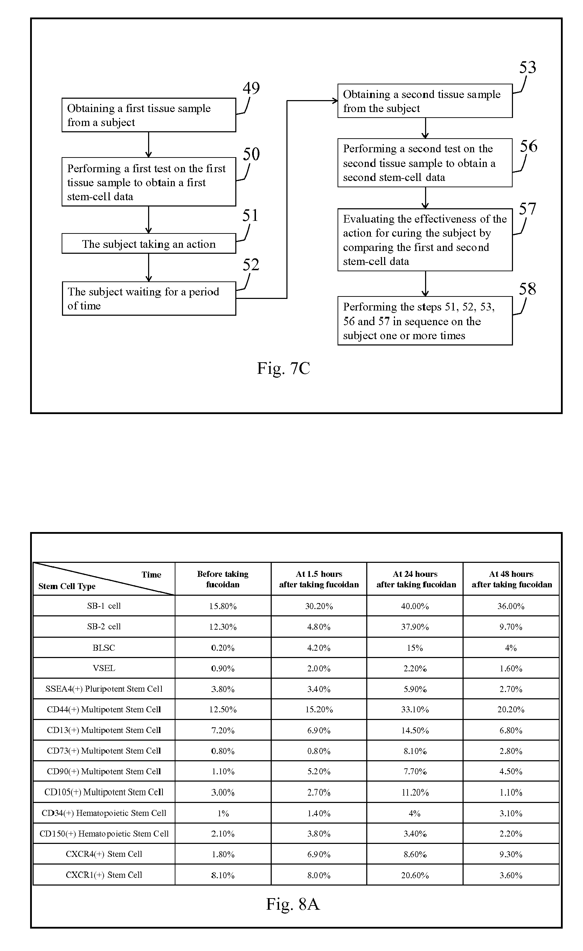

Alternatively, the Data A may be used to evaluate or identify the effectiveness of the one or more actions or stimuli in the step 51 for curing or treating the subject, who has a specific disease in this case, by controlling the dose, intensity, duration, frequency, and/or the time of the one or more actions or stimuli in the step 51. The specific disease may be prostate cancer, liver cancer, gastric cancer, oral cancer, colon cancer, breast cancer, lung cancer, nasopharyngeal carcinoma, cervical cancer, skin cancer, esophageal cancer, bladder cancer, pancreatic cancer, endometrial cancer, ovarian cancer, gallbladder cancer, thyroid carcinoma, leukemia, lymphoma, Non-Hodgkin lymphoma, melanoma, parkinsonism, Alzheimer's disease, dementia, diabetes mellitus, allergic rhinitis, brain tumor, or acquired immunodeficiency syndrome (AIDS). In this case, stem-cell data related to the subject before taking or being subjected to the one or more of the actions or stimuli (X) in the step 51 (hereinafter the "Data B") may need to be obtained. Then, by comparing the Data A and the Data B, the effectiveness of the one or more actions or stimuli depicted in the step 51 for curing or treating the subject having the specific disease may be identified, determined or evaluated. Fig. 7C shows a flow chart of identifying, determining or evaluating the effectiveness of the one or more actions or stimuli depicted in the step 51 for curing or treating the subject having the specific disease according to this case.

Referring to Fig. 7C, in step 49, a tissue sample (hereinafter the "first tissue sample") is extracted, taken, obtained or derived from the subject. The first tissue sample may be referred to the above-mentioned tissue sample (P). Next, in step 50, a first test or measurement including assessing and/or measuring stem cells is performed on the first tissue sample to obtain first stem-cell data (i.e., the Data B) related to one or more types of stem cells from the first tissue sample descriptively or quantitatively. For more detailed information on the first test or measurement, please refer to the above-mentioned test or measurement (M0), (M1) or (M2), the following steps 61-70 in Fig. 9, or the following steps 1-20 in Figs. 14A-14C. Alternatively, the first stem-cell data may be obtained by the following integrated system, apparatus, device or tool (AD) or the following single piece of system, device, tool or apparatus (TD).

The first stem-cell data include multiple results or data R_{1,1}-R_{a,b}, where "a" is a positive integer such as one of the numbers from 2 to 100, and "b" is a positive integer such as 1 or 2. The first number in the subscript of R (i.e., each of the numbers 1 through a immediately following the letter of R) indicates a certain selected type of stem cells. The second number in the subscript of R (i.e., each of the numbers 1 through b immediately following the first number) indicates a certain data type.

With regard to the first number in the subscript of R, the results or data R_{1,1}-R_{1,b} having the first number 1 in the subscript of R may be results or data related to SB-1 cells. The results or data R_{2,1}-R_{2,b} having the first number 2 in the subscript of R may be results or data related to SB-2 cells. The results or data R_{3,1}-R_{3,b} having the first number 3 in the subscript of R may be results or data related to BLSCs. The results or data R_{4,1}-R_{4,b} having the first number 4 in the subscript of R may be results or data related to VSELs. The results or data R_{5,1}-R_{5,b} having the first number 5 in the subscript of R may be results or data related to a specific type of multipotent stem cells, such as MSCs or MAPCs. The results or data R_{6,1}-R_{6,b} having the first number 6 in the subscript of R may be results or data related to another specific type of multipotent stem cells, such as HSCs, BMSCs or MASCs. The results or data R_{7,1}-R_{7,b} having the first number 7 in the subscript of R may be results or data related to a specific type of progenitor stem cells, such as pre-MSCs, MPPs, LRPs, CMPs, CLPs, or HPCs. The results or data R_{8,1}-R_{8,b} having the first number 8 in the subscript of R may be results or data related to another specific type of progenitor stem cells, such as MIAMI cells or mesenchymal progenitor cells.

With regard to the second number in the subscript of R, the results or data R_{1,1}-R_{a,1} having the second number 1 in the subscript of R may be results or data related to the number of stem cells. The results or data R_{1,2}-R_{a,2} having the second number 2 in the subscript of R may be results or data related to the percentage of stem cells. The results or data R_{1,3}-R_{a,3} having the second number 3 in the subscript of R may be results or data related to the count of stem cells per unit volume (e.g., milliliter) of a tissue sample (e.g., the first tissue sample). For further elaboration, the result R_{1,1} may indicate the number of SB-1 cells. The result R_{1,2} may indicate the percentage of SB-1 cells. The result R_{1,3} may indicate the count of SB-1 cells per unit volume (e.g., milliliter) of a tissue sample (e.g., the first tissue sample). The result R_{2,1} may indicate the number of SB-2 cells. The result R_{2,2} may indicate the percentage of SB-2 cells. The result R_{2,3} may indicate the count of SB-2 cells per unit volume (e.g., milliliter) of a tissue sample (e.g., the first tissue sample). Other notations are considered in a similar way.

Referring to Fig. 7C, after the step 49 or 50 is performed, the above-mentioned steps 51, 52 and 53 are performed in sequence. Therefore, a tissue sample (hereinafter the "second tissue sample") from the subject after taking or being subjected to the one or more actions or stimuli depicted in the step 51 is extracted, taken, obtained or derived. The second tissue sample may be referred to the above-mentioned tissue sample (P). The first and second tissue samples may be two samples obtained from the same type of tissue of the subject. For example, both of the first and second tissue samples may be obtained from peripheral blood, bone marrow, muscle, adipocyte or fat of the subject. In one example, both of the first and second tissue samples may be peripheral-blood samples.

Next, in step 56, a second test or measurement including assessing and/or measuring stem cells is performed on the second tissue sample to obtain second stem-cell data (i.e., the Data A) related to one or more types of stem cells from the second tissue sample descriptively or quantitatively. For more detailed information on the second test or measurement, please refer to the above-mentioned test or measurement (M0), (M1) or (M2), the following steps 61-70 in Fig. 9, or the following steps 1-20 in Figs. 14A-14C. Alternatively, the second stem-cell data may be obtained by the following integrated system, apparatus, device or tool (AD) or the following single piece of system, device, tool or apparatus (TD).

The second stem-cell data include information as the first stem-cell data illustrated in the step 50, that is, the second stem-cell data include multiple results or data R_{1,1}-R_{a,b}, wherein the first and second numbers in the subscript of R_{1,1}-R_{a,b} for the second stem-cell data are defined, described or specified as the first stem-cell data illustrated in the step 50, respectively. For collecting the same types of information and reducing experimental errors, both of the first and second tests or measurements for the first and second stem-cell data are performed using the same method described in the above-mentioned test or measurement (M0), (M1) or (M2), the following steps 61-70 in Fig. 9, or the following steps 1-20 in Figs. 14A-14C, or using the same device such as the following integrated system, apparatus, device or tool (AD) or the following single piece of system, device, tool or apparatus (TD).

Next, in step 57, by performing an analysis based on the first and second stem-cell data (e.g., including a comparison between the first and second stem-cell data), the effectiveness of the one or more actions or stimuli depicted in the step 51 for curing or treating the subject having the specific disease may be identified, determined or evaluated. The first and second stem-cell data may be written in an evaluation or examination report. In addition, the steps 49, 50, 51, 52, 53, 56 and 57 in Fig. 7C may be configured for identifying, determining, evaluating or testing the effect or effectiveness of the one or more actions or stimuli depicted in the step 51, such as taking or ingesting one or more pills of the brown algae supplement as mentioned in the following first experiment and Fig. 11.

Referring to Fig. 7C, after the step 56 or 57 is performed, step 58 may be performed if necessary. In the step 58, the same steps as the steps 51, 52, 53, 56 and 57 are performed on the same subject one or more times. During the step 58, each time when the step 53 is performed, a tissue sample is taken or obtained from the same subject after the step 52 is performed. During the step 58, each time when a test or measurement, like the test or measurement depicted in the step 56, is performed on a tissue sample from the same subject, stem-cell data, which may be referred to the first stem-cell data depicted in the step 50, related to one or more types of stem cells from the tissue sample are obtained. During the step 58, each time when the current stem-cell data are compared with the previous or first stem-cell data, such as comparing the second stem-cell data with the first stem-cell data as depicted in the step 57, the effectiveness of the one or more actions or stimuli depicted in the step 51 for curing or treating the subject having the specific disease may be identified, determined or evaluated. Each set of the stem-cell data obtained in the step 58 include information as the first stem-cell data illustrated in the step 50, that is, each set of the stem-cell data obtained in the step 58 include multiple results or data R_{1,1}-R_{a,b}, wherein the first and second numbers in the subscript of R_{1,1}-R_{a,b} for each set of the stem-cell data obtained in the step 58 are defined, described or specified as the first stem-cell data illustrated in the step 50, respectively. In addition, the step 58 in Fig. 7C may be configured for identifying, determining, evaluating or testing the effect or effectiveness of the one or more actions or stimuli in the step 51, such as taking or ingesting one or more pills of the brown algae supplement as mentioned in the following first experiment and Fig. 11.

In the following, a first experiment and related experimental results were shown, as an example, as a method to increase the stem cell counts in human bodies in vivo, for example, in the peripheral blood of human bodies. Figs. 8A-8F show stem-cell data of three human subjects (i.e., persons) before and after orally taking 30 pills of a brown algae fucoidan supplement. The stem-cell data shown in Figs. 8A-8F were obtained from the three human subjects by performing the same method as the steps 49-53 and 56-58. In this first experiment, each of the three human subjects orally took 30 pills of the brown algae fucoidan supplement in the step 51. Each pill of the brown algae fucoidan supplement weighs 151.3mg and contains mainly 0.1g of fuciodan and 0.04g of dietary fiber. The brown algae fucoidan supplement was produced in Okinawa, Japan and includes fucoidan extracted from brown algae grown in the sea around and near Okinawa, Japan. The brown algae is one kind of seaweed. The brown algae has various kinds, for example, mozuku, kelp, undaria pinnatifida, sargassum fusiforme, and etc. The green algae, blue-algae, or green-blue algae grows near the seashore or in the shallow sea, and grows relying on photosynthesis. The red algae grows in the deep sea relying on weak lights for photosynthesis. The brown algae grows in a sea between where the green/blue algae grows and where the red algae grows; in other words, brown algae grows in a sea with a middle range of depth.

Fig. 11 shows the content/ingredient information of the brown algae fucoidan supplement used in the first experiment. In Fig. 11, it shows that the pill of the brown algae fucoidan supplement contains 80% of a mozuku powder in weight, 15% of crystalline cellulose in weight, 3% of sucrose fatty acid esters in weight, and 2% of micro or fine silica (containing silicon dioxide) in weight. The mozuku powder was extracted from mozuku brown algae (one kind of seaweed) grown in the sea around and near Okinawa, Japan. The mozuku powder was then mixed with crystalline cellulose, sucrose fatty acid esters, and micro or fine silica (containing silicon dioxide) to form the pill of brown algae fucoidan supplement. In the first experiment, each person orally took 30 pills of the Okinawa brown algae fucoidan supplement, which means each person took about 4.5 grams (or greater than 3.5 grams) of the Okinawa brown algae supplement containing 80% (or more than 70%) of the mozuku powder in weight. Here 30 pills of the Okinawa brown algae fucoidan supplement, i.e., about 4.5 grams of the Okinawa brown algae supplement, contain 3 grams of fucoidan (or greater than 2 grams, or more than 60% of fucoidan in weight). Therefore, each person took 3 grams (or greater than 2 grams) of fucoidan.

Each person weighed about 75 Kg for the three persons in this first experiment. That means the dose of fucoidan is about 40 mg per Kg body weight. Alternatively, the fucoidan dose may be greater than 20, 30, 40 or 50 mg per Kg body weight. Fig. 12 shows a portion of the molecule structure of fucoidan. It contains rings, each comprising 5 carbons (C) and one oxygen (O). Note it contains radicals R. The radical R can be SO₃ or H. The SO₃ radicals play very important role for functions of fucoidan. The sulfur (S) is about 12% in weight of the fucoidan molecule in the Okinawa brown algae fucoidan supplement. For the embodiments of this invention, the weight percentage of sulfur (S) in the fucoidan molecule in the brown algae fucoidan supplement can be greater than 6%, 8%, or greater than 10%. Alternatively, the weight percentage of sulfur (S) in the fucoidan molecule in the brown algae fucoidan supplement can be greater than 12%, 16%, or greater than 20%.

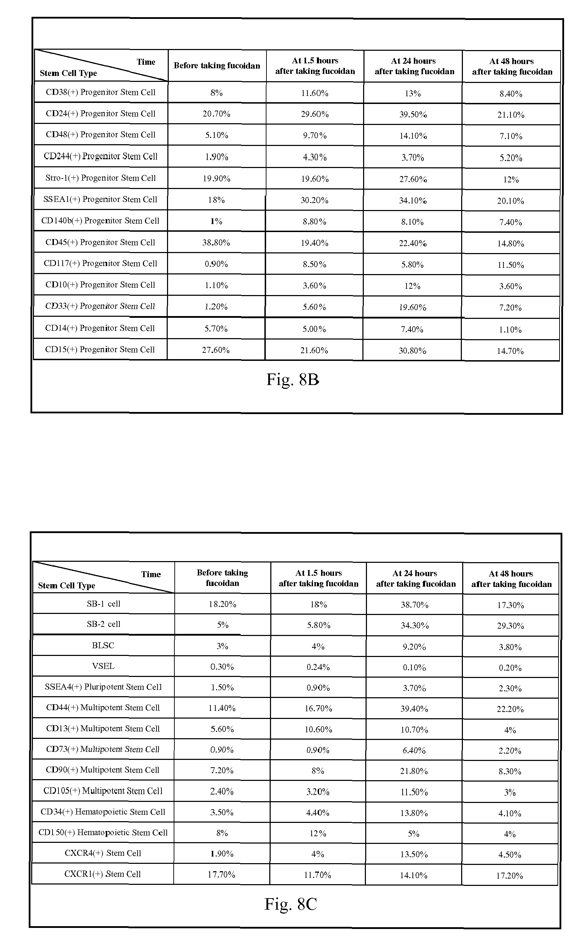

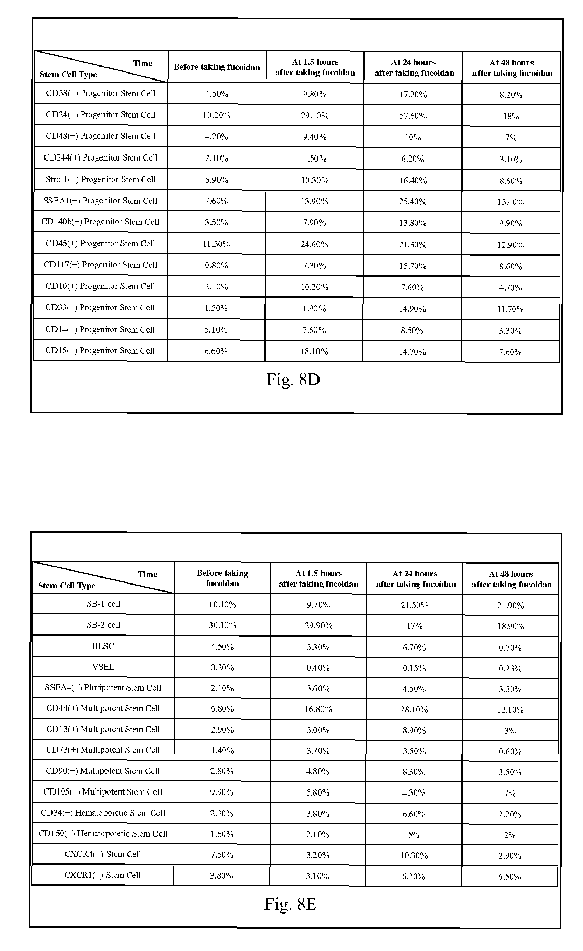

The three human subjects involved in the first experiment were: (1) Subject A: a woman in late twenties, whose experimental data are shown in Figs. 8A and 8B; (2) Subject B: a man in mid-fifties, whose experimental data are shown in Figs. 8C and 8D; and (3) Subject C: a man in early fifties, whose experimental data are shown in Figs. 8E and 8F. The stem-cell data in Figs. 8A and 8B show the percentage of each type of stem cells (total 27 types of stem cells) for Subject A before and at 1.5 hours, at 24 hours and at 48 hours after ingestion of the brown algae fucoidan supplement. The stem-cell data in Figs. 8C and 8D show the percentage of each type of stem cells (total 27 types of stem cells) for Subject B before and at 1.5 hours, at 24 hours and at 48 hours after ingestion of the brown algae fucoidan supplement. The stem-cell data in Figs. 8E and 8F show the percentage of each type of stem cells (total 27 types of stem cells) for Subject C before and at 1.5 hours, at 24 hours and at 48 hours after ingestion of the brown algae fucoidan supplement.

The experimental methods, procedures and specifications for stem cell studies, including using a flow cytometer, were described in the previous paragraphs. Here steps 61-70 depicted in Fig. 9 may be performed to obtain the percentage of each type of stem cells (total 27 types of stem cells) for each human subject before or at 1.5 hours, at 24 hours or at 48 hours after ingestion of the brown algae fucoidan supplement. Referring to Fig. 9, in the step 61, 5 milliliters of peripheral blood are obtained or collected from a subject (e.g., any one of the human subjects) and placed in a first tube to be mixed with a divalent cation chelating agent such as ethylenediaminetetraacetic acid (EDTA). Next, in the step 62, the 5 milliliters of peripheral blood in the first tube is added with 1 milliliter of 6% Hetastarch so as to form a first intermediate sample. The 6% Hetastarch is a solution containing 6% of Hetastarch in a phosphate-buffered saline (PBS), wherein every 100 mL PBS contains 6g of Hetastarch. Next, in the step 63, the first intermediate sample is incubated at a room temperature (e.g., 27 degrees Celsius) for a suitable time period (e.g., at least 30 minutes). Once two separate layers are formed in the first intermediate sample, the top layer (i.e., cell layer) of the first intermediate sample is pipetted out into a second tube without disturbance of the underlying layer (i.e., red-blood-cell (RBC) layer) of the first intermediate sample. Next, in the step 64, the content in the second tube is centrifuged at a suitable rotational speed (e.g., 3,000 rpm) for a suitable time period (e.g., 15 minutes) so as to obtain a centrifuged sample. Next, in the step 65, the supernatant of the centrifuged sample is discarded. Next, in the step 66, the pellets from the centrifuged sample are washed with a suitable salt solution (e.g., PBS) and re-suspended in 3 milliliters of a first medium (e.g., 1% or 2% BSA in PBS) so as to obtain a 3-milliliter sample.

Next, in the step 67, the 3-milliliter sample is divided into multiple sample containers such as test tubes, each containing 100 microliters of sample, i.e., 100-microliter sample, derived from the 3-milliter sample. Then each sample container may be added with a specific stain, which may contain one or more types of antibodies. The stain added in each sample container may be different from one another. In other words, the stain added in each sample container may include a specific type of antibodies, or a specific combination or group of various antibodies. The specific types of antibodies or the specific combinations or groups of various antibodies of the stain added in the respective sample containers are different from each another. The purpose of adding a specific stain in each sample container is to identify a specific cell (surface) marker, a specific group including multiple selected cell (surface) markers, or specific multiple stem cells. Each type of antibodies in the stains may be used for identifying a specific cell (surface) marker, a specific type of stem cells, or a specific group of selected types of stem cells or selected cell (surface) markers, which is different from the other types of antibodies in the stains. Therefore, each of the sample containers contains a second intermediate sample composed of the 100-microliter sample stained by a specific one of the stains having one or more specific types of antibodies.

Next, in the step 68, the second intermediate samples in the sample containers are incubated at a room temperature (e.g., 27 degrees Celsius) for a suitable time period (e.g., at least 30 minutes). Next, in the step 69, each of the second intermediate samples is mixed with about 400 microliters of a second medium (e.g., 1% or 2% BSA in PBS) so as to form 500 microliters of sample, i.e., 500-microliter sample, which is a mixture of the 100-microliter sample, stained by a corresponding one of the stains having one or more corresponding types of antibodies, and the second medium. Next, in the step 70, the 500-microliter samples in the sample containers are analyzed by a flow cytometer to obtain stem-cell data. For each 500-microliter sample, one million particles were counted and analyzed by the flow cytometer in the first experiment. The one million particles include stem cells, other cells, and particles from the added stain or second medium.

In the step 70 of Fig. 9, the one million particles, including stem cells, other cells and particles from the added stain or second medium, in each 500-microliter sample are differentiated by forward and side scattering coefficients measured by the flow cytometer to obtain corresponding cell or particle populations. Fig. 10 is a forward scattering coefficient (FSC)/side scattering coefficient (SSC) graph for the flow cytometer and shows the cell or particle populations for the one million particles, including stem cells, other cells, and particles from the added stain or second medium, in one of the 500-microliter samples. Referring to Fig. 10, the horizontal axis represents the forward scattering coefficient (FSC) ranging from 10³ to 10^{7.2}; the vertical axis represents the side scattering coefficient (SSC) ranging from 10¹ to 10^{7.2}. In this disclosure, FSC is proportional or related to the surface area or size of a cell or particle and the SSC is proportional or related to the granularity or internal complexity of a cell or particle. Dots in a right region of a vertical dotted line 81 marked in Fig. 10 may represent particles or cells having sizes greater than 1 micrometer. Dots in an upper region of a horizontal dotted line 82 marked in Fig. 10 may represent particles or cells having SSC greater than 10².

The cell or particle populations shown in Fig. 10 include: (1) micro particles, as indicated in a region P1; (2) cells greater than 1 micrometer in size and greater than 10² in SSC, as indicated in a region P3; (3) unknown micro particles, as indicated in a region P4; (4) a staining buffer, as indicated in a region P5; (5) lymphocytes, as indicated in a region P6; (6) red blood cells, as indicated in a region P7; (7) granulocytes, as indicated in a region P8; and (8) blood platelets, as indicated in a region P9. The region P3 includes the regions P1, P6, P7, P8 and P9. Some cells or particles of interest (hereinafter the "total cells or particles TS") are in the region P3 but not in the regions P1, P6, P7, P8 and P9, and one or more of the 27 types of stem cells, such as the SB-1 cells and/or the SB-2 cells, may be found in the total cells or particles TS. The cell or particle populations for the other 500-microliter samples may be similarly depicted as in Fig. 10.

The number or count of the total cells or particles TS and the number or count of stem cells of the selected type in the total cells or particles TS for each 500-microliter sample are measured by the flow cytometer. Then, using the above data, the percentages of the numbers or counts of the 27 types of stem cells to the numbers or counts of the total cells or particles TS respectively may be obtained as shown in Figs. 8A-8F. The percentage of each type of stem cells shown in Figs. 8A-8F can be calculated by dividing the number or count of each type of stem cells by the number or count of the corresponding total cells or particles TS and then multiplying each of the divided results by 100% (which converts the divided result into a percent figure). The stem-cell data from the flow cytometer in the step 70 include the percentage of the number or count of each type of stem cells (total 27 types of stem cells) to the number or count of the corresponding total cells or particles TS.

Therefore, by performing the method depicted in the steps 61-70 on the three human subjects, the percentages of the numbers or counts of the 27 types of stem cells to the numbers or counts of the total cells or particles TS respectively for the human subjects before and at 1.5 hours, at 24 hours and at 48 hours after ingestion of the brown algae fucoidan supplement can be obtained as shown in Figs. 8A-8F. As a result, the percentage change in the stem cells of each specific type for each of the human subjects between before and at 1.5 hours after ingestion of the brown algae fucoidan supplement, between before and at 24 hours after ingestion of the brown algae fucoidan supplement, or between before and at 48 hours after ingestion of the brown algae fucoidan supplement can be known and used to establish an optimal stem-cell harvesting time point (e.g., at 24 hours after ingestion of the brown algae fucoidan supplement in the first experiment) related to stem cell expansion in vivo.

Referring back to Figs. 8A-8F, compared the data of the human subjects before ingestion of the brown algae fucoidan supplement with the data of the human subjects at 24 hours after ingestion of the brown algae fucoidan supplement, most types of stem cells increase in percentage. Particularly, two-fold (two times) or more increases in percentage can be found for the SB-1 cells, CD44(+) multipotent stem cells, CD13(+) multipotent stem cells, CD73(+) multipotent stem cells, CD90(+) multipotent stem cells, CD34(+) hematopoietic stem cells, CD48(+) progenitor stem cells, CD244(+) progenitor stem cells, CD140b(+) progenitor stem cells, CD117(+) progenitor stem cells, CD10(+) progenitor stem cells, and CD33(+) progenitor stem cells by comparing the percentages of the numbers or counts of the above-mentioned types of stem cells to the numbers or counts of the corresponding total cells or particles TS for the human subjects at 24 hours after ingestion of the brown algae fucoidan supplement to those for the human subjects before ingestion of the brown algae fucoidan supplement.

According to the stem-cell data shown in Figs. 8A-8F, taking the brown algae fucoidan supplement is possible to cause or induce at least 2-fold (2 times) increase in percentage of some specific types of stem cells in peripheral blood of the human subjects at 24 hours after ingestion of the brown algae fucoidan supplement. Here "2-fold (2 times) increase" means the ratio between the percentages of some specific types of stem cells before and at 24 hours (or, for other case, at a specific time interval, for example, 1.5 hours, 3 hours, 6 hours, 9 hours, 12 hours, 18 hours, 30 hours, or 36 hours) after the ingestion of the brown algae fucoidan supplement. From Figs. 8A-8F, it can be 1.5-fold (1.5 times) increase, 3-fold (3 times) increase, or 4-fold (4 times) increase for other specific types of stem cells. Therefore, orally taking fucoidan, a major component of brown algae, may be an effective approach to increase stem cells in peripheral blood of human bodies or animal bodies. An action, such as orally taking 3 grams (greater than 2 grams) of fucoidan (from the above-mentioned brown algae fucoidan supplement) or one or more of the actions or stimuli (X), can increase, induce, or activate stem cells in the peripheral blood of a human or animal body by at least (or greater than) 1.5-fold (1.5 times) increase, 2-fold (2 times) increase, 3-fold (3 times) increase, or 4-fold (4 times) increase.

Some criteria may be set to decide whether the tissue sample (extracted after taking or being subjected to the one or more actions or stimuli in the step 51) obtained in the step 53 is useful for harvesting, obtaining or collecting stem cells in the step 54 in Fig. 7A. For example, referring to Fig. 7A, the tissue sample (extracted after taking or being subjected to the one or more actions or stimuli in the step 51) obtained in the step 53 may be used for harvesting, obtaining or collecting stem cells when measurement results or data show the percentage of the number or count of the SB-1 cells to the number or count of the total cells or particles TS is greater than 20%, 25%, or 30%, the percentage of the number or count of the SB-2 cells to the number or count of the total cells or particles TS is greater than 20%, 25% or 30%, and/or the percentage of the number or count of the BLSCs to the number or count of the total cells or particles TS is greater than 5% or 10%. Alternatively, referring to Fig. 7A, the tissue sample (extracted after taking or being subjected to the one or more actions or stimuli in the step 51) obtained in the step 53 may be used for harvesting, obtaining or collecting stem cells when measurement results or data show at least (or greater than) 1.5-fold (1.5 times) increase, 2-fold (2 times) increase, or 3-fold (3 times) increase in the SB-1 cells, the SB-2 cells, and/or the BLSCs. The x-fold (x times) increase is defined as in the above paragraph.

Based on the results in Figs. 8A-8F, orally taking fucoidan is believed and proved to increase the number of stem cells in peripheral blood of a human or animal. Therefore, a symbol, sign, statement or mark of indicating that orally taking the brown algae fucoidan supplement, i.e., one of the actions or stimuli (X), is effective to increase the number of some specific types of stem cells in a human body is allowed to be (or can be) labeled or printed on a packaging, container or wrapper of a product such as the brown algae fucoidan supplement. In an alternative example, the results in Figs. 8A-8F may be used to determine if the dose, intensity, duration, frequency, and/or the time of the action such as orally taking the brown algae fucoidan supplement is effective to increase the number of some specific types of stem cells in a human body. Based on the results in Figs. 8A-8F, taking 30 pills (containing 3-gram fucoidan, i.e., greater than 2 grams of fucoidan) of the brown algae fucoidan supplement one time or daily may be recommended for purpose of curing disease or injury or for purpose of health maintenance.

The 27 types of stem cells depicted in Figs. 8A-8F may be thought to be normally quiescent in tissues but will be mobilized to the peripheral blood and activated or differentiated to repair the damage tissues by ingesting the brown algae fucoidan supplement. Therefore, the fucoidan component in brown algae may play a critical role in the stem cell activation or generation or in the mobilization of stem cells into the bloodstream from niche sites, such as muscle or bone marrow. In other words, the major component of a brown algae, fucoidan, may be a mobilizer, generator or activator of stem cells. The above-mentioned embodiments may be employed for mobilizing, generating or activating stem cells of one or more types such as SB-1 cells and/or SB-2 cells.

Fig. 13 shows a method used to obtain stem-cell data related to a subject. Referring to Fig. 13, in step 101, an experimental subject such as the above-mentioned subject (S) takes or is subjected to one or more of the above-mentioned actions or stimuli (X). For example, the experimental subject orally takes 30 pills of a brown algae fucoidan supplement, which may be the same as that depicted in Figs. 11 and 12; therefore, the experimental subject takes 3 grams (i.e., greater than 2 grams) of fucoidan in the step 101. Alternatively, the experimental subject may be subjected to injection of a nutrient or supplement containing fucoidan or oligo fucoidan in the step 101. Next, in step 102, the experimental subject waits for a (predetermined) time interval (e.g., 1.5 hours, 12hours, 24 hours, 48 hours, between 0.5 hours and 3 hours, or between 1 hour and 2 hours) after taking or being subjected to the one or more of the actions or stimuli (X) in the step 101. Next, in step 103, a blood sample (i.e., a tissue sample) is obtained by, e.g., extracting or taking peripheral blood of the experimental subject at a specific time point to be placed into a blood collection tube such as ethylenediaminetetraacetic acid (EDTA) tube, wherein the specific time point is a time point at the end of the (predetermined) time interval. The volume of the blood sample may be greater than or equal to 5 milliliters or 10 milliliters. Next, in step 104, a measurement or analysis (hereinafter the "measurement MT") is performed on the blood sample to obtain stem-cell data related to the experimental subject at the specific time point. The stem-cell data may include the count of each specific type of stem cells as mentioned in the paragraphs in "Description of stem cells" per milliliter of peripheral blood of the experimental subject at the specific time point. Before the measurement MT in the step 104 is performed on the blood sample, the blood sample may be kept at between 1°C and 30°C (such as between 1°C and 10°C, between 10°C and 20°C, or between 20°C and 30°C). The time period between obtaining the blood sample in the step 103 and performing the measurement MT in the step 104 on the blood sample may be controlled to be equal to or less than 30 minutes, 60 minutes, 12 hours, 24 hours, 48 hours, 72 hours, or 1 week (such as between 10 and 30 minutes, between 30 and 60 minutes, between 0.5 and 2 hours, between 2 and 12 hours, between 12 and 24 hours, between 24 and 48 hours, or between 48 and 72 hours).

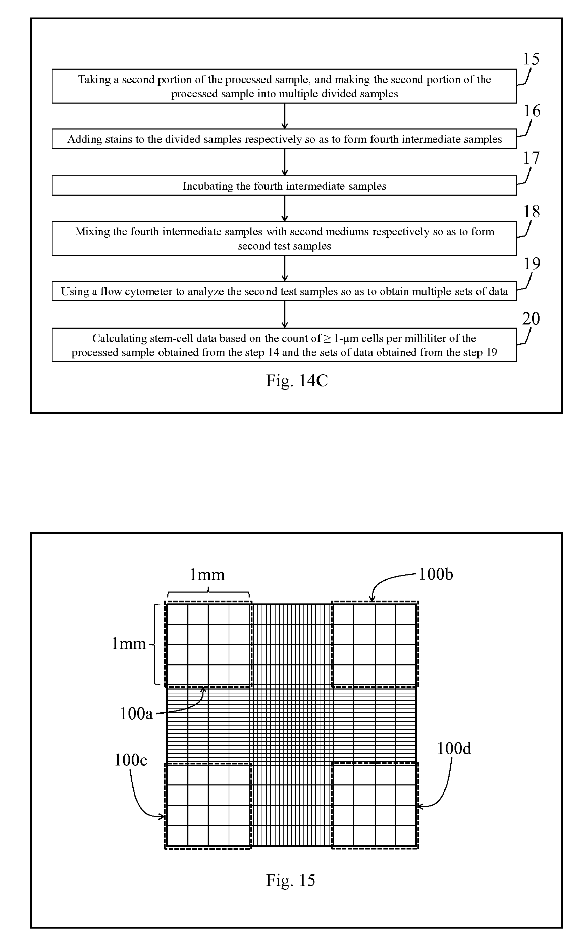

The measurement MT, depicted in the step 104 in Fig. 13, may include steps 1-8 depicted in Fig. 14A, steps 9-14 depicted in Fig. 14B, and steps 15-20 depicted in Fig. 14C. Referring to Fig. 14A, in the step 1, a blood sample (such as peripheral blood) in a blood collection tube such as EDTA tube, obtained in the step 103 in Fig. 13 for example, is added with an erythrocyte aggregation agent containing 6% Hetastarch (e.g., HetaSep™ solution) so as to form a first intermediate sample. The blood sample is obtained by extracting or taking peripheral blood of a subject such as the above-mentioned subject (S) to be placed into the blood collection tube and may be kept at between 1°C and 30°C (such as between 1°C and 10°C, between 10°C and 20°C, or between 20°C and 30°C) before the step 1 is performed on it. The time period between obtaining the blood sample and performing the step 1 on the blood sample may be controlled to be equal to or less than 30 minutes, 60 minutes, 12 hours, 24 hours, 48 hours, 72 hours, or 1 week (such as between 10 and 30 minutes, between 30 and 60 minutes, between 0.5 and 2 hours, between 2 and 12 hours, between 12 and 24 hours, between 24 and 48 hours, or between 48 and 72 hours). The volume of the blood sample may be greater than or equal to 5 milliliters or 10 milliliters, and the ratio of the volume of the blood sample to the volume of the erythrocyte aggregation agent containing 6% Hetastarch may be 5:1. The 6% Hetastarch is a solution containing 6% of Hetastarch in a phosphate-buffered saline (PBS), wherein every 100 mL PBS contains 6g of Hetastarch.

Next, in the step 2, the first intermediate sample is placed to a first tube (e.g., falcon tube) and incubated at a room temperature (e.g., about 27 degrees Celsius) for a suitable time period (e.g., between 30 minutes and 90 minutes). Once two separate layers are formed in the first intermediate sample in the first tube, the top layer (i.e., cell layer) of the first intermediate sample is pipetted out into a second tube without disturbance of the underlying layer (i.e., red-blood-cell (RBC) layer) of the first intermediate sample. Next, in the step 3, the pipetted content in the second tube is centrifuged at a suitable rotational speed (e.g., 1,800 rpm or 3,000 rpm) for a suitable time period (e.g., between 10 minutes and 20 minutes) so as to obtain a first centrifuged sample. Next, in the step 4, the supernatant of the first centrifuged sample is discarded, and pellets of the first centrifuged sample are re-suspended in a first salt solution without calcium and magnesium (e.g., Dulbecco's phosphate-buffered saline (DPBS) without calcium and magnesium) so as to form a second intermediate sample. The pellets of the first centrifuged sample have a portion derived from the blood sample. Next, in the step 5, the second intermediate sample is centrifuged at a suitable rotational speed (e.g., 1,800 rpm or 3,000 rpm) for a suitable time period (e.g., between 10 minutes and 20 minutes) so as to obtain a second centrifuged sample.

Next, in the step 6, the supernatant of the second centrifuged sample is discarded, and the pellets of the second centrifuged sample are re-suspended in a second salt solution without calcium and magnesium (e.g., DPBS without calcium and magnesium) so as to form a third intermediate sample. The pellets of the second centrifuged sample have a portion derived from the blood sample. Next, in the step 7, the third intermediate sample is centrifuged at a suitable rotational speed (e.g., 1,800 rpm or 3,000 rpm) for a suitable time period (e.g., between 10 minutes and 20 minutes) so as to obtain a third centrifuged sample. Next, in the step 8, the supernatant of the third centrifuged sample is discarded, and the pellets of the third centrifuged sample are re-suspended in a first medium (e.g., 1% bovine serum albumin (BSA) in PBS) so as to obtain a processed sample. Here 1% BSA in PBS is made by dissolving 0.1g of BSA into 10mL of PBS. The pellets of the third centrifuged sample have a portion derived from the blood sample. The volume of the processed sample may be greater than or equal to 0.8 milliliters or 3 milliliters and may be less than the volume of the blood sample in the step 1. The volume of the first medium may be approximately the same as the volume of the processed sample.

Referring to Fig. 14B, after the step 8 is performed to obtain the processed sample, the steps 9-14 are performed in sequence. In the step 9, a first portion of the processed sample is taken and transferred to a third tube. Next, in the step 10, the first portion of the processed sample in the third tube is added with and mixed with a stain (e.g., trypan blue) so as to form a first test sample. The volume of the first portion of the processed sample may be between 5 microliters and 50 microliters, such as 10 microliters, and may be the same as the volume of the stain. Next, in the step 11, a portion of the first test sample is loaded into a chamber of a hemocytometer. The volume of the portion of the first test sample may be between 10 microliters and 20 microliters, such as 10 microliters. The hemocytometer has a microscopic grid as shown in Fig. 15 under the chamber, and the microscopic grid includes four squares 100a-100d with 1mm in width, i.e., 1-mm squares, each of which is divided into sixteen squares with 0.25mm in width, i.e., 0.25-mm squares. In addition, the chamber is overlaid with a glass coverslip, and the space between the bottom surface of the glass coverslip and the floor of the chamber has a depth of 0.1mm. Therefore, each of the 1-mm squares 100a-100d represents or features a volume of 0.1mm³ or 10-⁴mL.

Referring to Fig. 14B, after the step 11 is performed, the hemocytometer is placed on a stage of a microscope in the step 12. The microscope may have a total power of magnification greater than or equal to 200 times and may have an ocular lens with a power of magnification greater than or equal to 10X and an objective lens with a power of magnification greater than or equal to 20X. Next, in the step 13, the count of cells having sizes greater than or equal to 1 micrometer, or the count of ≥ 1-µm cells, in one of the sixteen 0.25-mm squares in the 1-mm square 100a is obtained in the field of view of the microscope and accordingly the count of the ≥ 1-µm cells in the 1-mm square 100a may be estimated by multiplying the count of the ≥ 1-µm cells in the one of the sixteen 0.25-mm squares in the 1-mm square 100a by 16 (which is the number of the 0.25-mm squares in the 1-mm square 100a). Next, in the step 14, the count of the ≥ 1-µm cells per milliliter of the processed sample (i.e., the count per unit volume of the ≥ 1-µm cells in the processed sample) is estimated by, e.g., multiplying the count of the ≥ 1-µm cells in the 1-mm square 100a by a dilution factor (such as equal to or greater than 1.5, 2, or 3) and dividing the multiplied result by the volume of the 1-mm square 100a (i.e., 10⁻⁴mL). As an example, the dilution factor is 2 when the first portion of the processed sample is diluted with the stain having the same volume as the first portion of the processed sample. The ≥ 1-µm cells depicted in the steps 13 and 14 may include micro particles, granulocytes, red blood cells, blood platelets, white blood cells including lymphocytes, and various types of stem cells, wherein the micro particles may have no nucleus or cytoplasm.

Alternatively, in the step 14, the count of the ≥ 1-µm cells per milliliter of the processed sample may be estimated by multiplying the average count of the ≥ 1-µm cells per 1-mm square of the hemocytometer by the dilution factor and dividing the multiplied result by the average volume per 1-mm square of the hemocytometer (i.e., 10⁻⁴mL). In this case, the step 13 includes (1) counting cells having sizes greater than or equal to 1 micrometer, or the count of ≥ 1-µm cells, in one of the sixteen 0.25-mm squares in each of the four 1-mm squares 100a-100d in the field of view of the microscope so as to obtain four primitive counts of ≥ 1-µm cells respectively and (2) multiplying each of the four primitive counts of the ≥ 1-µm cells by 16 (which is the number of the 0.25-mm squares in each of the 1-mm square 100a-100d) so as to estimate the count of ≥ 1-µm cells in each of the 1-mm squares 100a-100d. Therefore, the average count of the ≥ 1-µm cells per 1-mm square of the hemocytometer is obtained by, e.g., summing the estimated counts of the ≥ 1-µm cells in the four 1-mm squares 100a-100d and dividing the summed result by 4 (which is the number of the 1-mm squares 100a-100d).

Referring to Fig. 14C, after the step 8 is performed to obtain the processed sample, the steps 15-20 are performed in sequence. In the step 15, a second portion of the processed sample is taken and divided into multiple sample containers such as test tubes, each containing L microliters of the second portion (hereinafter the "divided sample"), where L may be one of the numbers from 50 to 250, such as 100. Here the second portion of the processed sample is supposed to contain substantially the same content as the first portion of the processed sample in the step 9 in the case that the processed sample is homogeneous and contains even content. The total volume of the second portion of the processed sample may be between 0.5 milliliters and 18 milliliters, such as 2 milliliters. The number of the sample containers may be between 2 and 50. Next, in the step 16, each sample container is added with a specific stain, which may contain one or more types of antibodies. The stain added in each sample container may be different from one another and may include a specific type of antibodies or a specific combination or group of various antibodies. The specific types of antibodies or the specific combinations or groups of various antibodies of the stain added in the respective sample containers are different from each another. The purpose of adding specific various stains into the respective sample containers is to identify multiple specific cell (surface) markers or multiple specific types of stem cells each characterized by one or more various cell markers as mentioned in the paragraphs of "Description of stem cells" in this disclosure. Each type of antibodies in the stains may be used for identifying a specific cell (surface) marker, a specific type of stem cells, or a specific group of selected types of stem cells or selected cell (surface) markers, which is different from the other types of antibodies in the stains. Therefore, each of the sample containers contains a fourth intermediate sample composed of the divided sample stained by a specific one of the stains having one or more specific types of antibodies.

Next, in the step 17, the fourth intermediate samples in the sample containers are incubated at a room temperature (e.g., about 27 degrees Celsius) for a suitable time period (e.g., at least 30 minutes). Next, in the step 18, each of the fourth intermediate samples in the sample containers is mixed with a second medium (e.g., 1% BSA in PBS) so as to form a corresponding second test sample (acting as a flow-cytometer sample), which is a mixture of the divided sample, stained by a corresponding one of the stains having one or more types of antibodies, and the second medium. The volume of each second test sample may be between 250 microliters and 1,250 microliters, such as 500 microliters. The volume of the second medium to be mixed with each of the fourth intermediate samples may be between 200 microliters and 1,000 microliters (e.g., 400 microliters) and greater than the volume of the corresponding divided sample.

Next, in the step 19, the second test samples in the sample containers are analyzed by a flow cytometer to obtain multiple sets of data. Each set of data obtained from a corresponding one of the second test samples includes the count of cells having sizes greater than or equal to 1 micrometer (hereinafter the "cell count C1"), the count of cells in a region of high nucleus/cytoplasm ratio (hereinafter the "cell count C2"), and the percentage of the count of a specific (or selected) type of stem cells in the region of high nucleus/cytoplasm ratio to the cell count C2 (hereinafter the "stem-cell percentage SP"). Here the specific (or selected) type of stem cells may be any one of the types of stem cells depicted in the paragraphs in "Description of stem cells" or may be stem cells characterized by one or more cell (surface) makers, e.g., including CD349(+), Lgr5(+) or CD66e(+), as mentioned in the paragraphs in "Description of stem cells". The cells in the region of high nucleus/cytoplasm ratio (including the specific (or selected) type of stem cells) may be greater than or equal to 1 micrometer, 1.1 micrometers, 1.5 micrometers, 2 micrometers, 2.1 micrometers, or 3 micrometers in size (as defined by the above-mentioned size (Z) of a cell) or may be between 1 and 15 micrometers (such as between 2 and 6 micrometers) in size (as defined by the above-mentioned size (Z) of a cell). The cells in the region of high nucleus/cytoplasm ratio have nuclei and/or cytoplasm and include various types of stem cells, e.g., the specific (or selected) type of stem cells, as mentioned in the paragraphs in "Description of stem cells" but substantially exclude micro particles, granulocytes, red blood cells, blood platelets, and white blood cells including lymphocytes. The cells having sizes greater than or equal to 1 micrometer (i.e., a given threshold size) depicted in the steps 13, 14 and 19 have substantially the same characteristic. The cells having sizes greater than or equal to 1 micrometer depicted in the step 19 may include micro particles, granulocytes, red blood cells, blood platelets, white blood cells including lymphocytes, and the cells in the region of high nucleus/cytoplasm ratio. The above-mentioned micro particles may have no nucleus or cytoplasm.

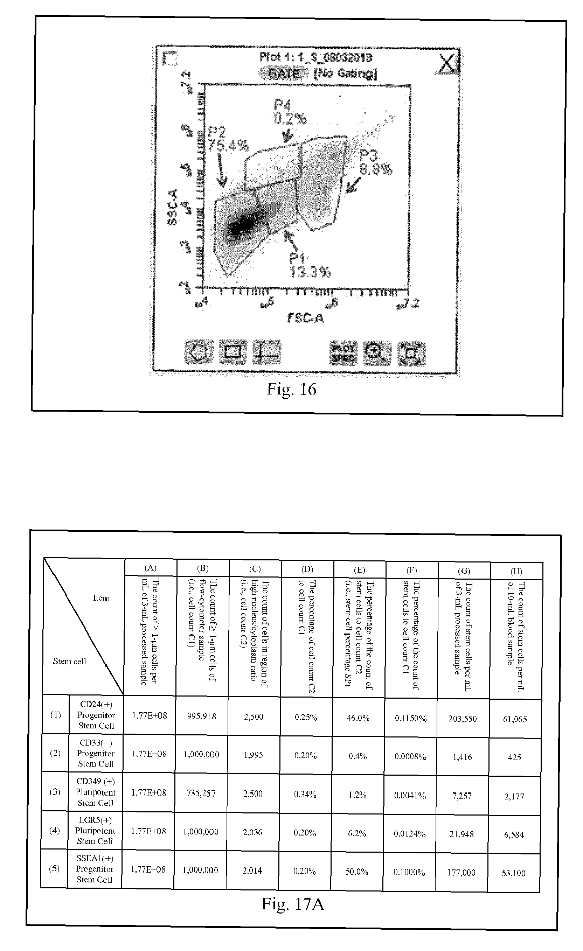

With respect to each set of data, the cell count C1 includes (1) the cell count C2, (2) the count of granulocytes, (3) the count of blood platelets, (4) the count of red blood cells, (5) the count of white blood cells, and (6) the count of micro particles. Fig. 16 shows a forward scattering coefficient (FSC)/side scattering coefficient (SSC) graph for the flow cytometer. Referring to Fig. 16, the horizontal axis represents the FSC, and the vertical axis represents the SSC. The FSC/SSC graph in Fig. 16 may be set up in advance for the flow cytometer analyzing each of the second test samples, including (1) a region P1 of blood platelets; (2) a region P2 of micro particles; (3) a region P3 of red and white blood cells; (4) the region of high nucleus/cytoplasm ratio, as indicated in a region P4; and (5) a region of granulocytes (not shown). Before the flow cytometer analyzes each of the second test samples, the (approximate) value of FSC that corresponds to 1µm in size may be set up using microbeads or polymer particles with 1 µm in size and used to set up a threshold region of cells having sizes greater than or equal to 1 micrometer to determine cells greater than or equal to 1 µm in size so as to obtain the cell counts C1 in the threshold region. The above regions P1-P4 and the above region of granulocytes are in the threshold region. In the step 19, the flow cytometer analyzes each second test sample until (1) the corresponding cell count C1 reaches a specific number that may be any number greater than or equal to five hundred thousand, eight hundred thousand, or one million or (2) the corresponding cell count C2 reaches a specific number that may be any number greater than 1000, 1500, 2000, 2500, 3000 or 3500. Accordingly, multiple sets of data related to the respective second test samples are obtained.

Referring to Fig. 14C, after the steps 14 and 19 are performed, the step 20 in Fig. 14C is performed to calculate stem-cell data based on the count of the ≥ 1-µm cells per milliliter of the processed sample, which may be obtained in the step 14, and the cell counts C1, cell counts C2 and stem-cell percentages SP of the sets of data, which may be obtained in the step 19. The stem-cell data may include the count of each specific type of stem cells, as mentioned in the paragraphs in "Description of stem cells", per milliliter of the blood sample (i.e., the count per unit volume of the each specific type of stem cells in the blood sample), which may be calculated by: (1) dividing the cell count C2 in the region P4 of high nucleus/cytoplasm ratio by the cell count C1 in the threshold region of cells having sizes greater than or equal to 1 micrometer, and multiplying the divided result by 100% (which converts the divided result into percent figure) so as to obtain the percentage of the cell count C2 to the cell count C1, i.e., a data including information related to a relationship between the cell count C2 and the cell count C1; (2) multiplying the percentage of the cell count C2 to the cell count C 1 by the stem-cell percentage SP for a selected type of stem cells and multiplying the multiplied result by 100% (which converts the multiplied result into percent figure) so as to obtain the percentage of the count of the selected type of stem cells to the cell count C1, i.e., a data relating to the processed sample and including information related to a relationship between the selected type of stem cells and the cell count C1; (3) multiplying the percentage of the count of the selected type of stem cells to the cell count C1 by the count of the ≥ 1-µm cells per milliliter of the processed sample, obtained in the step 14, so as to obtain the count of the selected type of stem cells per milliliter of the processed sample (i.e., the count per unit volume of the selected type of stem cells in the processed sample), which is a data including information related to a relationship between the selected type of stem cells and the processed sample; (4) dividing the volume of the processed sample (in milliliters) by the volume of the blood sample (in milliliters) so as to obtain a volume change (or sample preparation) factor, e.g., the ratio of the volume of the processed sample to the volume of the blood sample; and (5) multiplying the count of the selected type of stem cells per milliliter of the processed sample by the volume change (or sample preparation) factor so as to obtain the count of the selected type of stem cells per milliliter of the blood sample (i.e., the count per unit volume of the selected type of stem cells in the blood sample), which is a data including information related to a relationship between the selected type of stem cells and the blood sample and may be configured to represent the count of the selected type of stem cells per milliliter of peripheral blood of the subject. Here the selected type of stem cells may be any one of the types of stem cells depicted in the paragraphs in "Description of stem cells" or may be stem cells characterized by one or more common cell (surface) makers, e.g., including CD349(+), Lgr5(+) or CD66e(+), as mentioned in the paragraphs in "Description of stem cells". For obtaining the stem-cell data more accurately, the cell count C2 for the selected type of stem cells may be greater than or equal to 1,000 cells, 1,500 cells, 2,000 cells, or 2,500 cells.

For more elaboration, the count of cells depicted in Fig. 14B may be obtained for cells having sizes greater than or equal to a given threshold size (in this example of the count of the ≥ 1-µm cells or the four primitive counts of the ≥ 1-µm cells, the given threshold size equals 1 micrometer), and the cell counts C1 depicted in Fig. 14C may be obtained for cells having sizes greater than or equal to the given threshold size (in this example, the given threshold size equals 1 micrometer), where the given threshold size is 1 micrometer in this example or may be 0.5, 0.8, 1.5, 2, or 3 micrometer. For the cell counts C1 for cells having sizes greater than or equal to the given threshold size (in this example, the given threshold size equals 1 micrometer), the (approximate) value of FSC in the FSC/SSC graph for the flow cytometer analyzing each of the second test samples may be set up, using microbeads or polymer particles with the given threshold size (in this example, the given threshold size equals 1 micrometer), to establish the threshold region of cells having sizes greater than or equal to the corresponding given threshold size and to determine cells having sizes greater than or equal to the corresponding given threshold size (in this example, the given threshold size equals 1 micrometer).

Based on the above-mentioned approach depicted in Figs. 13, 14A, 14B and 14C, the present disclosure may use an integrated system, apparatus, device or tool (AD) to obtain the stem-cell data depicted in the step 20 for the blood sample of the subject in Fig. 13. The integrated system, apparatus, device or tool (AD) may include: (1) a first apparatus, device or tool for obtaining or taking the blood sample from the subject as mentioned in the step 103 of Fig. 13; (2) a second apparatus, device or tool (including, e.g., a centrifuge) for processing the blood sample to obtain or prepare the processed sample (i.e., a test sample) as mentioned in the steps 1-8 of Fig. 14A; (3) a third apparatus, device or tool (including, e.g., a hemocytometer, a microscope, and a processor/computer programed by a software) for obtaining a first data (e.g., the count of the ≥ 1-µm cells per milliliter of the processed sample, obtained in the step 14 of Fig. 14B) from the first portion of the processed sample by a first method, including e.g., counting particles or cells in the first portion of the processed sample using the hemocytometer (with the microscope), as mentioned in the steps 9-14 of Fig. 14B; (4) a fourth apparatus, device or tool (including, e.g., a flow cytometer and a processor/computer) for obtaining a second data (e.g., the stem-cell percentage SP for a selected type of stem cells) and a third data (e.g., the percentage of the cell count C2 for the selected type of stem cells to the cell count C1 for the selected type of stem cells) from the second portion of the processed sample by a second method, including, e.g., counting particles or cells in the second portion of the processed sample using the flow cytometer, as mentioned in the steps 15-20 of Fig. 14C; and (5) a fifth device, tool or apparatus (such as a computer or a processor) for: (a) performing a first operation or calculation of the second and third data, such as multiplying the second data by the third data (e.g., multiplying the stem-cell percentage SP by the percentage of the cell count C2 to the cell count C1) and multiplying the multiplied result by 100%, to obtain a fourth data (e.g., the percentage of the count of the selected type of stem cells to the cell count C1) relating to the processed sample; (b) performing a second operation or calculation of the first and fourth data, such as multiplying the first data by the fourth data (e.g., multiplying the count of the ≥ 1-µm cells per milliliter of the processed sample by the percentage of the count of the selected type of stem cells to the cell count C1), to obtain a fifth data (e.g., the count of the selected type of stem cells per milliliter of the processed sample) relating to the processed sample; (c) performing a third operation or calculation of the volume of the processed sample and the volume of the blood sample, such as dividing the volume of the processed sample by the volume of the blood sample, to obtain the volume change (or sample preparation) factor (e.g., the ratio of the volume of the processed sample to the volume of the blood sample); and (d) performing a fourth operation or calculation of the fifth data and the volume change (or sample preparation) factor, such as multiplying the fifth data by the volume change (or sample preparation) factor (e.g., multiplying the count of the selected type of stem cells per milliliter of the processed sample by the ratio of the volume of the processed sample to the volume of the blood sample), to obtain a sixth data (e.g., the count of the selected type of stem cells per milliliter of the blood sample) relating to the blood sample.

Two or more than two of the first, second, third, fourth and fifth devices, tools or apparatus may be combined into an integrated device, tool or apparatus or a single piece of device, tool or apparatus to perform the functions performed by the two or more than two of the first, second, third, fourth and fifth devices, tools or apparatus. For example, the first and second devices, tools or apparatus may be combined into a first integrated device or a first single piece of device, tool or apparatus to perform the functions performed by the first and second devices, tools or apparatus, and the third, fourth and fifth devices, tools or apparatus may be combined into a second integrated device or a second single piece of device, tool or apparatus to perform the functions performed by the third, fourth and fifth devices, tools or apparatus. Alternatively, the second, third, fourth and fifth devices, tools or apparatus may be combined into an integrated device or a single piece of device, tool or apparatus to perform the functions performed by the second, third, fourth and fifth devices, tools or apparatus. Alternatively, the second, third and fourth devices, tools or apparatus may be combined into an integrated device or a single piece of device, tool or apparatus to perform the functions performed by the second, third and fourth devices, tools or apparatus. Alternatively, the third and fourth devices, tools or apparatus may be combined into an integrated device or a single piece of device, tool or apparatus to perform the functions performed by the third and fourth devices, tools or apparatus. Alternatively, the fourth and fifth devices, tools or apparatus may be combined into an integrated device or a single piece of device, tool or apparatus to perform the functions performed by the fourth and fifth devices, tools or apparatus.

In this disclosure, the third device, tool or apparatus is used to obtain the count of cells per unit volume of the processed sample with sizes greater than or equal to the given threshold size (in this embodiment, the given threshold size equals 1 micrometer), and the fourth and fifth devices, tools or apparatus are used to obtain the percentage, also for the processed sample, of the selected type of stem cells in the cells with sizes greater than or equal to the given threshold size (in this embodiment, the given threshold size equals 1 micrometer). Multiplying the above obtained count of cells per unit volume of the processed sample by the above obtained percentage of the selected type of stem cells, the count of the selected type of stem cells per unit volume of the processed sample may be obtained. Multiplying the count of the selected type of stem cells per unit volume of the processed sample by the volume change (or sample preparation) factor, the count of the selected type of stem cells per unit volume of the blood sample may be obtained. By combining the cell count data from the third device, tool or apparatus and the percentage of the selected type of stem cells from the fourth and fifth devices, tools or apparatus, a more accurate count of the selected type of stem cells in the peripheral blood may be obtained.

Based on the above-mentioned approach depicted in Figs. 13, 14A, 14B and 14C, the present disclosure may use a single piece of system, device, tool or apparatus (TD) to obtain the stem-cell data depicted in the step 20 for the blood sample related the subject in Fig. 13. The single piece of system, device, tool or apparatus (TD) provides or performs: (1) a first function for obtaining or taking the blood sample from the subject as mentioned in the step 103 of Fig. 13; (2) a second function for processing the blood sample to obtain or prepare the processed sample (i.e., a test sample) as mentioned in the steps 1-8 of Fig. 14A; (3) a third function for obtaining a first data (e.g., the count of the ≥ 1-µm cells per milliliter of the processed sample, obtained in the step 14 of Fig. 14B) from the first portion of the processed sample by, e.g., a first method mentioned in the steps 9-14 of Fig. 14B; (4) a fourth function for obtaining a second data (e.g., the stem-cell percentage SP for a selected type of stem cells) and a third data (e.g., the percentage of the cell count C2 for the selected type of stem cells to the cell count C1 for the selected type of stem cells) from the second portion of the processed sample by, e.g., a second method mentioned in the steps 15-20 of Fig. 14C; (5) a fifth function for performing a first operation or calculation of the second and third data, such as multiplying the second data by the third data (e.g., multiplying the stem-cell percentage SP by the percentage of the cell count C2 to the cell count C1) and multiplying the multiplied result by 100%, to obtain a fourth data (e.g., the percentage of the count of the selected type of stem cells to the cell count C1) relating to the processed sample; (6) a sixth function for performing a second operation or calculation of the first and fourth data, such as multiplying the first data by the fourth data (e.g., multiplying the count of the ≥ 1-µm cells per milliliter of the processed sample by the percentage of the count of the selected type of stem cells to the cell count C1), to obtain a fifth data (e.g., the count of the selected type of stem cells per milliliter of the processed sample) relating to the processed sample; (7) a seventh function for performing a third operation or calculation of the volume of the processed sample and the volume of the blood sample, such as dividing the volume of the processed sample by the volume of the blood sample, to obtain the volume change (or sample preparation) factor (e.g., the ratio of the volume of the processed sample to the volume of the blood sample); and (8) an eighth function for performing a fourth operation or calculation of the fifth data and the volume change (or sample preparation) factor, such as multiplying the fifth data by the volume change (or sample preparation) factor (e.g., multiplying the count of the selected type of stem cells per milliliter of the processed sample by the ratio of the volume of the processed sample to the volume of the blood sample), to obtain a sixth data (e.g., the count of the selected type of stem cells per milliliter of the blood sample) relating to the blood sample.

The third function provided by the single piece of system, device, tool or apparatus (TD) is used to obtain the count of cells per unit volume of the processed sample with sizes greater than or equal to the given threshold size (in this embodiment, the given threshold size equals 1 micrometer), and the fourth and fifth functions provided by the single piece of system, device, tool or apparatus (TD) are used to obtain the percentage, also for the processed sample, of the selected type of stem cells in the cells with sizes greater than or equal to the given threshold size (in this embodiment, the given threshold size equals 1 micrometer). By multiplying the above obtained count of cells per unit volume of the processed sample by the above obtained percentage of the selected type of stem cells, the count of the selected type of stem cells per unit volume of the processed sample may be obtained. By multiplying the count of the selected type of stem cells per unit volume of the processed sample by the volume change (or sample preparation) factor, the count of the selected type of stem cells per unit volume of the blood sample may be obtained. By combining the cell count data from the third function performed by the single piece of system, device, tool or apparatus (TD) and the percentage of the selected type of stem cells from the fourth fifth functions performed by the single piece of system, device, tool or apparatus (TD), a more accurate count of the selected type of stem cells in the peripheral blood may be obtained.

Alternatively, according to an embodiment of the present disclosure, any combination of two or more of the above-mentioned first through eighth functions may be provided or performed by a single piece of system, device, tool or apparatus. In one example, the single piece of system, device, tool or apparatus provides or performs the third and fourth functions. In another example, the single piece of system, device, tool or apparatus provides or performs the second, third and fourth functions. In another example, the single piece of system, device, tool or apparatus provides or performs the third, fourth, fifth, sixth, and eighth functions. In another example, the single piece of system, device, tool or apparatus provides or performs the third through eighth functions.

Fig. 17A shows stem-cell data related to a man in mid-fifties, acting as the experimental subject, at 1.5 hours after ingestion of brown algae fucoidan supplement. The stem-cell data shown in Fig. 17A are obtained by performing the method depicted in Fig. 13 on the man. In this case, the man orally takes 30 pills of brown algae fucoidan supplement, which may be the same as that depicted in Figs. 11 and 12, in the step 101. Therefore, the man takes 3 grams (i.e., greater than 2 grams) of fucoidan in the step 101. In the step 102, the man waits for 1.5 hours after taking 30 pills of brown algae fucoidan supplement. In the step 103, a 10-mL blood sample is obtained by extracting or taking peripheral blood of the man to be placed into a blood collection tube. The 10-mL blood sample is processed to a 3-mL processed sample following the steps 1-8 in Fig. 14A. The 3-mL processed sample is divided into a first portion for the steps 9-14 in Fig. 14B and a second portion for the steps 15-19 in Fig. 14C. After the steps 9-14 in Fig. 14B are performed on the first portion of the 3-mL processed sample, the count of the ≥ 1-µm cells per milliliter of the 3-mL processed sample is obtained, as shown in column (A) in Fig. 17A. After the steps 15-19 in Fig. 14C are performed on the second portion of the 3-mL processed sample, the cell count C1, the cell count C2 and the stem-cell percentage SP for each type of stem cells (total 5 types of stem cells as shown in Fig. 17A) are obtained, as shown in columns (B), (C) and (E) in Fig. 17A, respectively. In this case, the flow cytometer in the step 19 analyzes each of the second test samples (acting as flow-cytometer samples), obtained by performing the steps 15-18 on the second portion of the 3-mL processed sample, until the corresponding cell count C1 reaches one million or the corresponding cell count C2 reaches 2,500. Accordingly, multiple sets of data related to the respective second test samples, obtained by performing the steps 15-18 on the second portion of the 3-mL processed sample, are obtained.

After the count of the ≥ 1-µm cells per milliliter of the 3-mL processed sample is obtained from the step 14 and the cell counts C1, the cell counts C2, and the stem-cell percentage SP for the each type of stem cells are obtained from the step 19, the stem-cell data related to the man at 1.5 hours after ingestion of the brown algae fucoidan supplement are obtained in the step 20. Here the stem-cell data include the count of each type of stem cells (total 5 types of stem cells as depicted in Fig. 17A) per milliliter of peripheral blood of the man at 1.5 hours after ingestion of the brown algae fucoidan supplement. Taking the CD24(+) progenitor stem cell as an example, referring to Fig. 17A, the count of the CD24(+) progenitor stem cells per milliliter of peripheral blood of the man at 1.5 hours after ingestion of the brown algae fucoidan supplement is calculated by: (1) dividing 2,500 cells (i.e., the cell count C2 in the region P4 of high nucleus/cytoplasm ratio), as shown in row (1) and column (C), by 995,918 cells (i.e., the cell count C1 in the threshold region of cells having sizes greater than or equal to 1 micrometer), as shown in row (1) and column (B), and multiplying the divided result (i.e., 0.0025) by 100% (which converts the divided result into percent figure) so as to obtain 0.25% (i.e., the percentage of the cell count C2 to the cell count C1), as shown in row (1) and column (D); (2) multiplying 0.25%, as shown in row (1) and column (D), by 46% (i.e., the percentage of the count of the CD24(+) progenitor stem cells to the cell count C2), as shown in row (1) and column (E), and multiplying the multiplied result (i.e., 0.00115) by 100% (which converts the multiplied result into percent figure) so as to obtain 0.1150% (i.e., the percentage of the count of the CD24(+) progenitor stem cells to the cell count C1), as shown in row (1) and column (F); (3) multiplying 0.1150%, as shown in row (1) and column (F), by 1.77E+08 of the ≥ 1-µm cells per milliliter of the 3-mL processed sample, as shown in row (1) and column (A), so as to obtain 203,550 of the CD24(+) progenitor stem cells per milliliter of the 3-mL processed sample, as shown in row (1) and column (G); (4) dividing 3 mL (i.e., the volume of the processed sample) by 10 mL (i.e., the volume of the blood sample) so as to obtain 0.3, i.e., the volume change (or sample preparation) factor; and (5) multiplying 203,550 of the CD24(+) progenitor stem cells per milliliter of the 3-mL processed sample, as shown in row (1) and column (G), by 0.3 (i.e., the volume change (or sample preparation) factor) so as to obtain 61,065 of the CD24(+) progenitor stem cells per milliliter of the 10-mL blood sample, which represents the count of the CD24(+) progenitor stem cells per milliliter of peripheral blood of the man at 1.5 hours after ingestion of the brown algae fucoidan supplement, as shown in row (1) and column (H). The count of each other type of stem cells per milliliter of peripheral blood of the man at 1.5 hours after ingestion of the brown algae fucoidan supplement may be calculated in a similar way.

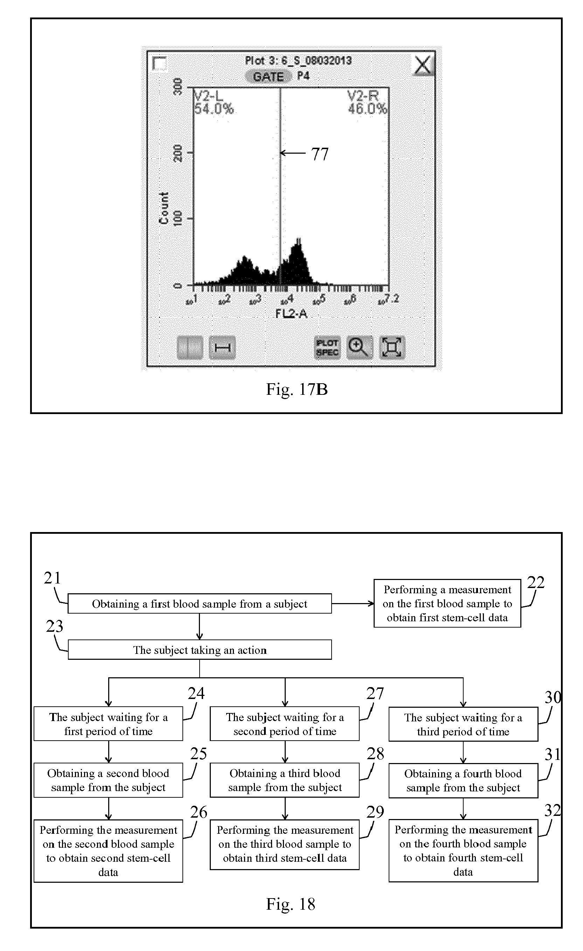

Fig. 17B is a fluorescence graph of the flow cytometer, which shows a fluorescence intensity distribution of cells in the region of high nucleus/cytoplasm ratio. The cells in the fluorescence graph are separated into right and left portions by a vertical line 77. The cells in the right portion of the vertical line 77 represent cells stained positive for CD24, i.e., the CD24(+) progenitor stem cells, and the cells in the left portion of the vertical line 77 represent cells stained negative for CD24. Therefore, the flow cytometer identifies the CD24(+) progenitor stem cells using the vertical line 77 so as to obtain the count of the CD24(+) progenitor stem cells depicted in row (1) and column (E) of Fig. 17A.

Fig. 18 shows a method for obtaining stem-cell data related to an experimental subject, such as the above-mentioned subject (S), at four specific time points in order to identify an optimal harvesting time point after one or more of the actions or stimuli (X), which may include a series of actions selected from the actions or stimuli (X), taken or subjected to by the experimental subject when one or more specific types of stem cells are optimal to be harvested by extracting a tissue sample, as illustrated in the paragraphs in "Description of subject (S) and tissue sample (P)", from the experimental subject or from a specific subject having the same biological category as the experimental subject. Referring to Fig. 18, in step 21, a first blood sample is obtained by extracting or taking peripheral blood of the experimental subject at a first specific time point to be placed into a blood collection tube such as EDTA tube. The volume of the first blood sample may be greater than or equal to 5 milliliters or 10 milliliters. Next, in step 22, the above-mentioned measurement MT is performed on the first blood sample to obtain first stem-cell data related to the experimental subject at the first specific time point. The first stem-cell data may include the count of a specific type of stem cells per unit volume (e.g., milliliter) of peripheral blood of the experimental subject at the first specific time point, i.e., a first obtained data relating to the count per unit volume (e.g., milliliter) of the specific type of stem cells in the peripheral blood of the experimental subject. Here the specific type of stem cells may be any one of the types of stem cells as mentioned in the paragraphs in "Description of stem cells" or may be stem cells characterized by one or more common cell (surface) makers, e.g., including CD349(+), Lgr5(+) or CD66e(+), as mentioned in the paragraphs in "Description of stem cells". The first blood sample may be processed and estimated in the same way as illustrated in Figs. 14A-14C. The first blood sample may be kept at between 1°C and 30°C (such as between 1°C and 10°C, between 10°C and 20°C, or between 20°C and 30°C) before the measurement MT in the step 22 is performed on it. The time period between obtaining the first blood sample in the step 21 and performing the measurement MT in the step 22 on the first blood sample may be controlled to be equal to or less than 30 minutes, 60 minutes, 12 hours, 24 hours, 48 hours, 72 hours, or 1 week (such as between 10 and 30 minutes, between 30 and 60 minutes, between 0.5 and 2 hours, between 2 and 12 hours, between 12 and 24 hours, between 24 and 48 hours, or between 48 and 72 hours). Alternatively, the first stem-cell data may be obtained by the integrated system, apparatus, device or tool (AD) or the single piece of system, device, tool or apparatus (TD).

After the first blood sample depicted in the step 21 is obtained, step 23 is performed. In the step 23, the experimental subject takes or is subjected to one or more of the above-mentioned actions or stimuli (X), which may include a series of actions selected from the actions or stimuli (X). For example, the experimental subject orally takes 30 pills of the brown algae fucoidan supplement depicted in the above-mentioned first experiment and Fig. 11; therefore, the experimental subject takes 3 grams (i.e., greater than 2 grams) of fucoidan in the step 23. Alternatively, the experimental subject injects a substance (e.g., a nutrient or supplement) containing fucoidan or oligo fucoidan. The first specific time point depicted in the step 21 may be a time point before the step 23. After the experimental subject takes or is subjected to the one or more of the actions or stimuli (X), a first method including following steps 24-26, a second method including following steps 27-29, and a third method including following steps 30-32 are performed, respectively.

Regarding the first method, in the step 24, the experimental subject waits for a first period of time, or a first predetermined time interval, (e.g., 1.5 hours, between 30 minutes and 3 hours, between 1 hour and 2 hours, or between 45 minutes and 12 hours) after taking or being subjected to the one or more actions or stimuli depicted in the step 23. Next, in the step 25, a second blood sample is obtained by extracting or taking peripheral blood of the experimental subject at a second specific time point to be placed into a blood collection tube such as EDTA tube. The volume of the second blood sample may be greater than or equal to 5 milliliters or 10 milliliters and may be the same as that of the first blood sample. The second specific time point is a time point at the end of the first period of time. Next, in the step 26, the above-mentioned measurement MT is performed on the second blood sample to obtain second stem-cell data related to the experimental subject at the second specific time point. The second stem-cell data may include the count of the specific type of stem cells per unit volume (e.g., milliliter) of peripheral blood of the experimental subject at the second specific time point, i.e., a second obtained data relating to the count per unit volume (e.g., milliliter) of the specific type of stem cells in the peripheral blood of the experimental subject. The second blood sample may be processed and estimated in the same way as illustrated in Figs. 14A-14C. Before the measurement MT in the step 26 is performed on the second blood sample, the second blood sample may be kept at the same temperature as the first blood sample is kept before the measurement MT in the step 22 is performed on the first blood sample. The time period between obtaining the second blood sample in the step 25 and performing the measurement MT in the step 26 on the second blood sample may be controlled to be equal to the time period between obtaining the first blood sample in the step 21 and performing the measurement MT in the step 22 on the first blood sample. Alternatively, the second stem-cell data may be obtained by the integrated system, apparatus, device or tool (AD) or the single piece of system, device, tool or apparatus (TD).

Regarding the second method, in the step 27, the experimental subject waits for a second period of time, or a second predetermined time interval, (e.g., 24 hours, between 12 hours and 36 hours, or between 3 hours and 36 hours) after taking or being subjected to the one or more actions or stimuli depicted in the step 23. Next, in the step 28, a third blood sample is obtained by extracting or taking peripheral blood of the experimental subject at a third specific time point to be placed into a blood collection tube such as EDTA tube. The volume of the third blood sample may be greater than or equal to 5 milliliters or 10 milliliters and may be the same as that of the first blood sample and that of the second blood sample. The third specific time point is a time point at the end of the second period of time. Next, in the step 29, the above-mentioned measurement MT is performed on the third blood sample to obtain third stem-cell data related to the experimental subject at the third specific time point. The third stem-cell data may include the count of the specific type of stem cells per unit volume (e.g., milliliter) of peripheral blood of the experimental subject at the third specific time point, i.e., a third obtained data relating to the count per unit volume (e.g., milliliter) of the specific type of stem cells in the peripheral blood of the experimental subject. The third blood sample may be processed and estimated in the same way as illustrated in Figs. 14A-14C. Before the measurement MT in the step 29 is performed on the third blood sample, the third blood sample may be kept at the same temperature as the first blood sample is kept before the measurement MT in the step 22 is performed on the first blood sample. The time period between obtaining the third blood sample in the step 28 and performing the measurement MT in the step 29 on the third blood sample may be controlled to be equal to the time period between obtaining the first blood sample in the step 21 and performing the measurement MT in the step 22 on the first blood sample. Alternatively, the third stem-cell data may be obtained by the integrated system, apparatus, device or tool (AD) or the single piece of system, device, tool or apparatus (TD).

Regarding the third method, in the step 30, the experimental subject waits for a third period of time, or a third predetermined time interval, (e.g., 48 hours, between 36 hours and 60 hours, between 60 hours and 84 hours, or greater than 84 hours) after taking or being subjected to the one or more actions or stimuli depicted in the step 23. Next, in the step 31, a fourth blood sample is obtained by extracting or taking peripheral blood of the experimental subject at a fourth specific time point to be placed into a blood collection tube such as EDTA tube. The volume of the fourth blood sample may be greater than or equal to 5 milliliters or 10 milliliters and may be the same as that of the first blood sample, that of the second blood sample, and that of the third blood sample. The fourth specific time point is a time point at the end of the third period of time. Next, in the step 32, the above-mentioned measurement MT is performed on the fourth blood sample to obtain fourth stem-cell data related to the experimental subject at the fourth specific time point. The fourth stem-cell data may include the count of the specific type of stem cells per unit volume (e.g., milliliter) of peripheral blood of the experimental subject at the fourth specific time point, i.e., a fourth obtained data relating to the count per unit volume (e.g., milliliter) of the specific type of stem cells in the peripheral blood of the experimental subject. The fourth blood sample may be processed and estimated in the same way as illustrated in Figs. 14A-14C. Before the measurement MT in the step 32 is performed on the fourth blood sample, the fourth blood sample may be kept at the same temperature as the first blood sample is kept before the measurement MT in the step 22 is performed on the first blood sample. The time period between obtaining the fourth blood sample in the step 31 and performing the measurement MT in the step 32 on the fourth blood sample may be controlled to be equal to the time period between obtaining the first blood sample in the step 21 and performing the measurement MT in the step 22 on the first blood sample. Alternatively, the fourth stem-cell data may be obtained by the integrated system, apparatus, device or tool (AD) or the single piece of system, device, tool or apparatus (TD).

Based on the first through fourth stem-cell data obtained in the steps 22, 26, 29 and 32, the changes in the count per unit volume of the specific typed of stem cells may be obtained, including (1) the change Δ1 of the count of the specific type of stem cells per unit volume of the peripheral blood of the experimental subject at the second specific time point, in the second stem-cell data, relative to the count of the specific type of stem cells per unit volume of the peripheral blood of the experimental subject at the first specific time point, in the first stem-cell data, (2) the change Δ2 of the count of the specific type of stem cells per unit volume of the peripheral blood of the experimental subject at the third specific time point, in the third stem-cell data, relative to the count of the specific type of stem cells per unit volume of the peripheral blood of the experimental subject at the first specific time point, in the first stem-cell data, and (3) the change Δ3 of the count of the specific type of stem cells per unit volume of the peripheral blood of the experimental subject at the fourth specific time point, in the fourth stem-cell data, relative to the count of the specific type of stem cells per unit volume of the peripheral blood of the experimental subject at the first specific time point, in the first stem-cell data. The change Δ1 may be obtained by performing a first analysis based on the first obtained data obtained in the step 22 and the second obtained data obtained in the step 26, and the first analysis includes a comparison between the first obtained data and the second obtained data. The change Δ2 may be obtained by performing a second analysis based on the first obtained data obtained in the step 22 and the third obtained data obtained in the step 29, and the second analysis includes a comparison between the first obtained data and the third obtained data. The change Δ3 may be obtained by performing a third analysis based on the first obtained data obtained in the step 22 and the fourth obtained data obtained in the step 32, and the third analysis includes a comparison between the first obtained data and the fourth obtained data.

The greatest one of the changes Δ1-Δ3 for the specific type of stem cells may be determined by comparing each pair selected from the changes Δ1-Δ3, such as comparing the change Δ1 and the change Δ2, so as to identify or determine an optimal harvesting time period (e.g., the first period of time, the second period of time, or the third period of time) and/or an optimal harvesting time point (e.g., one of the second, third and fourth specific time points) for the specific type of stem cells related to the experimental subject. When the greatest one of the changes Δ1-Δ3 is the change Δ1, the optimal harvesting time period is the first period of time and the optimal harvesting time point is the second specific time point. When the greatest one of the changes Δ1-Δ3 is the change Δ2, the optimal harvesting time period is the second period of time and the optimal harvesting time point is the third specific time point. When the greatest one of the changes Δ1-Δ3 is the change Δ3, the optimal harvesting time period is the third period of time and the optimal harvesting time point is the fourth specific time point. Accordingly, when the specific type of stem cells is to be extracted from a certain subject, which may be the experimental subject or a subject having the same biological category as the experimental subject, a tissue sample, as illustrated in the paragraphs in "Description of subject (S) and tissue sample (P)", for the specific type of stem cells may be extracted from the certain subject at the optimal harvesting time point after the certain subject takes or is subjected to the one or more actions or stimuli depicted in the step 23. The method depicted in Fig. 18, including the steps 21-32, may be configured for monitoring the increasing number of stem cells of the specific type in the peripheral blood of the experimental subject after the experimental subject takes or is subjected to the one or more actions or stimuli depicted in the step 23 to cultivate in vivo the specific type of stem cells.

Alternatively, multiple experimental subjects having the same biological category may perform the same method illustrated in Fig. 18 so as to obtain first through fourth stem-cell data, depicted in Fig. 18, for each experimental subject. Based on the first through fourth stem-cell data related to the experimental subjects, the changes in the count per unit volume of the specific typed of stem cells may be obtained, including (1) the change Δ1 of the count of the specific type of stem cells per unit volume of the peripheral blood at the second specific time point, in the second stem-cell data, relative to the count of the specific type of stem cells per unit volume of the peripheral blood at the first specific time point, in the first stem-cell data, for each experimental subject, (2) the change Δ2 of the count of the specific type of stem cells per unit volume of the peripheral blood at the third specific time point, in the third stem-cell data, relative to the count of the specific type of stem cells per unit volume of the peripheral blood at the first specific time point, in the first stem-cell data, for each experimental subject, and (3) the change Δ3 of the count of the specific type of stem cells per unit volume of the peripheral blood at the fourth specific time point, in the fourth stem-cell data, relative to the count of the specific type of stem cells per unit volume of the peripheral blood at the first specific time point, in the first stem-cell data, for each experimental subject. The most statistically significant distribution for changes of the specific type of stem cells may be determined by comparing each pair selected from the distributions for the changes Δ1-Δ3 of the specific type of stem cells for the experimental subjects, such as comparing the distribution for the changes Δ1 of SB-1 cells and the distribution for the changes Δ2 of SB-1 cells, so as to identify or determine an optimal harvesting time period (e.g., the first period of time, the second period of time, or the third period of time) and/or an optimal harvesting time point (e.g., one of the second, third and fourth specific time points) for the specific type of stem cells related to the experimental subjects.

When the most statistically significant distribution for changes of the specific type of stem cells is the distribution for the changes Δ1 of the specific type of stem cells, the optimal harvesting time period is the first period of time and the optimal harvesting time point is the second specific time point. When the most statistically significant distribution for changes of the specific type of stem cells is the distribution for the changes Δ2 of the specific type of stem cells, the optimal harvesting time period is the second period of time and the optimal harvesting time point is the third specific time point. When the most statistically significant distribution for changes of the specific type of stem cells is the distribution for the changes Δ3 of the specific type of stem cells, the optimal harvesting time period is the third period of time and the optimal harvesting time point is the fourth specific time point. Accordingly, when the specific type of stem cells is to be extracted from a certain subject, which may be a subject having the same biological category as the experimental subjects or one of the experimental subjects, a tissue sample, as illustrated in the paragraphs in "Description of subject (S) and tissue sample (P)", for the specific type of stem cells may be extracted from the certain subject at the optimal harvesting time point after the certain subject takes or is subjected to the one or more actions or stimuli depicted in the step 23.

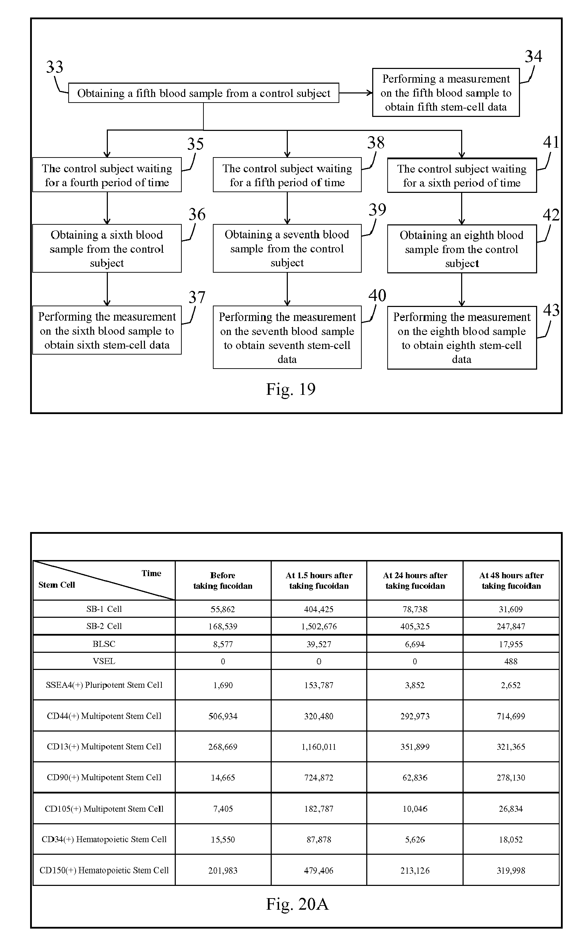

Fig. 19 shows a method for obtaining stem-cell data related to a control subject, such as the above-mentioned subject (S), at four specific time points to be compared with the stem-cell data related to the experimental subject obtained from the method illustrated in Fig. 18 for confirming if the change between two sets of the stem-cell data related to the experimental subject at two respective different time points after taking or being subjected to the one or more of the actions or stimuli (X) in the step 23 derives from the one or more of the actions or stimuli (X). The method for the control subject, as illustrated in Fig. 19, is similar to the method for the experimental subject, as illustrated in Fig. 18, but the control subject does not take or is not subjected to the one or more of the actions or stimuli (X) which the experimental subject takes or to which the experimental subject is subjected in the step 23 of the method in Fig. 18. The stem-cell data obtained from the method for the control subject are used as standards to evaluate changes of the stem-cell data obtained from the method for the experimental subject in Fig. 18.

Referring to Fig. 19, in step 33, a fifth blood sample is obtained by extracting or taking peripheral blood of the control subject at a fifth specific time point to be placed into a blood collection tube such as EDTA tube. The volume of the fifth blood sample may be greater than or equal to 5 milliliters or 10 milliliters and may be the same as that of the first blood sample depicted in the step 21 of Fig. 18. The fifth specific time point is a time point at the beginning of the method for the control subject. Next, in step 34, the above-mentioned measurement MT is performed on the fifth blood sample to obtain fifth stem-cell data related to the control subject at the fifth specific time point. The fifth stem-cell data may include the count of a specific type of stem cells per unit volume (e.g., milliliter) of peripheral blood of the control subject at the fifth specific time point, i.e., a fifth obtained data relating to the count per unit volume (e.g., milliliter) of the specific type of stem cells in the peripheral blood of the control subject. The fifth blood sample may be processed and estimated in the same way as illustrated in Figs. 14A-14C. The specific type of stem cells counted in the method for the control subject in Fig. 19 may be the same as that counted in the method for the experimental subject in Fig. 18. Before the measurement MT in the step 34 is performed on the fifth blood sample, the fifth blood sample may be kept at the same temperature as the first blood sample is kept before the measurement MT in the step 22 is performed on the first blood sample. The time period between obtaining the fifth blood sample in the step 33 and performing the measurement MT in the step 34 on the fifth blood sample may be controlled to be equal to the time period between obtaining the first blood sample in the step 21 and performing the measurement MT in the step 22 on the first blood sample. After the fifth blood sample is obtained in the step 33, a fourth method including following steps 35-37, a fifth method including following steps 38-40, and a sixth method including following steps 41-43 are performed, respectively.

Regarding the fourth method, in the step 35, the control subject waits for a fourth period of time, or a fourth predetermined time interval, (e.g., 1.5 hours, between 30 minutes and 3 hours, between 1 hour and 2 hours, or between 45 minutes and 12 hours) after the fifth blood sample is obtained in the step 33. The fourth period of time is the same in length as the first period of time depicted in the step 24 of Fig. 18. Next, in the step 36, a sixth blood sample is obtained by extracting or taking peripheral blood of the control subject at a sixth specific time point to be placed into a blood collection tube such as EDTA tube. The volume of the sixth blood sample may be greater than or equal to 5 milliliters or 10 milliliters and may be the same as that of the fifth blood sample. The sixth specific time point is a time point at the end of the fourth period of time. Next, in the step 37, the above-mentioned measurement MT is performed on the sixth blood sample to obtain sixth stem-cell data related to the control subject at the sixth specific time point. The sixth stem-cell data may include the count of the specific type of stem cells per unit volume (e.g., milliliter) of peripheral blood of the control subject at the sixth specific time point, i.e., a sixth obtained data relating to the count per unit volume (e.g., milliliter) of the specific type of stem cells in the peripheral blood of the control subject. The sixth blood sample may be processed and estimated in the same way as illustrated in Figs. 14A-14C. Before the measurement MT in the step 37 is performed on the sixth blood sample, the sixth blood sample may be kept at the same temperature as the first blood sample is kept before the measurement MT in the step 22 is performed on the first blood sample. The time period between obtaining the sixth blood sample in the step 36 and performing the measurement MT in the step 37 on the sixth blood sample may be controlled to be equal to the time period between obtaining the first blood sample in the step 21 and performing the measurement MT in the step 22 on the first blood sample.

Regarding the fifth method, in the step 38, the control subject waits for a fifth period of time, or a fifth predetermined time interval, (e.g., 24 hours, between 12 hours and 36 hours, or between 3 hours and 36 hours) after the fifth blood sample is obtained in the step 33. The fifth period of time is the same in length as the second period of time depicted in the step 27 of Fig. 18. Next, in the step 39, a seventh blood sample is obtained by extracting or taking peripheral blood of the control subject at a seventh specific time point to be placed into a blood collection tube such as EDTA tube. The volume of the seventh blood sample may be greater than or equal to 5 milliliters or 10 milliliters and may be the same as that of the fifth blood sample and that of the sixth blood sample. The seventh specific time point is a time point at the end of the fifth period of time. Next, in the step 40, the above-mentioned measurement MT is performed on the seventh blood sample to obtain seventh stem-cell data related to the control subject at the seventh specific time point. The seventh stem-cell data may include the count of the specific type of stem cells per unit volume (e.g., milliliter) of peripheral blood of the control subject at the seventh specific time point, i.e., a seventh obtained data relating to the count per unit volume (e.g., milliliter) of the specific type of stem cells in the peripheral blood of the control subject. The seventh blood sample may be processed and estimated in the same way as illustrated in Figs. 14A-14C. Before the measurement MT in the step 40 is performed on the seventh blood sample, the seventh blood sample may be kept at the same temperature as the first blood sample is kept before the measurement MT in the step 22 is performed on the first blood sample. The time period between obtaining the seventh blood sample in the step 39 and performing the measurement MT in the step 40 on the seventh blood sample may be controlled to be equal to the time period between obtaining the first blood sample in the step 21 and performing the measurement MT in the step 22 on the first blood sample.

Regarding the sixth method, in the step 41, the control subject waits for a sixth period of time, or a sixth predetermined time interval, (e.g., 48 hours, between 36 hours and 60 hours, between 60 hours and 84 hours, or greater than 84 hours) after the fifth blood sample is obtained in the step 33. The sixth period of time is the same in length as the third period of time depicted in the step 30 of Fig. 18. Next, in the step 42, an eighth blood sample is obtained by extracting or taking peripheral blood of the control subject at an eighth specific time point to be placed into a blood collection tube such as EDTA tube. The volume of the eighth blood sample may be greater than or equal to 5 milliliters or 10 milliliters and may be the same as that of the fifth blood sample, that of the sixth blood sample, and that of the seventh blood sample. The eighth specific time point is a time point at the end of the sixth period of time. Next, in the step 43, the above-mentioned measurement MT is performed on the eighth blood sample to obtain eighth stem-cell data related to the control subject at the eighth specific time point. The eighth stem-cell data may include the count of the specific type of stem cells per unit volume (e.g., milliliter) of peripheral blood of the control subject at the eighth specific time point, i.e., an eighth obtained data relating to the count per unit volume (e.g., milliliter) of the specific type of stem cells in the peripheral blood of the control subject. The eighth blood sample may be processed and estimated in the same way as illustrated in Figs. 14A-14C. Before the measurement MT in the step 43 is performed on the eighth blood sample, the eighth blood sample may be kept at the same temperature as the first blood sample is kept before the measurement MT in the step 22 is performed on the first blood sample. The time period between obtaining the eighth blood sample in the step 42 and performing the measurement MT in the step 43 on the eighth blood sample may be controlled to be equal to the time period between obtaining the first blood sample in the step 21 and performing the measurement MT in the step 22 on the first blood sample.

Based on the first through fourth stem-cell data obtained in the steps 22, 26, 29 and 32 and the fifth through eighth stem-cell data obtained in the steps 34, 37, 40 and 43, the changes in the count of the specific typed of stem cells may be obtained, including (1) the change Δ1 of the count of the specific type of stem cells per unit volume of the peripheral blood of the experimental subject at the second specific time point, in the second stem-cell data, relative to the count of the specific type of stem cells per unit volume of the peripheral blood of the experimental subject at the first specific time point, in the first stem-cell data, (2) the change Δ2 of the count of the specific type of stem cells per unit volume of the peripheral blood of the experimental subject at the third specific time point, in the third stem-cell data, relative to the count of the specific type of stem cells per unit volume of the peripheral blood of the experimental subject at the first specific time point, in the first stem-cell data, (3) the change Δ3 of the count of the specific type of stem cells per unit volume of the peripheral blood of the experimental subject at the fourth specific time point, in the fourth stem-cell data, relative to the count of the specific type of stem cells per unit volume of the peripheral blood of the experimental subject at the first specific time point, in the first stem-cell data, (4) the change δ1 of the count of the specific type of stem cells per unit volume of the peripheral blood of the control subject at the sixth specific time point, in the sixth stem-cell data, relative to the count of the specific type of stem cells per unit volume of the peripheral blood of the control subject at the fifth specific time point, in the fifth stem-cell data, (5) the change δ2 of the count of the specific type of stem cells per unit volume of the peripheral blood of the control subject at the seventh specific time point, in the seventh stem-cell data, relative to the count of the specific type of stem cells per unit volume of the peripheral blood of the control subject at the fifth specific time point, in the fifth stem-cell data, and (6) the change δ3 of the count of the specific type of stem cells per unit volume of the peripheral blood of the control subject at the eighth specific time point, in the eighth stem-cell data, relative to the count of the specific type of stem cells per unit volume of the peripheral blood of the control subject at the fifth specific time point, in the fifth stem-cell data.

The change Δ1 may be obtained by performing a first analysis based on the first obtained data obtained in the step 22 and the second obtained data obtained in the step 26, and the first analysis includes a comparison between the first obtained data and the second obtained data. The change Δ2 may be obtained by performing a second analysis based on the first obtained data obtained in the step 22 and the third obtained data obtained in the step 29, and the second analysis includes a comparison between the first obtained data and the third obtained data. The change Δ3 may be obtained by performing a third analysis based on the first obtained data obtained in the step 22 and the fourth obtained data obtained in the step 32, and the third analysis includes a comparison between the first obtained data and the fourth obtained data. The change δ1 may be obtained by performing a fourth analysis based on the fifth obtained data obtained in the step 34 and the sixth obtained data obtained in the step 37, and the fourth analysis includes a comparison between the fifth obtained data and the sixth obtained data. The change δ2 may be obtained by performing a fifth analysis based on the fifth obtained data obtained in the step 34 and the seventh obtained data obtained in the step 40, and the fifth analysis includes a comparison between the fifth obtained data and the seventh obtained data. The change δ3 may be obtained by performing a sixth analysis based on the fifth obtained data obtained in the step 34 and the eighth obtained data obtained in the step 43, and the sixth analysis includes a comparison between the fifth obtained data and the eighth obtained data.

The greatest one of the changes Δ1-Δ3 for the specific type of stem cells may be determined by comparing each pair selected from the changes Δ1-Δ3 and then may be compared to the corresponding one of the changes δ1-δ3 to confirm that the greatest one of the changes Δ1-Δ3 is caused by the one or more actions or stimuli depicted in the step 23. If the greatest one of the changes Δ1-Δ3 is (statistically) significantly better than the corresponding one of the changes δ1-δ3 the greatest one of the changes Δ1-Δ3 may be confirmed to be caused by the one or more actions or stimuli depicted in the step 23. When the greatest one of the changes Δ1-Δ3 is the change Δ1, the corresponding one of the changes δ1-δ3 is the change δ1. When the greatest one of the changes Δ1-Δ3 is the change Δ2, the corresponding one of the changes δ1-δ3 is the change δ2. When the greatest one of the changes Δ1-Δ3 is the change Δ3, the corresponding one of the changes δ1-δ3 is the change δ3. Thereby, an optimal harvesting time period for the specific type of stem cells may be obtained and may be (1) the first period of time depicted in the step 24 when the greatest one of the changes Δ1-Δ3 is the change Δ1 and is (statistically) significantly better than the change δ1, (2) the second period of time depicted in the step 27 when the greatest one of the changes Δ1-Δ3 is the change Δ2 and is (statistically) significantly better than the change δ2, or (3) the third period of time depicted in the step 30 when the greatest one of the changes Δ1-Δ3 is the change Δ3 and is (statistically) significantly better than the change δ3. In addition, an optimal harvesting time point for the specific type of stem cells may be obtained and may be (1) the second specific time point depicted in the step 25 when the greatest one of the changes Δ1-Δ3 is the change Δ1 and is (statistically) significantly better than the change δ1, (2) the third specific time point depicted in the step 28 when the greatest one of the changes Δ1-Δ3 is the change Δ2 and is (statistically) significantly better than the change δ2, or (3) the fourth specific time point depicted in the step 31 when the greatest one of the changes Δ1-Δ3 is the change Δ3 and is (statistically) significantly better than the change δ3. If the greatest one of the changes Δ1-Δ3 is not (statistically) significantly better than the corresponding one of the changes δ1-δ3, the greatest one of the changes Δ1-Δ3 may not be confirmed to be caused by the one or more actions or stimuli depicted in the step 23.

Accordingly, when the specific type of stem cells is to be extracted from a certain subject, which may be the experimental subject or a subject having the same biological category as the experimental subject, a tissue sample, as illustrated in the paragraphs in "Description of subject (S) and tissue sample (P)", for the specific type of stem cells may be extracted from the certain subject at the optimal harvesting time point after the certain subject takes or is subjected to the one or more actions or stimuli depicted in the step 23.

Alternatively, multiple control subjects having the same biological category may perform the same method illustrated in Fig. 19 so as to obtain fifth through eighth stem-cell data, depicted in Fig. 19, for each control subject, and multiple experimental subjects having the same biological category as the control subjects may perform the same method illustrated in Fig. 18 so as to obtain first through fourth stem-cell data, depicted in Fig. 18, for each experimental subject. Based on the first through fourth stem-cell data related to the experimental subjects and the fifth through eighth stem-cell data related to the control subjects, the changes in the count of the specific typed of stem cells may be obtained, including (1) the change Δ1 of the count of the specific type of stem cells per unit volume of the peripheral blood at the second specific time point, in the second stem-cell data, relative to the count of the specific type of stem cells per unit volume of the peripheral blood at the first specific time point, in the first stem-cell data, for each experimental subject, (2) the change Δ2 of the count of the specific type of stem cells per unit volume of the peripheral blood at the third specific time point, in the third stem-cell data, relative to the count of the specific type of stem cells per unit volume of the peripheral blood at the first specific time point, in the first stem-cell data, for each experimental subject, (3) the change Δ3 of the count of the specific type of stem cells per unit volume of the peripheral blood at the fourth specific time point, in the fourth stem-cell data, relative to the count of the specific type of stem cells per unit volume of the peripheral blood at the first specific time point, in the first stem-cell data, for each experimental subject, (4) the change δ1 of the count of the specific type of stem cells per unit volume of the peripheral blood at the sixth specific time point, in the sixth stem-cell data, relative to the count of the specific type of stem cells per unit volume of the peripheral blood at the fifth specific time point, in the fifth stem-cell data, for each control subject, (5) the change δ2 of the count of the specific type of stem cells per unit volume of the peripheral blood at the seventh specific time point, in the seventh stem-cell data, relative to the count of the specific type of stem cells per unit volume of the peripheral blood at the fifth specific time point, in the fifth stem-cell data, for each control subject, and (6) the change δ3 of the count of the specific type of stem cells per unit volume of the peripheral blood at the eighth specific time point, in the eighth stem-cell data, relative to the count of the specific type of stem cells per unit volume of the peripheral blood at the fifth specific time point, in the fifth stem-cell data, for each control subject.

The most statistically significant distribution for changes of the specific type of stem cells may be determined by comparing each pair selected from the distributions for the changes Δ1-Δ3 of the specific type of stem cells for the experimental subjects, such as comparing the distribution for the changes Δ1 of SB-1 cells and the distribution for the changes Δ2 of SB-1 cells, and then compared to the corresponding one of the distributions for the changes δ1-δ3 of the specific type of stem cells for the control subjects, such as to be compared to the distribution for the changes δ1 of SB-1 cells if the distribution for the changes Δ1 of SB-1 cells is the most statistically significant distribution for changes of SB-1 cells, to confirm that the most statistically significant distribution for changes of the specific type of stem cells, such as the distribution for the changes Δ1 of SB-1 cells, is caused by the one or more actions or stimuli depicted in the step 23. If the most statistically significant distribution for changes of the specific type of stem cells, such as the distribution for the changes Δ1 of SB-1 cells, is statistically significantly better than the corresponding one of the distributions for the changes δ1-δ3 of the specific type of stem cells, such as the distribution for the changes δ1 of SB-1 cells, the most statistically significant distribution for changes of the specific type of stem cells, such as the distribution for the changes Δ1 of SB-1 cells, may be confirmed to be caused by the one or more actions or stimuli depicted in the step 23. When the most statistically significant distribution for changes of the specific type of stem cells is the distribution for the changes Δ1 of the specific type of stem cells, the corresponding one of the distributions for the changes δ1-δ3 of the specific type of stem cells is the distribution for the changes δ1 of the specific type of stem cells. When the most statistically significant distribution for changes of the specific type of stem cells is the distribution for the changes Δ2 of the specific type of stem cells, the corresponding one of the distributions for the changes δ1-δ3 of the specific type of stem cells is the distribution for the changes δ2 of the specific type of stem cells. When the most statistically significant distribution for changes of the specific type of stem cells is the distribution for the changes Δ3 of the specific type of stem cells, the corresponding one of the distributions for the changes δ1-δ3 of the specific type of stem cells is the distribution for the changes δ3 of the specific type of stem cells. If the most statistically significant distribution for changes of the specific type of stem cells is not statistically significantly better than the corresponding one of the distributions for the changes δ1-δ3 of the specific type of stem cells, the most statistically significant distribution for changes of the specific type of stem cells may not be confirmed to be caused by the one or more actions or stimuli depicted in the step 23. Thereby, an optimal harvesting time period and time point for the specific type of stem cells related to the experimental subjects are obtained and may be (1) the first period of time and the second specific time point depicted in the steps 24 and 25 when the most statistically significant distribution for changes of the specific type of stem cells is the distribution for the changes Δ1 of the specific type of stem cells and is statistically significantly better than the distribution for the changes δ1 of the specific type of stem cells, (2) the second period of time and the third specific time point depicted in the steps 27 and 28 when the most statistically significant distribution for changes of the specific type of stem cells is the distribution for the changes Δ2 of the specific type of stem cells and is statistically significantly better than the distribution for the changes δ2 of the specific type of stem cells, or (3) the third period of time and the fourth specific time point depicted in the steps 30 and 31 when the most statistically significant distribution for changes of the specific type of stem cells is the distribution for the changes Δ3 of the specific type of stem cells and is statistically significantly better than the distribution for the changes δ3 of the specific type of stem cells. Accordingly, when the specific type of stem cells is to be extracted from a certain subject, which may be a subject having the same biological category as the experimental subjects or one of the experimental subjects, a tissue sample, as illustrated in the paragraphs in "Description of subject (S) and tissue sample (P)", for the specific type of stem cells may be extracted from the certain subject at the optimal harvesting time point after the certain subject takes or is subjected to the one or more actions or stimuli depicted in the step 23.

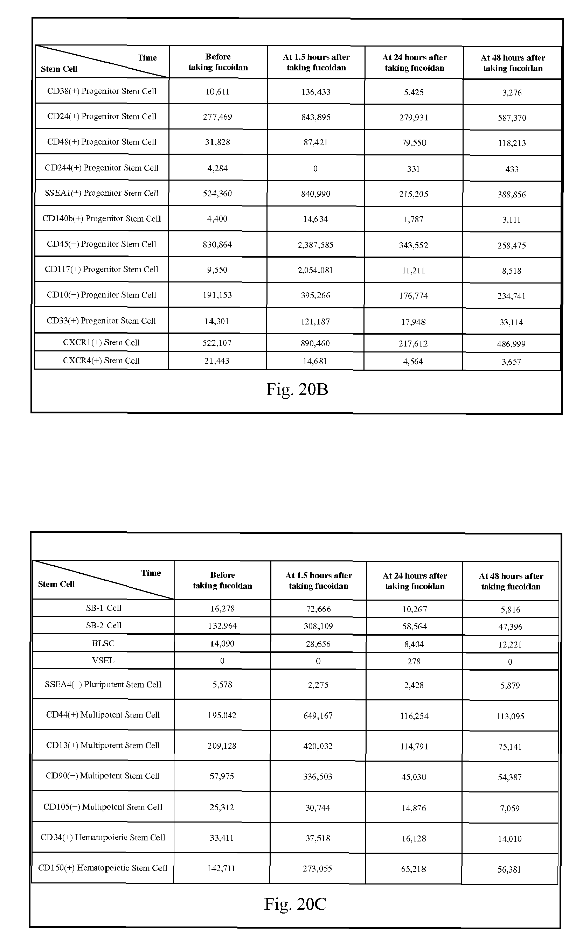

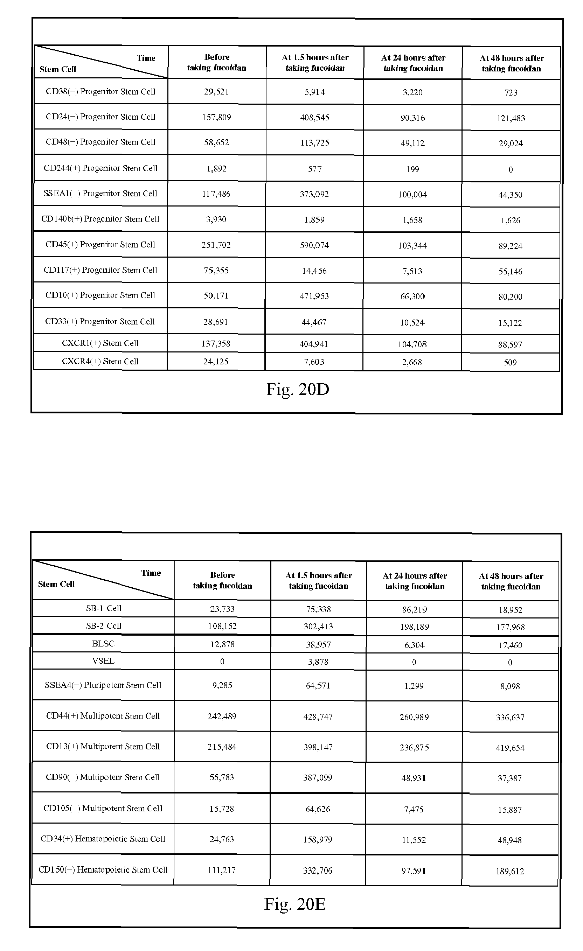

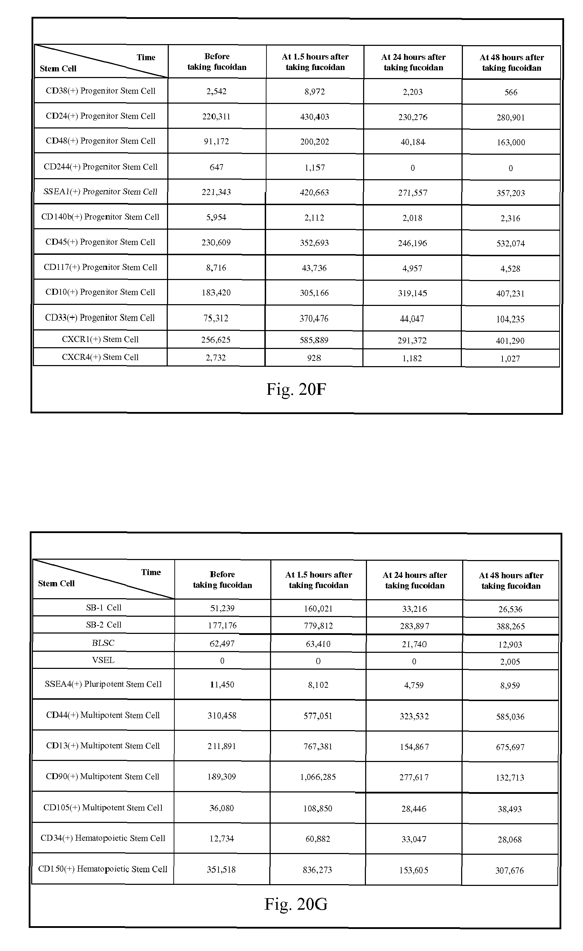

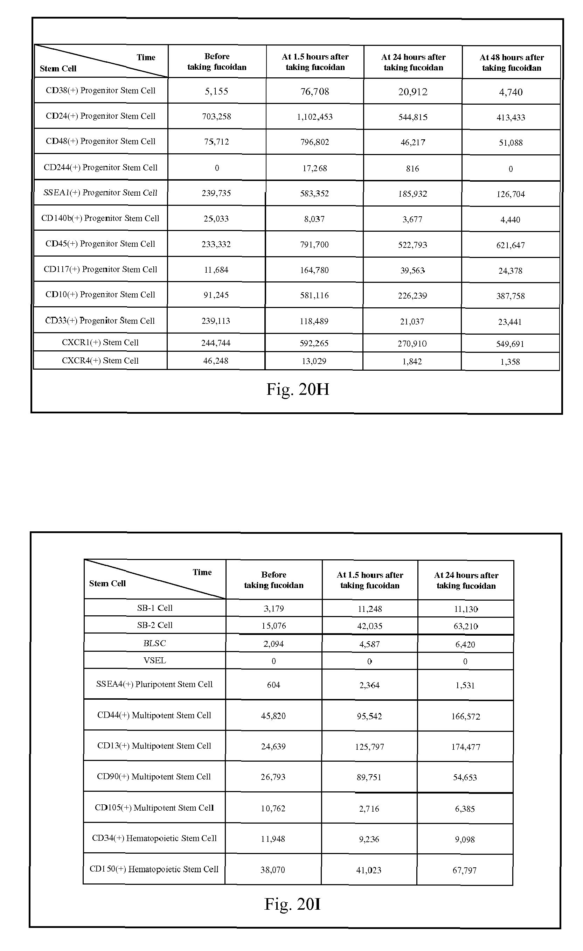

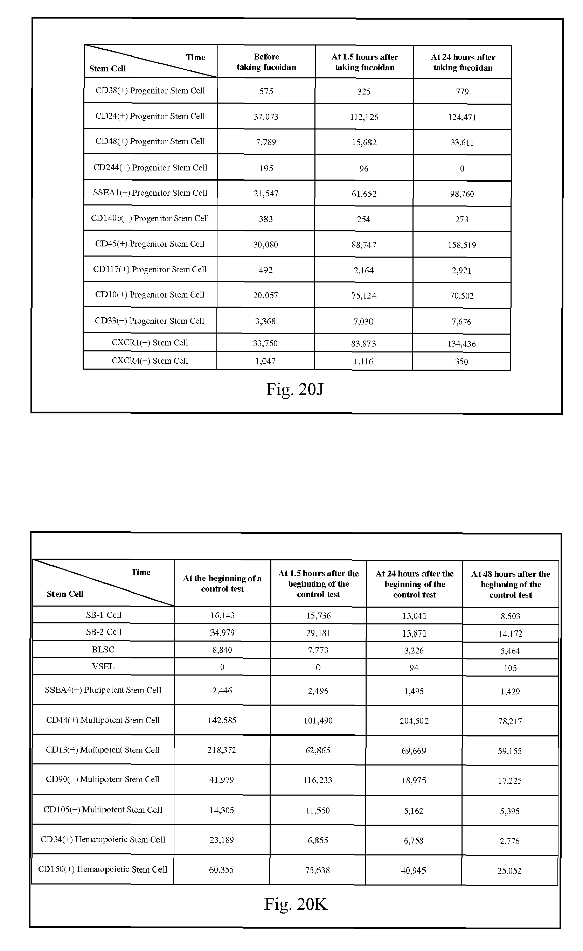

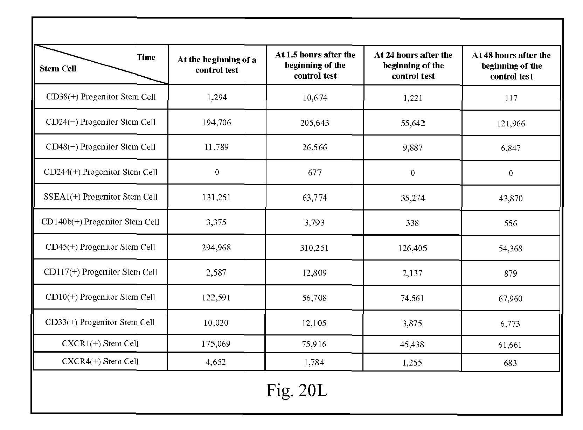

In the following, a second experiment and related stem-cell data were shown, as an example, as a method to obtain the count of stem cells per milliliter of peripheral blood of respective human bodies. In the second experiment, five human subjects were selected as experimental subjects in an evaluation group and performed the above method depicted in Fig. 18, and a human subject (hereinafter the "Subject CP") was selected as a control subject for the second experiment and performed the above method depicted in Fig. 19. The five human subjects in the evaluation group are: (1) Subject E1: a man in mid-fifties, whose stem-cell data are shown in Figs. 20A and 20B; (2) Subject E2: a man in early forties, whose stem-cell data are shown in Figs. 20C and 20D; (3) Subject E3: a man in mid-fifties, whose stem-cell data are shown in Figs. 20E and 20F; (4) Subject E4: a man in mid-thirties, whose stem-cell data are shown in Figs. 20G and 20H; and (5) Subject E5: a woman in mid-twenties, whose stem-cell data are shown in Figs. 20I and 20J. The Subject CP is a woman in mid-twenties, whose stem-cell data are shown in Figs. 20K and 20L.

The method depicted in the steps 21-32 of Fig. 18 was performed on each of the Subjects E1-E4 so as to obtain stem-cell data at four specific time points for each of the Subjects E1-E4. The steps 21-29 of the method depicted in Fig. 18 were performed on the Subject E5 so as to obtain stem-cell data related to the Subject E5 at three specific time points. In the method for each of the experimental subjects, i.e., the Subjects E1-E5, each of the Subjects E1-E5 orally took 30 pills of a brown algae fucoidan supplement, which may be the same as that depicted in Figs. 11 and 12, in the step 23 of Fig. 18. Also, each of the Subjects E1-E5 took 3 grams (i.e., greater than 2 grams) of fucoidan in the step 23 of Fig. 18. In addition, each of the Subjects E1-E5 weighed about 65 Kg. That means the dose of fucoidan is about 46 mg per Kg body weight. Alternatively, the fucoidan dose may be greater than 20, 30, 40 or 50 mg per Kg body weight.

Figs. 20A and 20B show the count of each type of stem cells (total 23 types of stem cells) per milliliter of peripheral blood of the Subject E1 at the four specific time points. Figs. 20C and 20D show the count of each type of stem cells (total 23 types of stem cells) per milliliter of peripheral blood of the Subject E2 at the four specific time points. Figs. 20E and 20F show the count of each type of stem cells (total 23 types of stem cells) per milliliter of peripheral blood of the Subject E3 at the four specific time points. Figs. 20G and 20H show the count of each type of stem cells (total 23 types of stem cells) per milliliter of peripheral blood of the Subject E4 at the four specific time points. For each of the Subjects E1-E4, the four specific time points include (1) a first time point before ingestion of the brown algae fucoidan supplement (i.e., the first specific time point depicted in the step 21 in the Fig. 18), (2) a second time point at 1.5 hours after ingestion of the brown algae fucoidan supplement (i.e., a time point at the end of the first period of time depicted in the step 24 in Fig. 18), (3) a third time point at 24 hours after ingestion of the brown algae fucoidan supplement (i.e., a time point at the end of the second period of time depicted the step 27 in Fig. 18), and (4) a fourth time point at 48 hours after ingestion of the brown algae fucoidan supplement (i.e., a time point at the end of the third period of time depicted the step 30 in Fig. 18).

Figs. 20I and 20J show the count of each type of stem cells (total 23 types of stem cells) per milliliter of peripheral blood of the Subject E5 at the three specific time points. For the Subject E5, the three specific time points include (1) a first time point before ingestion of the brown algae fucoidan supplement (i.e., the first specific time point depicted in the step 21 in the Fig. 18), (2) a second time point at 1.5 hours after ingestion of the brown algae fucoidan supplement (i.e., a time point at the end of the first period of time depicted in the step 24 in Fig. 18), and (3) a third time point at 24 hours after ingestion of the brown algae fucoidan supplement (i.e., a time point at the end of the second period of time depicted the step 27 in Fig. 18).

The method depicted in the steps 33-43 of Fig. 19 was performed on the Subject CP so as to obtain stem-cell data related to the Subject CP at four specific time points, in which the Subject CP did not take any brown algae fucoidan supplement. Figs. 20K and 20L show the count of each type of stem cells (total 23 types of stem cells) per milliliter of peripheral blood of the Subject CP at the four specific time points. For the Subject CP, the four specific time points include (1) a fifth time point of extracting a blood sample from the Subject CP (i.e., the fifth specific time point depicted the step 33 in Fig. 19), (2) a sixth time point at 1.5 hours after the fifth time point (i.e., the sixth specific time point at the end of the fourth period of time depicted in the step 35 in Fig. 19), (3) a seventh time point at 24 hours after the fifth time point (i.e., the seventh specific time point at the end of the fifth period of time depicted in the step 38 in Fig. 19), and (4) an eighth time point at 48 hours after the fifth time point (i.e., the eighth specific time point at the end of the sixth period of time depicted in the step 41 in Fig. 19).

Based on these stem-cell data shown in Figs. 20A-20L, an optimal harvesting time point can be determined to be 1.5 hours after stem cell expansion in vivo or after ingestion of the brown algae fucoidan supplement for some specific types of stem cells listed below. More than two-fold increases in the counts of some types of stem cells per milliliter of peripheral blood of the Subjects E1-E5 extracted at 1.5 hours after ingestion of the brown algae fucoidan supplement are listed below: SB-1 cells, SB-2 cells, BLSCs, SSEA4(+) pluripotent stem cells, CD13(+) multipotent stem cells, CD90(+) multipotent stem cells, CD105(+) multipotent stem cells, CD34(+) hematopoietic stem cells, CD150(+) hematopoietic stem cells, CD24(+) progenitor stem cells, SSEA1(+) progenitor stem cells, CD45(+) progenitor stem cells, CD33(+) progenitor stem cells, CD10(+) progenitor stem cells, and CXCR1(+) stem cells.

According to these stem-cell data shown in Figs. 20A-20L, taking the brown algae fucoidan supplement is possible to cause or induce at least 2-fold increases in the counts of some specific types of stem cells per milliliter of peripheral blood of a human or animal body at 1.5 hours after ingestion of the brown algae fucoidan supplement. Here "2-fold increase" means the ratio of the count of a certain type of stem cells per milliliter of peripheral blood of a subject (e.g., human or animal body) before the ingestion of the brown algae fucoidan supplement to the count of the certain type of stem cells per milliliter of peripheral blood of the subject at 1.5 hours after the ingestion of the brown algae fucoidan supplement equals 2. From Figs. 20A-20L, 1.5-fold increases, 3-fold increases, or 4-fold increases in the counts of some specific types of stem cells per milliliter of peripheral blood of one of the Subjects E1-E5 at 1.5 hours after ingestion of the brown algae fucoidan supplement may be found. Therefore, orally taking fucoidan, a major component of brown algae, may be an effective approach to increase some types of stem cells in peripheral blood of a human or animal body. For more elaboration, an action, such as orally taking 3 grams (i.e., greater than 2 grams) of fucoidan (from the above-mentioned brown algae fucoidan supplement) or one or more of the actions or stimuli (X), can increase, induce, or activate some types of stem cells in the peripheral blood of a human or animal body by at least (or greater than) 1.5-fold increase, at least (or greater than) 2-fold increase, at least (or greater than) 3-fold increase, or at least (or greater than) 4-fold increase.

In summary, this invention discloses multiple methods as below:

A method of harvesting (or obtaining) and storing stem cells from a first subject (for example, a human body or an animal body) includes: (1) said first subject taking or being subjected to an action (for example, taking 4.5 grams (or greater than 3.5 grams) of an Okinawa brown algae supplement containing 80% (or more than 70%) of a mozuku powder in weight, wherein said 4.5 grams of said Okinawa brown algae supplement comprises 3 grams of fucoidan (or greater/more than 2 grams, or more than 60% of fucoidan in weight)), wherein said action was previously evaluated effective in increasing the number of a type or selected types of stem cells (such as SB-1 cells and/or SB-2 cells) in vivo in said first subject; (2) after said first subject taking or being subject to said action, said first subject waiting for a (predetermined) time interval (such as between 15 minutes and 60 minutes, between 20 minutes and 100 minutes, between 30 minutes and 2 hours, between 0.5 hours and 3 hours, between 1 hour and 12 hours, between 12 hours and 36 hours, or between 36 hours and 50 hours); (3) after said first subject waiting for said (predetermined) time interval, taking a tissue sample (for example, the peripheral blood of said human body or said animal body) from said first subject; (4) harvesting, collecting or extracting stem cells of said type or said selected types from said tissue sample; and (5) storing said stem cells of said type or said selected types at a temperature lower than 0 degrees Celsius (for example lower than -30 degree Celsius, or lower than -80 degree Celsius) in a device (such as refrigerator) provided by an organization (e.g., cell bank) for a period of time (for example, longer than 1 month or longer than 1 year). In an example, after said storing said stem cells of said type or said selected types, said stem cells of said type or said selected types may be applied to, such as injected into, or spread over or in, said first subject. In another example, after said storing said stem cells of said type or said selected types, said stem cells of said type or said selected types may be applied to, such as injected into or spread over or in, a second subject (for example, another human body or another animal body).

A method of storing stem cells from a first subject (for example, a human body or an animal body) includes: (1) said first subject taking or being subjected to an action (for example, taking 4.5 grams (or greater than 3.5 grams) of an Okinawa brown algae supplement containing 80% (or more than 70%) of a mozuku powder in weight, wherein said 4.5 grams of said Okinawa brown algae supplement comprises 3 grams of fucoidan (or greater than 2 grams, or more than 60% of fucoidan in weight)), wherein said action was previously evaluated effective in increasing the number of a type or selected types of stem cells (such as SB-1 cells and/or SB-2 cells) in vivo in said first subject; (2) after said first subject taking or being subject to said action, said first subject waiting for a (predetermined) time interval (such as between 15 minutes and 60 minutes, between 20 minutes and 100 minutes, between 30 minutes and 2 hours, between 0.5 hours and 3 hours, between 1 hour and 12 hours, between 12 hours and 36 hours, or between 36 hours and 50 hours); (3) after said first subject waiting for said (predetermined) time interval, taking a tissue sample (for example, the peripheral blood of said human body or said animal body) from said first subject, wherein said tissue sample comprises stem cells of said type or said selected types; and (4) storing said tissue sample comprising said stem cells of said type or said selected types at a temperature lower than 0 degrees Celsius (for example lower than -30 degree Celsius or lower than -80 degree Celsius) in a device (such as refrigerator) provided by an organization (e.g., cell bank) for a period of time (for example, longer than 1 month or longer than 1 year). In an example, after said storing said tissue sample comprising said stem cells of said type or said selected types, said tissue sample comprising said stem cells of said type or said selected types may be applied to, such as injected into, or spread over or in, said first subject. In another example, after said storing said tissue sample comprising said stem cells of said type or said selected types, said tissue sample comprising said stem cells of said type or said selected types may be applied to, such as injected into or spread over or in, a second subject (for example, another human body or another animal body).

A method of harvesting stem cells from a first subject (for example, a human body or an animal body) includes: (1) said first subject taking or being subjected to an action (for example, taking 4.5 grams (or greater than 3.5 grams) of an Okinawa brown algae supplement containing 80% (or more than 70%) of a mozuku powder in weight, wherein said 4.5 grams of said Okinawa brown algae supplement comprises 3 grams of fucoidan (or greater than 2 grams, or more than 60% of fucoidan in weight)), wherein said action was previously evaluated effective in increasing the number of a type or selected types of stem cells (such as SB-1 cells and/or SB-2 cells) in vivo in said first subject; (2) after said first subject taking or being subject to said action, said first subject waiting for a (predetermined) time interval (such as between 15 minutes and 60 minutes, between 20 minutes and 100 minutes, between 30 minutes and 2 hours, between 0.5 hours and 3 hours, between 1 hour and 12 hours, between 12 hours and 36 hours, or between 36 hours and 50 hours); (3) after said first subject waiting for said (predetermined) time interval, taking a tissue sample (for example, the peripheral blood of said human body or said animal body) from said first subject; and (4) harvesting or extracting stem cells of said type or said selected types from said tissue sample. In an example, after said harvesting or extracting said stem cells of said type or said selected types from said tissue sample (without or skipping the above-mentioned storage at very low temperature in a device (such as refrigerator) for a period of time such as longer than 1 month or longer than 1 year), said stem cells of said type or said selected types may be applied to, such as injected into, or spread over or in, said first subject. In another example, after said extracting said stem cells of said type or said selected types from said tissue sample (without or skipping the above-mentioned storage at very low temperature in in said device for said period of time), said stem cells of said type or said selected types may be applied to, such as injected into or spread over or in, a second subject (for example, another human body or another animal body).

A method of storing stem cells from a first subject (for example, a human body or an animal body) includes: (1) said first subject taking or being subjected to an action (for example, taking 4.5 grams (or greater than 3.5 grams) of an Okinawa brown algae supplement containing 80% (or more than 70%) of a mozuku powder in weight, wherein said 4.5 grams of said Okinawa brown algae supplement comprises 3 grams of fucoidan (or greater than 2 grams, or more than 60% of fucoidan in weight)), wherein said action was previously evaluated effective in increasing the number of a type or selected types of stem cells (such as SB-1 cells and/or SB-2 cells) in vivo in said first subject; (2) after said first subject taking or being subject to said action, said first subject waiting for a (predetermined) time interval (such as between 15 minutes and 60 minutes, between 20 minutes and 100 minutes, between 30 minutes and 2 hours, between 0.5 hours and 3 hours, between 1 hour and 12 hours, between 12 hours and 36 hours, or between 36 hours and 50 hours); and (3) after said first subject waiting for said (predetermined) time interval, taking a tissue sample (for example, the peripheral blood of said human body or said animal body) from said first subject, wherein said tissue sample comprises stem cells of said type or said selected types. In an example, after said taking said tissue sample comprising said stem cells of said type or said selected types from said first subject (without or skipping the above-mentioned storage at very low temperature in a device (such as refrigerator) for a period of time such as longer than 1 month or longer than 1 year), said tissue sample comprising said stem cells of said type or said selected types may be applied to, such as injected into, or spread over or in, said first subject. In another example, after said taking said tissue sample comprising said stem cells of said type or said selected types from said first subject (without or skipping the above-mentioned storage at very low temperature in said device for said period of time), said tissue sample comprising said stem cells of said type or said selected types may be applied to, such as injected into, or spread over or in, a second subject (for example, another human body or another animal body).

A method of storing and harvesting stem cells from a first subject (for example, a human body or an animal body) includes: (1) said first subject taking or being subjected to an action (for example, taking 4.5 grams (or greater than 3.5 grams) of an Okinawa brown algae supplement containing 80% (or more than 70%) of a mozuku powder in weight, wherein said 4.5 grams of said Okinawa brown algae supplement comprises 3 grams of fucoidan (or greater than 2 grams, or more than 60% of fucoidan in weight)), wherein said action was previously evaluated effective in increasing the number of a type or selected types of stem cells (such as SB-1 cells and/or SB-2 cells) in vivo in said first subject; (2) after said first subject taking or being subject to said action, said first subject waiting for a (predetermined) time interval (such as between 15 minutes and 60 minutes, between 20 minutes and 100 minutes, between 30 minutes and 2 hours, between 0.5 hours and 3 hours, between 1 hour and 12 hours, between 12 hours and 36 hours, or between 36 hours and 50 hours); (3) after said first subject waiting for said (predetermined) time interval, taking a tissue sample (for example, the peripheral blood of said human body or said animal body) from said first subject, wherein said tissue sample comprises stem cells of said type or said selected types; (4) storing said tissue sample comprising said stem cells of said type or said selected types at a temperature lower than 0 degrees Celsius (for example, lower than -30 degree Celsius or lower than -80 degree Celsius) a device (such as refrigerator) provided by an organization (e.g., cell bank) for a period of time (for example, longer than 1 month or longer than 1 year); and (5) after said storing said tissue sample comprising said stem cells of said type or said selected types, harvesting or extracting said stem cells of said type or said selected types from said tissue sample. In an example, after said harvesting or extracting said stem cells of said type or said selected types from said tissue sample, said stem cells of said type or said selected types may be applied to, such as injected into, or spread over or in, said first subject. In another example, after said harvesting or extracting said stem cells of said type or said selected types from said tissue sample, said stem cells of said type or said selected types may be applied to, such as injected into or spread over or in, a second subject (for example, another human body or another animal body).

A method of discovering a new type of stem cells, which have not been found before, includes: (1) a subject (for example, a human body or an animal body) taking or being subjected to an action (for example, taking 4.5 grams (or greater than 3.5 grams) of an Okinawa brown algae supplement containing 80% (or more than 70%) of a mozuku powder in weight, wherein said 4.5 grams of said Okinawa brown algae supplement comprises 3 grams of fucoidan (or greater than 2 grams, or more than 60% of fucoidan in weight)), wherein said action may be previously evaluated effective in increasing the number of a certain type or types of stem cells in vivo; (2) after said subject taking or being subjected to said action, said subject waiting for a (predetermined) time interval (such as between 15 minutes and 60 minutes, between 20 minutes and 100 minutes, between 30 minutes and 2 hours, between 0.5 hours and 3 hours, between 1 hour and 12 hours, between 12 hours and 36 hours, or between 36 hours and 50 hours); (3) after said subject waiting for said (predetermined) time interval, taking a tissue sample (for example the peripheral blood) from said subject; and (4) processing said tissue sample using an analysis method for detecting a new type of stem cells, wherein said analysis method comprises using procedures to detect whether cells from said tissue sample have cell nuclei and can express and/or lack expression of one or more selected cell (surface) markers, and wherein said analysis method comprises using a flow cytometer.

A method of evaluating the effectiveness of an action (for example, taking 4.5 grams (or greater than 3.5 grams) of an Okinawa brown algae supplement containing 80% (or more than 70%) of a mozuku powder in weight, wherein said 4.5 grams of said Okinawa brown algae supplement includes 3 grams of fucoidan (or greater than 2 grams, or more than 60% of fucoidan in weight)) for curing a subject (for example, a human body or an animal body) having a disease such as cancer, parkinsonism or Alzheimer's disease, includes: (1) obtaining a first tissue sample such as peripheral-blood sample from said subject; (2) obtaining first stem-cell data related to information of a type or selected types of stem cells (such as SB-1 cells and/or SB-2 cells) from said first tissue sample by an analyzing or measuring method (e.g., the measurement MT) or an apparatus (e.g., the integrated system, apparatus, device or tool (AD), or the single piece of system, device, tool or apparatus (TD)); (3) after said obtaining said first tissue sample or said first stem-cell data, said subject taking or being subjected to said action; (4) after said subject taking or being subjected to said action, said subject waiting for a (predetermined) time interval (such as between 15 minutes and 60 minutes, between 20 minutes and 100 minutes, between 30 minutes and 2 hours, between 0.5 hours and 3 hours, between 1 hour and 12 hours, between 12 hours and 36 hours, or between 36 hours and 50 hours); (5) after said subject waiting for said (predetermined) time interval, obtaining a second tissue sample such as peripheral-blood sample from said subject; (6) obtaining second stem-cell data related to said type or said selected types of stem cells from said second tissue sample by said analyzing or measuring method or said apparatus; and (7) performing an analysis based on said first stem-cell data and said second stem-cell data (e.g., including a comparison between said second stem-cell data and said first stem-cell data. Said type or said selected types of stem cells may be referred to the paragraphs in "Description of stem cells". Said first stem-cell data may include the count of said type or said selected types of stem cells per unit volume (e.g., milliliter) of said first tissue sample (i.e., the count per unit volume of said type or said selected types of stem cells in said first tissue sample), and said second stem-cell data may include the count of said type or said selected types of stem cells per unit volume (e.g., milliliter) of said second tissue sample (i.e., the count per unit volume of said type or said selected types of stem cells in said second tissue sample). Said first stem-cell data may further include the percentage, of the count of said type or said selected types of stem cells to the count in a region of particles having sizes greater than or equal to a threshold size, for said first tissue sample, and said second stem-cell data may further include the percentage, of the count of said type or said selected types of stem cells to the count in a region of particles having sizes greater than or equal to said threshold size, for said second tissue sample. Said threshold size may be 0.5 micrometers, 0.8 micrometers, 1 micrometer, 1.5 micrometers, 2 micrometers, or 3 micrometers.

A method of evaluating the effectiveness of an action (for example, taking 4.5 grams (or greater than 3.5 grams) of an Okinawa brown algae supplement containing 80% (or more than 70%) of a mozuku powder in weight, wherein said 4.5 grams of said Okinawa brown algae supplement includes 3 grams of fucoidan (or greater than 2 grams, or more than 60% of fucoidan in weight)) for curing a subject (such as a human body or an animal body) having a disease such as cancer, parkinsonism or Alzheimer's disease, includes: (1) obtaining a first tissue sample such as peripheral-blood sample from said subject; (2) obtaining first stem-cell data related to a type or selected types of stem cells (such as SB-1 cells and/or SB-2 cells) from said first tissue sample by an analyzing or measuring method (such as the measurement MT) or an apparatus (such as the integrated system, apparatus, device or tool (AD), or the single piece of system, device, tool or apparatus (TD)); (3) after said obtaining said first tissue sample or said first stem-cell data, said subject taking or being subjected to said action; (4) after said subject taking or being subjected to said action, said subject waiting for a first (predetermined) time interval (such as between 15 minutes and 60 minutes, between 20 minutes and 100 minutes, between 30 minutes and 2 hours, between 0.5 hours and 3 hours, between 1 hour and 12 hours, between 12 hours and 36 hours, or between 36 hours and 50 hours); (5) after said subject waiting for said first (predetermined) time interval, obtaining a second tissue sample such as peripheral-blood sample from said subject; (6) obtaining second stem-cell data related to said type or said selected types of stem cells from said second tissue sample by said analyzing or measuring method or said apparatus; (7) performing a first analysis based on said second stem-cell data and said first stem-cell data (e.g., including a comparison between said first stem-cell data and said second stem-cell data); (8) after said obtaining said second tissue sample or said second stem-cell data, said subject taking or being subjected to said action again; (9) after said subject taking or being subjected to said action again, said subject waiting for a second (predetermined) time interval (such as between 15 minutes and 60 minutes, between 20 minutes and 100 minutes, between 30 minutes and 2 hours, between 0.5 hours and 3 hours, between 1 hour and 12 hours, between 12 hours and 36 hours, or between 36 hours and 50 hours); (10) after said subject waiting for said second (predetermined) time interval, obtaining a third tissue sample such as peripheral-blood sample from said subject; (11) obtaining third stem-cell data related to said type or said selected types of stem cells from said third tissue sample by said analyzing or measuring method or said apparatus; and (12) performing a second analysis based on said third stem-cell data and said first or second stem-cell data (e.g., including a comparison between said third stem-cell data and said first or second stem-cell data). Said type or said selected types of stem cells may be referred to the paragraphs in "Description of stem cells". Said first stem-cell data may include the count of said type or said selected types of stem cells per unit volume (e.g., milliliter) of said first tissue sample (i.e., the count per unit volume of said type or said selected types of stem cells in said first tissue sample), said second stem-cell data may include the count of said type or said selected types of stem cells per unit volume of said second tissue sample (i.e., the count per unit volume of said type or said selected types of stem cells in said second tissue sample), and said third stem-cell data may include the count of said type or said selected types of stem cells per unit volume of said third tissue sample (i.e., the count per unit volume of said type or said selected types of stem cells in said third tissue sample). Said first stem-cell data may further include the percentage, of the count of said type or said selected types of stem cells to the count in a region of particles having sizes greater than or equal to a threshold size, for said first tissue sample, said second stem-cell data may further include the percentage, of the count of said type or said selected types of stem cells to the count in said region of particles having sizes greater than or equal to said threshold size, for said second tissue sample, and said third stem-cell data may further include the percentage, of the count of said type or said selected types of stem cells to the count in said region of particles having sizes greater than or equal to said threshold size, for said third tissue sample. Said threshold size may be 0.5 micrometers, 0.8 micrometers, 1 micrometer, 1.5 micrometers, 2 micrometers, or 3 micrometers.

A method of evaluating or testing the effect or effectiveness of one or more of the above-mentioned actions or stimuli (X), such as taking or ingesting 4.5 grams (or greater than 3.5 grams) of an Okinawa brown algae supplement containing 80% (or more than 70%) of a mozuku powder in weight, wherein said 4.5 grams of said Okinawa brown algae supplement includes 3 grams of fucoidan (or greater than 2 grams, or more than 60% of fucoidan in weight), includes: (1) obtaining a first tissue sample such as peripheral-blood sample from a subject (such as a human body or an animal body); (2) obtaining a first stem-cell data relating to information of a type or selected types of stem cells (such as SB-1 cells and/or SB-2 cells) from said first tissue sample by an analyzing or measuring method (such as the measurement MT) or an apparatus (such as the integrated system, apparatus, device or tool (AD), or the single piece of system, device, tool or apparatus (TD)); (3) after said obtaining said first tissue sample or said first stem-cell data, said subject taking or being subjected to said one or more of the above-mentioned actions or stimuli (X); (4) after said subject taking or being subjected to said one or more of the above-mentioned actions or stimuli (X), said subject waiting for a

(predetermined) time interval (such as between 15 minutes and 60 minutes, between 20 minutes and 100 minutes, between 30 minutes and 2 hours, between 0.5 hours and 3 hours, between 1 hour and 12 hours, between 12 hours and 36 hours, or between 36 hours and 50 hours); (5) after said subject waiting for said (predetermined) time interval, obtaining a second tissue sample such as peripheral-blood sample from said subject; (6) obtaining a second stem-cell data relating to information of said type or said selected types of stem cells from said second tissue sample by said analyzing or measuring method or said apparatus; and (7) performing an analysis based on said first stem-cell data and said second stem-cell data (e.g., including a comparison between said first stem-cell data and said second stem-cell data. Said type or said selected types of stem cells may be referred to the paragraphs in "Description of stem cells". Said first stem-cell data may include the count of said type or said selected types of stem cells per unit volume (e.g., milliliter) of said first tissue sample (i.e., the count per unit volume of said type or said selected types of stem cells in said first tissue sample), and said second stem-cell data may include the count of said type or said selected types of stem cells per unit volume of said second tissue sample (i.e., the count per unit volume of said type or said selected types of stem cells in said second tissue sample). Said first stem-cell data may further include the percentage, of the count of said type or said selected types of stem cells to the count in a region of particles having sizes greater than or equal to a threshold size, for said first tissue sample, and said second stem-cell data may further include the percentage, of the count of said type or said selected types of stem cells to the count in said region of particles having sizes greater than or equal to said threshold size, for said second tissue sample. Said threshold size may be 0.5 micrometers, 0.8 micrometers, 1 micrometer, 1.5 micrometers, 2 micrometers, or 3 micrometers.

A method of monitoring the increasing number of stem cells of a selected type in the peripheral blood of a subject (such as the above-mentioned human body or the above-mentioned non-human body) after taking or being subjected to one or more of the above-mentioned actions or stimuli (X) to cultivate in vivo said selected type of stem cells (such as SB-1 cells or SB-2 cells), includes: (1) obtaining a first tissue sample from said subject; (2) obtaining a first data relating to the count per unit volume (e.g., milliliter) of said selected typed of stem cells from said first tissue sample by an analyzing or measuring method (such as the above-mentioned measurement MT) or an apparatus (such as the above-mentioned integrated system, apparatus, device or tool (AD) or the above-mentioned single piece of system, device, tool or apparatus (TD)); (3) after said obtaining said first tissue sample, said subject taking or being subjected to said one or more of the above-mentioned actions or stimuli (X); (4) after said subject taking or being subjected to said one or more of the above-mentioned actions or stimuli (X), said subject waiting for a first (predetermined) time interval (such as between 30 minutes and 3 hours or between 45 minutes and 12 hours); (5) after said subject waiting for said first (predetermined) time interval, obtaining a second tissue sample from said subject; (6) obtaining a second data relating to the count per unit volume (e.g., milliliter) of said selected typed of stem cells from said second tissue sample by said analyzing or measuring method or said apparatus; (7) performing a first analysis based on said first data and said second data (e.g., including a comparison between said first data and said second data so as to obtain a first change of the count of said selected type of stem cells per unit volume (e.g., milliliter); (8) after said subject taking or being subjected to said one or more of the above-mentioned actions or stimuli (X), said subject waiting for a second (predetermined) time interval (such as between 12 hours and 36 hours or between 36 hours and 60 hours), wherein said second (predetermined) time interval is not equal to said first (predetermined) time interval; (9) after said subject waiting for said second (predetermined) time interval, obtaining a third tissue sample from said subject; (10) obtaining a third data relating to the count per unit volume (e.g., milliliter) of said selected typed of stem cells from said third tissue sample by said analyzing or measuring method or said apparatus; and (11) performing a second analysis based on said first data and said third data (e.g., including a comparison between said first data and said third data) so as to obtain a second change of the count of said selected type of stem cells per unit volume (e.g., milliliter). Said first, second and third tissue samples may be obtained from the peripheral blood of said subject. Said selected typed of stem cells may be any one of the types of stem cells depicted in the paragraphs in "Description of stem cells" or may be stem cells characterized by one or more cell (surface) makers, e.g., including CD349(+), Lgr5(+) or CD66e(+), as mentioned in the paragraphs in "Description of stem cells".

A method of obtaining a stem-cell data (such as a stem cell count per unit volume) for a tissue sample (such as a peripheral blood) related to a subject (such as a human body or an animal body) includes: (1) obtaining said tissue sample (with a volume of 10 ml, for example) from said subject; (2) processing said tissue sample to obtain or prepare a test sample (with a volume of 3 ml, for example); (3) obtaining a first data (such as the count per unit volume (e.g., milliliter) of a first specific group of particles, including cells) from a first portion of said test sample by a first method including counting particles in said first portion using a hemocytometer (with a microscope); (4) obtaining a second data (such as a percentage of the count of a specific type of stem cells to that of a second specific group of particles, including cells) and a third data (such as a percentage of the count of said second specific group of particles to that of a third specific group of particles, including cells) from a second portion of said test sample by a second method including counting particles in said second portion using a flow cytometer, wherein said first portion is supposed to contain substantially the same content as said second portion in the case that said test sample is homogeneous and contains even content, wherein said first and third groups of particles have substantially the same characteristic and have sizes greater than or equal to a given threshold size (such as 0.5 micrometers, 0.8 micrometers, 1 micrometer, 1.5 micrometers, 2 micrometers, or 3 micrometers); (5) performing a first operation or calculation of said second and third data, such as multiplying said second data by said third data, to obtain a fourth data (such as a percentage of the count of said specific type of stem cells to that of said third specific group of particles) related to said test sample; (6) performing a second operation or calculation of said first and fourth data, such as multiplying said first data by said fourth data, to obtain a fifth data (such as the count per unit volume (e.g., milliliter) of said specific type of stem cells in said test sample) related to said test sample; and (7) performing a third operation or calculation of said fifth data and a volume change (or sample preparation) factor, such as multiplying said fifth data by said volume change (or sample preparation) factor, to obtain a sixth data (such as the count per unit volume (e.g., milliliter) of said specific type of stem cells in said tissue sample) related to said tissue sample, e.g., said peripheral blood. The method may further include performing a fourth operation or calculation of the volume of said test sample and the volume of said tissue sample, such as dividing the volume of said test sample by the volume of said tissue sample, to obtain said volume change (or sample preparation) factor.

Said stem-cell data may include the count of said specific type of stem cells per unit volume (e.g., milliliter) of peripheral blood of said subject. Said third specific group of particles include said second specific group of particles and other groups of particles and/or cells including micro particles, granulocytes, red blood cells, blood platelets, and white blood cells containing lymphocytes. Said second specific group of particles include said specific type of stem cells and may substantially exclude micro particles, granulocytes, red blood cells, blood platelets, and white blood cells containing lymphocytes. Said given threshold size (of said first and third specific groups of particles) may be 0.5 micrometers, 0.8 micrometers, 1 micrometer, 1.5 micrometers, 2 micrometers, or 3 micrometers. The sizes of particles in said second specific group may be greater than or substantially equal to 1 micrometer, 1.1 micrometers, 1.5 micrometers, 2 micrometers, 2.1 micrometers, or 3 micrometers. All of said first, second and third specific groups of particles may be or may include groups of cells. Said specific type of stem cells may be one type of pluripotent stem cells as mentioned in the paragraphs in "Description of stem cells", one type of multipotent stem cells as mentioned in the paragraphs in "Description of stem cells", or one type of progenitor stem cells as mentioned in the paragraphs in "Description of stem cells". Said tissue sample may be a blood, peripheral blood or other tissues. Said subject may be a human or an animal. Said processing said tissue sample may include mixing an erythrocyte aggregation agent with said tissue sample and after said mixing said erythrocyte aggregation agent with said tissue sample, re-suspending pellets derived from said tissue sample in a solution or medium. Said solution or medium may be a salt solution without calcium and magnesium or may be bovine serum albumin in a phosphate-buffered saline. Said volume change (or sample preparation) factor can be calculated or obtained based on the preparation of said test sample from said tissue sample. For example, 10 ml of said tissue sample is processed, resulting in 3 ml of said test sample, whereby said volume change (or sample preparation) factor is 3/10, i.e., the ratio of the volume of said test sample to the volume of said tissue sample).

In summary, the hemocytometer (with the microscope) is used to obtain the count of particles (including cells) per unit volume (e.g., milliliter) of the test sample processed from the tissue sample (e.g., peripheral blood) with sizes greater than or equal to the given threshold size; while the flow cytometer is used to obtain the percentage, also for the test sample, of the specific type of stem cells in the particles (including cells) with sizes greater than or equal to the given threshold size. By multiplying the above obtained count of the particles per unit volume of the test sample by the above obtained percentage of the specific type of stem cells in the particles, the count of the specific type of stem cells per unit volume (e.g., milliliter) of the test sample can be obtained. By multiplying the count of the specific type of stem cells per unit volume of the test sample by the volume change (or sample preparation) factor, the count of the specific type of stem cells per unit volume (e.g., milliliter) of the tissue sample (i.e., the peripheral blood) can be obtained. By combining the cell count data from the hemocytometer and the percentage of the specific type of stem cells measured from the flow cytometer, a more accurate count of the specific type of stem cells in the peripheral blood can be obtained.

This invention discloses an integrated system, device, tool or apparatus to obtain a stem-cell data (such as a stem cell count per unit volume) for a tissue sample (such as a peripheral blood) related to a subject (such as a human body), including: (1) a first device, tool or apparatus for obtaining said tissue sample (with a volume of 10 mL, for example) from said subject; (2) a second device, tool or apparatus (including, e.g., a centrifuge) for processing said tissue sample to obtain or prepare a test sample (with a volume of 3 mL, for example); (3) a third device, tool or apparatus (including, e.g., a hemocytometer, a microscope, and a processor or computer with a software) for obtaining a first data (such as the count per unit volume (e.g., milliliter) of a first specific group of particles) from a first portion of said test sample; (4) a fourth device, tool or apparatus (including, e.g., a flow cytometer and a processor or computer) for obtaining a second data (such as a percentage of the count of a specific type of stem cells to that of a second specific group of particles) and a third data (such as a percentage of the count of said second specific group of particles to that of a third specific group of particles) from a second portion of said test sample, wherein said first portion is supposed to contain substantially the same content as said second portion in the case that said test sample is homogeneous and contains even content, wherein said first and third groups of particles have substantially the same characteristic and have sizes greater than or equal to a given threshold size; and (5) a fifth device, tool or apparatus (such as a computer or processor) for (a) performing a first operation or calculation of said second and third data, such as multiplying said second data by said third data, to obtain a fourth data (such as a percentage of the count of said specific type of stem cells to that of said third specific group of particles) related to said test sample, (b) performing a second operation or calculation of said first and fourth data, such as multiplying said first data by said fourth data, to obtain a fifth data (such as the count per unit volume (e.g., milliliter) of said specific type of stem cells in said test sample) related to said test sample, and (c) performing a third operation or calculation of said fifth data and a volume change (or sample preparation) factor, such as multiplying said fifth data by said volume change (or sample preparation) factor, to obtain a sixth data (such as the count per unit volume (e.g., milliliter) of said specific type of stem cells in said tissue sample) related to said tissue sample. Said fifth device, tool or apparatus may be further configured for performing a fourth operation or calculation of the volume of said test sample and the volume of said tissue sample, such as dividing the volume of said test sample by the volume of said tissue sample, to obtain said volume change (or sample preparation) factor. Two or more than two of said first, second, third, fourth and fifth devices, tools or apparatus may be combined into an integrated device, tool or apparatus or a single piece of device, tool or apparatus to perform the functions performed by the two or more than two of said first, second, third, fourth and fifth devices, tools or apparatus. For example, said first and second devices, tools or apparatus may be combined into a first integrated device or a first single piece of device, tool or apparatus to perform the functions performed by said first and second devices, tools or apparatus, and said third, fourth and fifth devices, tools or apparatus may be combined into a second integrated device or a second single piece of device, tool or apparatus to perform the functions performed by said third, fourth and fifth devices, tools or apparatus. Alternatively, said second, third, fourth and fifth devices, tools or apparatus may be combined into an integrated device or a single piece of device, tool or apparatus to perform the functions performed by said second, third, fourth and fifth devices, tools or apparatus. Alternatively, said second, third and fourth devices, tools or apparatus may be combined into an integrated device or a single piece of device, tool or apparatus to perform the functions performed by said second, third and fourth devices, tools or apparatus. Alternatively, said third and fourth devices, tools or apparatus may be combined into an integrated device or a single piece of device, tool or apparatus to perform the functions performed by said third and fourth devices, tools or apparatus. Alternatively, said fourth and fifth devices, tools or apparatus may be combined into an integrated device or a single piece of device, tool or apparatus to perform the functions performed by said fourth and fifth devices, tools or apparatus. Said stem-cell data may include the count of said specific type of stem cells per unit volume (e.g., milliliter) of peripheral blood of said subject. Said third specific group of particles include said second specific group of particles and other groups of particles and/or cells including micro particles, granulocytes, red blood cells, blood platelets, and white blood cells containing lymphocytes. Said second specific group of particles include said specific type of stem cells and may substantially exclude micro particles, granulocytes, red blood cells, blood platelets, and white blood cells containing lymphocytes. Said given threshold size (of said first and third specific groups of particles) may be 0.5 micrometers, 0.8 micrometers, 1 micrometer, 1.5 micrometers, 2 micrometers, or 3 micrometers. The sizes of particles in said second specific group may be greater than or substantially equal to 1 micrometer, 1.1 micrometers, 1.5 micrometers, 2 micrometers, 2.1 micrometers, or 3 micrometers. All of said first, second and third specific groups of particles may be or may include groups of cells. Said specific type of stem cells may be one type of pluripotent stem cells as mentioned in the paragraphs in "Description of stem cells", one type of multipotent stem cells as mentioned in the paragraphs in "Description of stem cells", or one type of progenitor stem cells as mentioned in the paragraphs in "Description of stem cells". Said tissue sample may be a peripheral blood sample. Said subject may be a human or an animal. Said processing said tissue sample may include mixing an erythrocyte aggregation agent with said tissue sample and after said mixing said erythrocyte aggregation agent with said tissue sample, re-suspending pellets derived from said tissue sample in a solution or medium. Said solution or medium may be a salt solution without calcium and magnesium or may be bovine serum albumin in a phosphate-buffered saline. Said volume change (or sample preparation) factor can be calculated or obtained based on the preparation of said test sample from said tissue sample. For example, 10 mL of said tissue sample is processed, resulting in 3 mL of said test sample, whereby said volume change (or sample preparation) factor is 3/10, i.e., the ratio of the volume of said test sample to the volume of said tissue sample.

In summary, the third device, tool or apparatus is used to obtain the count of particles (including cells) per unit volume (e.g., milliliter) of the test sample processed from the tissue sample (e.g., peripheral blood) with sizes greater than or equal to the given threshold size; while the fourth and fifth devices, tools or apparatus are used to obtain the percentage, also for the test sample, of the specific type of stem cells in the particles (including cells) with sizes greater than or equal to the given threshold size. By multiplying the above obtained count of the particles per unit volume of the test sample by the above obtained percentage of the specific type of stem cells in the particles, the count of the specific type of stem cells per unit volume (e.g., milliliter) of the test sample can be obtained. By multiplying the count of the specific type of stem cells per unit volume of the test sample by the volume change (or sample preparation) factor, the count of the specific type of stem cells per unit volume (e.g., milliliter) of the tissue sample (i.e., the peripheral blood) can be obtained. By combining the cell count data from the third device, tool or apparatus and the percentage of the specific type of stem cells from the fourth and fifth devices, tools or apparatus, a more accurate count of the specific type of stem cells in the peripheral blood can be obtained.

This invention further discloses a single piece of system, device, tool or apparatus providing or performing any combination of the two or more following functions: (1) a first function for obtaining a tissue sample (with a volume of 10 mL, for example) from a subject; (2) a second function for processing said tissue sample to obtain or prepare a test sample (with a volume of 3 mL, for example); (3) a third function for obtaining a first data (such as the count per unit volume (e.g., milliliter) of a first specific group of particles) from a first portion of said test sample; (4) a fourth function for obtaining a second data (such as a percentage of the count of a specific type of stem cells to that of a second specific group of particles) and a third data (such as a percentage of the count of said second specific group of particles to that of a third specific group of particles) from a second portion of said test sample, wherein said first portion is supposed to contain substantially the same content as said second portion in the case that said test sample is homogeneous and contains even content, wherein said first and third groups of particles have substantially the same characteristic and have sizes greater than or equal to a given threshold size; (5) a fifth function for performing a first operation or calculation of said second and third data, such as multiplying said second data by said third data, to obtain a fourth data (such as a percentage of the count of said specific type of stem cells to that of said third specific group of particles) related to said test sample; (6) a sixth function for performing a second operation or calculation of said first and fourth data, such as multiplying said first data by said fourth data, to obtain a fifth data (such as the count per unit volume (e.g., milliliter) of said specific type of stem cells in said test sample) related to said test sample; (7) a seventh function for performing a third operation or calculation of the volume of said test sample and the volume of said tissue sample, such as dividing the volume of said test sample by the volume of said tissue sample, to obtain a volume change (or sample preparation) factor; and (8) an eighth function for performing a fourth operation or calculation of said fifth data and said volume change (or sample preparation) factor, such as multiplying said fifth data by said volume change (or sample preparation) factor, to obtain a sixth data (such as the count per unit volume (e.g., milliliter) of said specific type of stem cells in said tissue sample) related to said tissue sample. The count per unit volume of said specific type of stem cells in said tissue sample may be the count of said specific type of stem cells per unit volume of peripheral blood of said subject. Said third specific group of particles include said second specific group of particles and other groups of particles and/or cells including micro particles, granulocytes, red blood cells, blood platelets, and white blood cells containing lymphocytes. Said second specific group of particles include said specific type of stem cells and may substantially exclude micro particles, granulocytes, red blood cells, blood platelets, and white blood cells containing lymphocytes. Said given threshold size (of said first and third specific groups of particles) may be 0.5 micrometers, 0.8 micrometers, 1 micrometer, 1.5 micrometers, 2 micrometers, or 3 micrometers. The sizes of particles in said second specific group may be greater than or substantially equal to 1 micrometer, 1.1 micrometers, 1.5 micrometers, 2 micrometers, 2.1 micrometers, or 3 micrometers. All of said first, second and third specific groups of particles may be or may include groups of cells. Said specific type of stem cells may be one type of pluripotent stem cells as mentioned in the paragraphs in "Description of stem cells", one type of multipotent stem cells as mentioned in the paragraphs in "Description of stem cells", or one type of progenitor stem cells as mentioned in the paragraphs in "Description of stem cells". Said tissue sample may be a peripheral blood sample. Said subject may be a human or an animal. Said processing said tissue sample may include mixing an erythrocyte aggregation agent with said tissue sample and after said mixing said erythrocyte aggregation agent with said tissue sample, re-suspending pellets derived from said tissue sample in a solution or medium. Said solution or medium may be a salt solution without calcium and magnesium or may be bovine serum albumin in a phosphate-buffered saline. Said volume change (or sample preparation) factor can be calculated or obtained based on the preparation of said test sample from said tissue sample. For example, 10 mL of said tissue sample is processed, resulting in 3 mL of said test sample, whereby said volume change (or sample preparation) factor is 3/10, i.e., the ratio of the volume of said test sample to the volume of said tissue sample.

In one example, the single piece of system, device, tool or apparatus provides or performs the third and fourth functions. In another example, the single piece of system, device, tool or apparatus provides or performs the second, third and fourth functions. In another example, the single piece of system, device, tool or apparatus provides or performs the third, fourth, fifth, sixth, and eighth functions. In another example, the single piece of system, device, tool or apparatus provides or performs the third through eighth functions. In another example, the single piece of system, device, tool or apparatus provides or performs the first through eighth functions.

In summary, the third function provided by the single piece of system, device, tool or apparatus is used to obtain the count of particles (including cells) per unit volume (e.g., milliliter) of the test sample processed from the tissue sample (e.g., peripheral blood) with sizes greater than or equal to the given threshold size; while the fourth and fifth functions provided by the single piece of system, device, tool or apparatus are used to obtain the percentage, also for the test sample, of the specific type of stem cells in the particles (including cells) with sizes greater than or equal to the given threshold size. By multiplying the above obtained count of the particles per unit volume of the test sample by the above obtained percentage of the specific type of stem cells in the particles, the count of the specific type of stem cells per unit volume (e.g., milliliter) of the test sample can be obtained. By multiplying the count of the specific type of stem cells per unit volume of the test sample by the volume change (or sample preparation) factor, the count of the specific type of stem cells per unit volume (e.g., milliliter) of the tissue sample (i.e., the peripheral blood) can be obtained. By combining the cell count data from the third function performed by the single piece of system, device, tool or apparatus and the percentage of the specific type of stem cells from the fourth and fifth functions performed by the single piece of system, device, tool or apparatus, a more accurate count of the specific type of stem cells in the peripheral blood can be obtained.

The components, steps, features, benefits and advantages that have been discussed are merely illustrative. None of them, nor the discussions relating to them, are intended to limit the scope of protection in any way. Numerous other embodiments are also contemplated. These include embodiments that have fewer, additional, and/or different components, steps, features, benefits and advantages. These also include embodiments in which the components and/or steps are arranged and/or ordered differently.

Unless otherwise stated, all measurements, values, ratings, positions, magnitudes, sizes, and other specifications that are set forth in this specification, including in the claims that follow, are approximate, not exact. They are intended to have a reasonable range that is consistent with the functions to which they relate and with what is customary in the art to which they pertain. Furthermore, unless stated otherwise, the numerical ranges provided are intended to be inclusive of the stated lower and upper values. Moreover, unless stated otherwise, all material selections and numerical values are representative of preferred embodiments and other ranges and/or materials may be used.

The scope of protection is limited solely by the claims, and such scope is intended and should be interpreted to be as broad as is consistent with the ordinary meaning of the language that is used in the claims when interpreted in light of this specification and the prosecution history that follows, and to encompass all structural and functional equivalents thereof.

## Claims

1. A method of obtaining a stem-cell data, comprising:
obtaining a tissue sample from a subject;
processing said tissue sample into a test sample;
counting a first group of particles in a first portion of said test sample so as to obtain a first data;
counting a type of stem cells in a second group of particles in a second portion of said test sample so as to obtain a second data; and
obtaining a third data based on said first and second data.

2. The method of Claim 1, wherein said second group of particles comprise multiple white blood cells, multiple red blood cells, multiple blood platelets, and multiple granulocytes.

3. The method of Claim 1 further comprising said counting said first group of particles in said first portion of said test sample using a hemocytometer.

4. The method of Claim 1 further comprising said counting said type of stem cells in said second group of particles in said second portion of said test sample using a flow cytometer.

5. The method of Claim 1, wherein said type of stem cells comprise a type of pluripotent stem cells.

6. The method of Claim 1, wherein said type of stem cells comprise a type of multipotent stem cells.

7. The method of Claim 1, wherein said type of stem cells comprise a CD9(+), CD349(+) cell between 0.1 and 6.0 micrometers in size.

8. The method of Claim 1, wherein said type of stem cells comprise a Lgr5(+) cell between 0.1 and 6.0 micrometers in size.

9. The method of Claim 1, wherein said type of stem cells comprise a CD9(-), SSEA4(+) cell between 0.1 and 6.0 micrometers in size.

10. The method of Claim 1, wherein said tissue sample comprises a peripheral blood of said subject.

11. The method of Claim 1, wherein said first and second groups of particles have sizes greater than or substantially equal to a threshold size.

12. The method of Claim 11, wherein said threshold size is 1 micrometer.

13. The method of Claim 1, wherein said first data comprises the count per unit volume of said first group of particles in said test sample.

14. The method of Claim 1, wherein said second data comprises a percentage of the count of said type of stem cells to that of said second group of particles.

15. The method of Claim 1, wherein said third data comprises the count per unit volume of said type of stem cells in said tissue sample.
